(19) Europäisches Patentamt
European Patent Office
Office européen des brevets

(11) **EP 2 510 122 B1**

(12) **FASCICULE DE BREVET EUROPEEN**

(45) Date de publication et mention de la délivrance du brevet:
**12.04.2017 Bulletin 2017/15**

(21) Numéro de dépôt: **10807461.8**

(22) Date de dépôt: **08.12.2010**

(51) Int Cl.:
*C12Q 1/68* (2006.01)

(86) Numéro de dépôt international:
**PCT/FR2010/052650**

(87) Numéro de publication internationale:
**WO 2011/070297 (16.06.2011 Gazette 2011/24)**

(54) **UTILISATION DE MIARNS COMME BIOMARQUEURS DANS LE DIAGNOSTIC DE GLIOMES**

VERWENDUNG VON MI-RNAS ALS BIOMARKER ZUR DIAGNOSE VON GLIOMEN

USE OF MI-RNAS AS BIOMARKERS FOR DIAGNOSING GLIOMAS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.12.2009 FR 0958737**
**23.04.2010 FR 1053142**

(43) Date de publication de la demande:
**17.10.2012 Bulletin 2012/42**

(73) Titulaire: **Université Joseph Fourier**
**38041 Grenoble Cedex 09 (FR)**

(72) Inventeurs:
• **BERGER, François**
**F-38240 Meylan (FR)**
• **ISSARTEL, Jean-Paul**
**F-38120 Saint Egrève (FR)**
• **EL ATIFI-BOREL, Michèle**
**F-38190 Froges (FR)**
• **LAGES, Elodie**
**F-38000 Grenoble (FR)**
• **GUTTIN, Audrey**
**F-38730 Chassignieu (FR)**

(74) Mandataire: **Grosset-Fournier, Chantal Catherine et al**
**Grosset-Fournier & Demachy**
**54, rue Saint-Lazare**
**75009 Paris (FR)**

(56) Documents cités:
• **SILBER JOACHIM ET AL: "miR-124 and miR-137 inhibit proliferation of glioblastoma multiforme cells and induce differentiation of brain tumor stem cells", BMC MEDICINE, BIOMED CENTRAL LTD., LONDON, GB, vol. 6, no. 1, 24 juin 2008 (2008-06-24), page 14, XP021037895, ISSN: 1741-7015**
• **CIAFRE ET AL: "Extensive modulation of a set of microRNAs in primary glioblastoma", BIOCHEMICAL AND BIOPHYSICAL RESEARCH COMMUNICATIONS, ACADEMIC PRESS INC. ORLANDO, FL, US, vol. 334, no. 4, 9 septembre 2005 (2005-09-09), pages 1351-1358, XP005001537, ISSN: 0006-291X**
• **NEGRINI M ET AL: "MicroRNAs and cancer-new paradigms in molecular oncology", CURRENT OPINION IN CELL BIOLOGY, CURRENT SCIENCE, LONDON, GB LNKD-DOI:10.1016/J.CEB.2009.03.002, vol. 21, no. 3, 1 juin 2009 (2009-06-01), pages 470-479, XP026161494, ISSN: 0955-0674 [extrait le 2009-05-04]**
• **SILBER JOACHIM ET AL: "microRNAs in Gliomas: Small Regulators of a Big Problem", NEUROMOLECULAR MEDICINE, vol. 11, no. 3, Sp. Iss. SI, septembre 2009 (2009-09), pages 208-222, XP002579555, ISSN: 1535-1084**
• **JESSE CHUNG-SEAN PANG ET AL: "Oncogenic role of microRNAs in brain tumors", ACTA NEUROPATHOLOGICA, SPRINGER, BERLIN, DE, vol. 117, no. 6, 3 avril 2009 (2009-04-03), pages 599-611, XP019713406, ISSN: 1432-0533**

**Description**

La présente invention concerne l'utilisation d'au moins deux miARNs pour la mise en oeuvre d'une méthode diagnostic spécifique in vitro d'une tumeur cérébrale appartenant au groupe constitué de 2 types de tumeurs : ODG, GBM, et d'identification du type de la tumeur, comprenant :

(1) la mesure du niveau d'expression dans un échantillon biologique provenant d'un patient suspecté de présenter un gliome, - un premier miARN choisi parmi hsa-miR-155, hsa-miR-lOa, hsa-miR-409 ou hsa-miR-134, et - un deuxième miARN choisi parmi hsa-miR-126, hsa-miR-15b, hsa-miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-151, hsa-miR-26b, hsa-miR-34b*, hsa-miR-155 ou hsa-miR-10a, ledit deuxième miARN choisi étant différent du susdit premier miARN choisi,

(2) la comparaison entre les niveaux d'expression des susdits miARNs dans le susdit échantillon et les niveaux d'expression des susdits miARNs dans un échantillon sain de référence, un échantillon de GBM de référence, et un échantillon d'ODG de référence;

(a) dans laquelle la déduction de la présence de GBM dans l'échantillon provenant du patient suspecté de présenter un gliome est faite, lorsque :

(i) le niveau d'expression du premier miARN mesuré dans ledit échantillon est au moins 3 fois supérieur ou inférieur au niveau d'expression du susdit miARN mesuré dans l'échantillon sain de référence, et,

(ii) le niveau d'expression du premier miARN mesuré dans ledit échantillon est au moins 3 fois supérieur au niveau d'expression du susdit miARN mesuré dans l'échantillon d'ODG de référence, et

(iii) le niveau d'expression du deuxième miARN mesuré dans ledit échantillon est au moins 3 fois supérieur ou inférieur au niveau d'expression du susdit miARN mesuré dans l'échantillon sain de référence, et,

(iv) le niveau d'expression du deuxième miARN mesuré dans ledit échantillon est égal, dans la limite de 20% en plus ou 20% en moins, au niveau d'expression du susdit miARN dans l'échantillon de GBM de référence; et,

(b) dans laquelle la déduction de la présence d'ODG dans l'échantillon provenant du patient suspecté de présenter un gliome est faite, lorsque :

(i) le niveau d'expression du premier miARN mesuré dans ledit échantillon est au moins 3 fois supérieur ou inférieur au niveau d'expression du susdit miARN mesuré dans l'échantillon sain de référence, et,

(ii) le niveau d'expression du premier miARN mesuré dans ledit échantillon est au moins 3 fois inférieur au niveau d'expression du susdit miARN mesuré dans l'échantillon de GBM de référence, et,

(iii) le niveau d'expression du deuxième miARN mesuré dans ledit échantillon est au moins 3 fois supérieur ou inférieur au niveau d'expression du susdit miARN mesuré dans l'échantillon sain de référence, et,

(iv) le niveau d'expression du deuxième miARN mesuré dans ledit échantillon est égal, dans la limite de 20% en plus ou 20% en moins, au niveau d'expression du susdit miARN dans l'échantillon d'ODG de référence.

Les tumeurs du système nerveux central (SNC) sont pour la plupart des gliomes (50%) regroupant les astrocytomes et les oligodendrogliomes. Ces tumeurs constituent une cause importante de morbidité et de mortalité et représentent un problème important de santé publique. En effet, les tumeurs du SNC constituent la deuxième cause de décès par cancer chez les enfants et la troisième cause chez l'homme adulte (15-34 ans). L'incidence globale des tumeurs du SNC est voisine de 7000 nouveaux cas dépistés chaque année en France. Enfin, sur 6 millions de décès annuels dans le monde à cause d'une forme quelconque de cancer, 1 à 2 % sont imputables aux tumeurs du SNC (Données de l'OMS).

L'exérèse de la tumeur constitue un moyen, a priori radical, pour éradiquer une tumeur. Mais dans la pratique clinique, l'efficacité de ce traitement est souvent compromise, car l'ablation de la tumeur souvent ne peut pas être exhaustive. En effet, à cause de l'imprécision des méthodes de diagnostic actuelles, les limites de la tumeur sont parfois mal évaluées lors du diagnostic.

La radiothérapie largement utilisée en complément de la chirurgie ne permet pas de supprimer définitivement les tumeurs. La chimiothérapie, utilisant divers composés pharmaceutiques d'intérêt anti-cancéreux, tels que le cisplatine, ou le temodal, est un autre traitement souvent utilisé dans la lutte contre des tumeurs. Cependant, la chimiothérapie n'est pas efficace pour tous les gliomes, soit parce que certains gliomes, par exemple les glioblastomes, sont peu chimio-sensibles, soit parce que certains composés pharmaceutiques sont incapables de passer la barrière hémato-encéphalique.

A l'heure actuelle, en dehors de l'analyse histologique réalisée par l'anatomo-pathologiste, il n'existe que quelques rares tests-diagnostics basés sur des approches moléculaires. Les observations microscopiques de la morphologie des cellules sont complétées dans quelques laboratoires par la recherche de certains marqueurs protéiques à l'aide d'anticorps spécifiques. Ces tests immunohistochimiques présentent néanmoins de nombreux inconvénients pour l'instant. Ils n'ont qu'un faible débit de productivité (ils ne permettent la détection que d'un seul antigène à la fois), ne permettent pas de distinguer sans ambiguïté les différents types de cellules tumorales et n'offrent que de médiocres possibilités de détection quantitative des marqueurs. Les diagnostics réalisés sur la détection de marqueurs génétiques (tels que les pertes d'hétérozygotie 1p et 19q dans les oligodendrogliomes) sont actuellement peu usités.

Les microARNs (miARN) représentent une classe d'ARN simple brin de petite taille (21 à 25nt), endogènes, non codants, d'identification relativement récente. Les gènes responsables de l'expression de ces miARNs sont préalablement transcrits sous formes de longs précurseurs, eux-mêmes clivés en pré-miARN par la RNAse III Drosha. La maturation de ces pré-miARN est effectuée par une autre RNAse, Dicer, qui donne lieu à un duplex d'ARN noté [miARN : miARN*]. Seul l'un des 2 brins, le brin mature, participe au complexe ribonucléoprotéique RISC (RNA-induced silencing complex) pour fonctionner comme régulateur post-transcriptionnel (Bartel et al., Cell, 2004. 116(2) : 281-97).

Les miARNs peuvent réguler l'expression des gènes soit par dégradation d'ARN messagers cibles, soit par répression de leur traduction suivant le degré de complémentarité entre le miARN et son ARNm cible. Ces ARNs jouant un rôle important dans la régulation traductionnelle, ils sont donc fortement impliqués dans de nombreux processus cellulaires tels que le développement, la différenciation, la prolifération, l'apoptose et la réponse aux stress, processus souvent dérégulés dans les tumeurs.

De nombreux travaux scientifiques ont été consacrés au rôle des miARN dans les processus d'oncogenèse (Benard, J. and S. Douc-Rasy, Bull Cancer, 2005. 92(9) : p. 757-62; Hammond Curr Opin Genet Dev, 2006. 16(1) : p. 4-9). Maintenant, il est reconnu que des miARNs sont activement impliqués dans le développement des cancers.

Chez l'Homme, le nombre de gènes codant les précurseurs de miARNs actuellement identifiés est de 721 (Sanger miRBase version 14, sept 2009). Certains de ces miARNs ont été détectés comme marqueurs tumoraux dans le contexte des cancers du sein et de la prostate et sont donc considérés comme des miARN oncogènes ou suppresseurs de tumeur suivant leur expression dans les tumeurs et leur rôle post-transcriptionnel.

Dans les dernières années, l'altération éventuelle des niveaux d'expression des miARNs dans les tumeurs gliales a déjà fait l'objet de plusieurs publications scientifiques.

Une publication de Ciafre et al. en 2005 (Biochem Biophys Res Commun. 2005. Sep 9 ; 334(4) : 1351-8) compare les niveaux d'expression de 245 miARNs dans les glioblastomes et dans les tissus normaux, et met en évidence un profil d'expression distinct de ces miARNs dans les glioblastomes par rapport au profil d'expression dans les tissus normaux. Cette publication décrit que miR-221 est surexprimé dans les glioblastomes, alors que miR-128, miR-181a, miR181b et miR181c sont sous exprimés dans les glioblastomes.

Au même moment, Chen (N Engl J Med, 2005. 353 (17) : 1768-71) décrit que miR-21 est fortement surexprimé dans les glioblastomes par rapport aux tissus normaux.

Un autre balayage des niveaux d'expression de 192 miARNs, dans les tissus de tumeurs gliales et des tissus normaux, a été effectué par Silber et al. en 2008 (BMC Med. 2008. Jun 24 ; 6 :14.). Cette étude vise à analyser une modification éventuelle du profil d'expression des miARNs dans les astrocytomes anaplasiques (grade III) ou les glioblastomes (grade IV), de tumeurs de différents grades d'un même type de tumeur. Cette étude montre un profil d'expression modifié des miARNs dans les astrocytomes anaplasiques et les glioblastomes, par rapport aux tissus normaux. Les miARNs mentionnés dans cette publication, dont l'expression est modifiée, ne sont pas identiques à ceux déjà mentionnés dans les publications antérieures.

Cependant, jusqu'à présent, aucune étude antérieure n'a fait un balayage exhaustif, car l'ensemble des microARNs exprimés dans les cellules humaines n'est jamais exploré.

Afin de lutter efficacement contre les tumeurs gliales, la mise en place d'une méthode de diagnostic de gliomes, fiable, permettant de distinguer précisément un tissu de tumeur gliale d'un tissu normal, est donc d'une importance décisive.

Les gliomes ou tumeurs gliales sont classés par l'Organisation Mondiale de la Santé (OMS) en 4 grades histologiques, le grade indiquant principalement le degré de malignité (voir le tableau 1).

**Tableau 1**

| Grade I | Astrocytome pilocytique curable par chirurgie |
|---|---|
| Grade II | Oligoastrocytomes deux types de cellules : astrocytes et oligodendrogliales |
| | Oligodendrogliomes, |
| | Astrocytomes diffus |

(suite)

| Grade III | Oligodendrogliomes anaplasiques |
|---|---|
| | Oligoastrocytomes anaplasiques |
| | Astrocytomes anaplasiques |
| Grade IV | Glioblastome multiforme |

Les oligodendrogliomes, classés en grade II par OMS, sont des gliomes de bas grade, développés à partir des oligodendrocytes. Ce gliome a été jusqu'à ce jour très souvent sous-diagnostiqué, à cause de défauts de méthode de diagnostic efficace. Néanmoins, contrairement aux autres tumeurs gliales, les oligodendrogliomes sont remarquablement chimio-sensibles. La médiane de survie, pour les oligodendrogliomes, est d'environ 7 ans.

En revanche, les glioblastomes, classés en grade IV selon la classification de l'OMS, sont développés à partir des astrocytes, qui sont des passerelles entre le sang et les neurones, assurent la nutrition des neurones, gèrent les connections interneuronales, et régulent les neurotransmetteurs. Les glioblastomes sont des tumeurs astrocytaires malignes. En l'absence d'exérèse chirurgicale satisfaisante et de chimio-sensibilité vis-à-vis de la plupart des médicaments anticancéreux actuellement mis sur le marché, le pronostic de ces tumeurs est très pessimiste La survie moyenne n'est que d'environ 1 an pour les glioblastomes après traitement.

Par conséquent, une méthode de diagnostic capable de distinguer rapidement et correctement un oligodendrogliome d'un glioblastome permet à un patient de recevoir des soins correspondant à son état physique et d'augmenter ses chances de survie.

Cependant, dans les publications antérieures mentionnées ci-dessus, le matériel biologique de départ est constitué de glioblastomes comparés à du tissu péritumoral ou du tissu sain ; ou des cellules de glioblastomes en culture comparées à du tissu cérébral sain ; ou des glioblastomes, correspondant à des astrocytomes de haut grade, comparées à des tumeurs astrocytaires de bas grades, telles que des astrocytomes anaplastiques. En revanche, les comparaisons entre glioblastomes et oligodendrogliomes n'ont jamais été abordées dans l'art antérieur.

Donc, un autre aspect de l'invention a pour objectif de mettre en place une méthode de diagnostic permettant de distinguer précisément un glioblastome d'un oligodendrogliome.

L'invention repose sur une constatation inattendue faite par les Inventeurs lors d'un balayage des niveaux d'expression de 282 microARNs dans les tissus normaux, et les tissus provenant de deux types de tumeurs de nature et d'origine différents, dont les glioblastomes (GBM) et les oligodendrogliomes (ODG).

En effet, les Inventeurs ont constaté que le profil d'expression de ces miARNs est nettement différent dans les tissus de tumeur gliale par rapport à celui présent dans les tissus normaux.

Basée sur la comparaison faite entre le profil d'expression de 282 miARNs dans les tumeurs gliales (glioblastomes ou oligodendrogliomes) et des tissus cérébraux sains, la présente invention a repéré 19 miARNs dont l'expression est soit systématiquement surexprimée, soit systématiquement sous-exprimée, dans les tumeurs gliales par rapport aux tissus cérébraux sains.

Ainsi, le premier aspect de la méthode a pour objet l'utilisation d'au moins un miARN choisi parmi hsa-miR-126, hsa-miR-15b, hsa- miR-16, hsa-miR-17, hsa-miR-20a, hsa-let7a, hsa-let7f, hsa-let7d, hsa-miR-374, hsa-miR-409, hsa-miR-134, hsa-miR-339, hsa-miR-127, hsa-miR-151, hsa-miR-26b, hsa-miR-34b*, hsa-miR-155, hsa-miR-210 ou hsa-miR-10a pour la mise en oeuvre d'une méthode de diagnostic *in vitro* de gliomes.

La séquence nucléique et le numéro d'accession pour chaque miARN mentionné dans la présente méthode sont présentés ci-dessous dans le tableau 2.

**Tableau 2**

| miARNs exprimés différemment dans les tumeurs gliales par rapport à du tissu cérébral sain | Numéro de séquence | Accession MIMAT | séquence |
|---|---|---|---|
| hsa-miR-126 | SEQ ID NO : 1 | MIMAT0000445 | ucguaccgugaguaauaaugcg |
| hsa-miR-16 | SEQ ID NO : 2 | MIMAT0000069 | uagcagcacguaaauauuggcg |
| hsa-miR-20a | SEQ ID NO : 3 | MIMAT0000075 | uaaagugcuuauagugcagguag |
| hsa-let 7a | SEQ ID NO : 4 | MIMAT0000062 | ugagguaguagguuguauaguu |
| hsa-let 7f | SEQ ID NO : 5 | MIMAT0000067 | ugagguaguagauuguauaguu |
| hsa-let 7d | SEQ ID NO : 6 | MIMAT0000065 | agagguaguagguugcauaguu |

(suite)

| miARNs exprimés différemment dans les tumeurs gliales par rapport à du tissu cérébral sain | Numéro de séquence | Accession MIMAT | séquence |
|---|---|---|---|
| hsa-miR-374a | SEQ ID NO : 7 | MIMAT0000727 | uuauaauacaaccugauaagug |
| hsa-miR-15b | SEQ ID NO : 8 | MIMAT0000417 | uagcagcacaucaugguuuaca |
| hsa-miR-127-3p | SEQ ID NO : 9 | MIMAT 0000446 | ucggauccgucugagcuuggcu |
| hsa-miR-339-5p | SEQ ID NO : 10 | MIMAT0000764 | ucccuguccuccaggagcucacg |
| hsa-miR-409-5p | SEQ ID NO : 11 | MIMAT0001638 | agguuacccgagcaacuuugca |
| hsa-miR-134 | SEQ ID NO : 12 | MIMAT0000447 | ugugacugguugaccagagggg |
| hsa-miR-155 | SEQ ID NO : 13 | MIMAT0000646 | uuaaugcuaaucgugauaggggu |
| hsa-miR-210 | SEQ ID NO : 14 | MIMAT0000267 | cugugcgugugacagcggcuga |
| hsa-miR-10a | SEQ ID NO : 15 | MIMAT0000253 | uacccuguagauccgaauuugug |
| hsa-miR-26b | SEQ ID NO : 16 | MIMAT0000083 | uucaaguaauucaggauaggu |
| hsa-miR-34b* | SEQ ID NO : 17 | MIMAT0000685 | uaggcagugucauuagcugauug |
| hsa-miR-17 | SEQ ID NO : 18 | MIMAT0000070 | caaagugcuuacagugcagguag |
| hsa-miR-151-3p | SEQ ID NO : 19 | MIMAT0000757 | cuagacugaagcuccuugagg |

Dans la présente méthode on entend que hsa-miR-20 et hsa-miR-20a correspondent à la même séquence nucléique.

Les miARNs mentionnés ci-dessus sont référencés dans la base de données miRBase (http://www.mirbase.org/) (*miRBase : tools for microRNA genomic*, Griffiths-Jones et al., NAR 2008 36(Database Issue):D154-D158) où la séquence nucléique d'un miARN peut être retrouvée par un numéro d'accession MIMAT ou une annotation uniforme. Un système d'annotation a été mis en place pour nommer les différents miARNs (*A uniform system for microRNA annotation*, Ambros et al., RNA 2003 9(3):277-279). Dans ce système, les microARNs sont désignés par un numéro. Précédent ce numéro d'identification, se trouve l'abréviation « miR » ou « mir », qui permet une distinction entre le microARN mature (miR) et la boucle précurseur (hairpin) du microARN (mir), correspondant respectivement à un numéro d'accession MIMAT et MI. Un préfixe de trois ou quatre lettres est utilisé pour distinguer les origines génomiques de ce miARN. Par exemple « hsa-miR-126 » signifie que ce miARN mature provient du génome humain. Parfois, les microARN matures peuvent être très proches (une seule base de différence). Dans ce cas, les microARNs portant un même numéro sont distingués entre eux par une lettre minuscule comme a, b, c...par exemple hsa-miR-26b. Parfois une boucle précurseur peut générer, de son côté 5' et son coté 3', deux microARNs matures qui sont distingués l'un de l'autre par l'annotation « 5p » ou « 3p ». Par exemple « hsa-miR-151-3p » signifie un miARN mature provenant du côté 3' de son précurseur.

Les miARNs utilisés dans la présente méthode sont les miARNs matures provenant du génome humain, qui peuvent être produits naturellement par des cellules, ou synthétisés par des méthodes chimiques conventionnelles, ou des méthodes biologiques, telles qu'un vecteur recombinant.

Dans la présente méthode les expressions « miARN » et « microARN » peuvent être remplacées l'une par l'autre. Les expressions tumeurs gliales ou gliomes sont équivalentes. Glioblastomes et oligodendrogliomes sont deux types de gliomes. Tissu sain ou échantilllon sain signifient tissu non tumoral ou échantillon non tumoral. Ces expressions au singulier peuvent s'entendre au pluriel et réciproquement.

Dans un mode de réalisation avantageux, la méthode concerne l'utilisation d'au moins un miARN choisi parmi hsa-miR-126, hsa-miR-15b, hsa-miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-151, hsa-miR-26b ou hsa-miR-34b*, pour la mise en oeuvre d'une méthode de diagnostic *in vitro* de gliomes.

L'expression aberrante de ces 16 miARNs dans une tumeur gliale n'a jamais été mentionnée par les publications antérieures, soit parce que le balayage des miARNs effectué dans les travaux antérieurs n'est pas exhaustif, (par exemple l'étude de Silber et al. en 2008 ne concerne que 192 miARNs), soit parce que la technique employée auparavant ne permet pas de donner une plus haute résolution par rapport à la technique adoptée dans l'invention. En effet, l'étude de Ciafrè et al. en 2005, utilisant la technique de la « puce à ADN » pour quantifier l'expression des miARNs, ne montre pas une différence significative entre le niveau d'expression de miR-16 dans les tissus de glioblastomes et le niveau d'expression du susdit miARN dans les tissus normaux, alors que la présente description illustre que miR-16 est surexprimé dans les glioblastomes, grâce à la technique de la PCR quantitative, qui permet de distinguer de manière plus précise une différence de quantité d'ARN entre deux échantillons.

Parmi l'ensemble des 19 miARNs repérés dans la description il y a 5 miARNs, à savoir hsa-miR-155, hsa-miR-210 ou hsa-miR-10a, hsa-miR-409 et hsa-miR-134, dont le profil d'expression est très particulier. Le profil d'expression de ces 5 miARNs, qui est différent dans les tumeurs par rapport à celui dans les tissus normaux, est également différencié entre les glioblastomes et les oligodendrogliomes. Les niveaux d'expression de ces 5 miARNs sont systématiquement plus élevés dans les glioblastomes par rapport à ceux dans les oligodendrogliomes (voir le tableau 3 dans l'exemple 2).

Dans un mode de réalisation avantageux, la méthode concerne l'utilisation d'au moins un miARN choisi parmi hsa-miR-155, hsa-miR-210 ou hsa-miR-10a, hsa-miR-409 ou hsa-miR-134 pour la mise en oeuvre d'une méthode de diagnostic spécifique *in vitro* de gliomes, ladite méthode permettant de distinguer le glioblastome de l'oligodendrogliome.

Dans un mode de réalisation particulièrement avantageux, la méthode concerne l'utilisation d'au moins deux miARNs pour la mise en oeuvre d'une méthode diagnostic spécifique in vitro d'une tumeur cérébrale appartenant au groupe constitué de 2 types de tumeurs : ODG, GBM, et d'identification du type de la tumeur, dans laquelle :

- un premier miARN est choisi parmi hsa-miR-155, hsa-miR-210, hsa-miR-10a, hsa-miR-409 ou hsa-miR-134 ;
- un deuxième miARN est choisi parmi hsa- miR-126, hsa-miR-15b, hsa- miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-151, hsa-miR-26b, hsa-miR-34b*, hsa-miR-155, hsa-miR-210 ou hsa-miR-10a,
- le premier et le deuxième miARN sont différents.

Un autre aspect de la description a pour objet une méthode de diagnostic *in vitro* de gliomes. Cette méthode permet de diagnostiquer si un patient est atteint d'un gliome.

Cette méthode comprend les étapes suivantes :

- la mesure du niveau d'expression d'au moins un miARN choisi parmi hsa-miR-126, hsa-miR-15b, hsa-miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-151, hsa-miR-26b, hsa-miR-34b*, hsa-miR-155, hsa-miR-210 ou hsa-miR-10a, dans un échantillon biologique provenant d'un patient suspecté de présenter un gliome,
- la comparaison :

  - entre le niveau d'expression d'au moins un susdit miARN dans le susdit échantillon et le niveau d'expression du susdit miARN dans un échantillon sain de référence, ou
  - entre le niveau d'expression d'au moins un premier susdit miARN dans l'échantillon provenant d'un patient suspecté de présenter un gliome et le niveau d'expression d'au moins un deuxième susdit miARN dans l'échantillon provenant dudit patient suspecté de présenter un gliome.

La comparaison des niveaux d'expressions des miARNs peut être réalisée entre un échantillon provenant d'un patient suspecté de présenter un gliome et un échantillon sain de référence, ou un échantillon de référence provenant d'un patient présentant un glioblastome ou d'un patient présentant un oligodendrogliome.

Par « échantillon biologique », on entend un échantillon tissulaire, notamment un tissu cérébral obtenu par exérèse, ou un échantillon de liquides biologiques, notamment un échantillon sanguin.

Un échantillon tissulaire cérébral peut être un tissu du système nerveux central provenant du cortex cérébral, du cortex cérébelleux, des noyaux gris centraux ou des noyaux du tronc cérébral.

Un échantillon de liquide biologique peut être un sérum sanguin ou un plasma sanguin.

Lorsqu'il s'agit d'un échantillon tissulaire cérébral, « Un échantillon sain de référence » est un tissu cérébral dépourvu de tout caractère tumoral d'après une analyse histologique réalisée par les anatomopathologistes. Le tissu sain de référence peut être un tissu du système nerveux central provenant du cortex cérébral, du cortex cérébelleux, des noyaux gris centraux ou des noyaux du tronc cérébral. Le tissu sain de référence est préférentiellement prélevé à partir d'un même patient (tissu sain de zone périphérique à la tumeur). Alternativement il peut être constitué de fragments encéphaliques obtenus après chirurgie dite de cortectomie réalisée sur des individus ne présentant pas de tumeurs du cerveau mais dans le cadre d'intervention pour traitement de l'épilepsie ou suite à des traumatismes crâniens.

Lorsqu'il s'agit d'un échantillon de liquides biologiques, « un échantillon sain de référence » est prelevé d'une personne saine, et notamment d'une personne ne présentant aucun cancer.

Lorsque « un échantillon biologique provenant d'un patient suspecté de présenter un gliome » est un échantillon tissulaire cérébral, il s'agit d'un tissu prélevé, par biopsie ou par exérèse au bloc opératoire ou toute autre méthode de prélèvement intratumoral adapté, du siège tumoral soupçonné d'un patient. Le siège tumoral soupçonné est repéré préalablement par une méthode classique, telle qu'une IRM, un PET Scan (Scanner par image de rayons gamma), ou un scanner classique.

Lorsque « un échantillon biologique provenant d'un patient suspecté de présenter un gliome » est un échantillon sanguin, cet échantillon est obtenu d'un patient par prélèvement du sang selon les méthodes conventionnelles,

« Un échantillon de référence provenant d'un patient présentant un glioblastome ou d'un patient présentant un oligodendrogliome» est un échantillon provenant d'un patient dont la présence de l'une des deux tumeurs a été certifiée par l'analyse anatomo-pathologique. Idéalement, le diagnostic peut également être confirmé par des analyses moléculaires telles que par exemple l'analyse des profils d'expression de gènes de ces tissus tumoraux. Les tumeurs sont ensuite caractérisées en utilisant la méthode de classification de Li et al (Li A, Walling J, Ahn S, Kotliarov Y, Su Q, Quezado M, Oberholtzer JC, Park J, Zenklusen JC, Fine HA. Cancer Res. 2009 Mar 1;69(5):2091-9) qui repose sur l'utilisation des niveaux d'expression d'un jeu de 54 gènes. Les glioblastomes ou oligodendrogliomes sont classés respectivement dans les groupes G et O de la publication suscitée.

« Un échantillon de référence provenant d'un patient présentant un glioblastome ou d'un patient présentant un oligodendrogliome » est un échantillon tissulaire cérébral, le susdit tissu peut être un fragment de tumeur gliale obtenu par exérèse chez un patient et dont le type de tumeur a été certifié par l'analyse anatomo-pathologique.

Les miARNs mesurés dans cette méthode sont contenus dans des fractions de purification d'ARNs obtenus soit à partir d'extraits ou lysats provenant de tissus cérébraux obtenus par biopsie ou par exérèse au bloc opératoire ou toute autre méthode de prélèvement intratumoral adapté, lorsqu'il s'agit d'un échantillon tissulaire ; soit à partir d'extraits ou lysats des différentes fractions des liquides biologiques en utilisant des méthodes adaptées, lorsqu'il s'agit d'un échantillon de liquides biologiques.

Les extraits de tissus utilisés pour les dosages correspondent à des fractions obtenues après lyse des tissus et purification plus ou moins poussée des ARNs selon des méthodes conventionnelles (par exemple la méthode mirVana commercialisée par Ambion, ou RNeasy par Qiagen, ou méthodes de purification par électrophorèse). Selon les méthodes, les fractions contiennent des ARN totaux, ou des ARNs de tailles inférieures à 200 bases, ou des fractions enrichies en microARN.

Les miARNs mesurés dans un échantillon sanguin peuvent être la totalité des miARNs circulants dans le plasma ou le sérum, ou les miARNs localisés dans les microvésicules présentes dans le sérum.

La totalité des miARNs circulants dans le sang est purifiée directement à partir de prélèvements de sang selon les méthodes conventionelles, telles que ce qui est décrit par Mitchell PS et al. (Mitchell PS et al., Proc Natl Acad Sci USA. 2008 Jul 29;105(30):10513-8) ou par Lodes MJ (Lodes MJ et al., PLoS One. 2009 Jul 14;4(7):e6229).

Les miARNs localisés dans les microvésicules présentes dans le sérum sont purifiés selon une approche décrite par exemple par Skog et al (Skog et al., Nat Cell Biol. 2008 Dec;10(12):1470-6).

Le terme « niveau d'expression » d'un miARN dans un échantillon correspond à une valeur de mesure propre à un miARN, mais exprimé soit en unité arbitraire, soit en unité de masse, de molécules ou de concentration, ou en valeur normalisée par rapport à une autre mesure, en particulier en valeur normalisée par rapport aux quantités du même miARN dans un tissu de référence (sain ou tumoral)

Le niveau d'expression des miARNs peut être mesuré par toute méthode conventionnelle, telle que

- l'hybridation sur « puces à ADN »,
- des méthodes de séquençage à haut débit d'un grand nombre de miARN individualisés,
- la PCR en temps réel,
- le Northern blot,

ou encore par toute autre méthode spécifique des miARNs.

Le niveau d'expression des miARNs peut être mesuré par la technique de la « puce à ADN ». La technique de la « puce à ADN » est bien connue de l'homme du métier. Il s'agit de l'hybridation de miARNs extraits sur un support solide composé d'une membrane nylon, d'une surface de silicium ou de verre, éventuellement de nano-billes ou particules, comportant des oligonucléotides de séquences connues fixés sur le support ou adhérents à celui-ci. La complémentarité des oligonucléotides fixés avec les séquences des microARNs ou de leurs produits de conversion (produits d'amplification, cDNA, ARN ou cARN) permet de générer un signal (fluorescence, luminescence, radioactivité, signal électrique...) selon les techniques de marquage employées, au niveau des oligonucléotides immobilisés sur les supports (puces à ADN). Ce signal est détecté par un équipement spécifique et une valeur d'intensité de ce signal propre pour chaque miARN est ainsi enregistrée. Plusieurs types de puces destinées à la détection des miARNs sont déjà mis sur le marché, comme par exemple les GeneChip® miRNA commercialisés par Affymetrix, miRcury arrays par Exiqon, miRXplore microarrays par Miltenyi.

Dans le cas de l'analyse haut débit par séquençage, les miARNs sont extraits et purifiés d'un échantillon, isolés les uns des autres par des méthodes proposées par les fournisseurs d'équipements de séquençage tels que Roche, Invitrogen. Ce genre d'analyse consiste à individualiser les molécules des différents microARNs, de procéder à une étape d'amplification et de séquencer les produits (« clones d'acides nucléiques ») ainsi générés. La réalisation de très nombreuses séquences pour identifier chacun de ces « clones » (plusieurs milliers) permet de générer un listing des microARNs présents dans un échantillon et de quantifier chacun de ces miARNs en comptant tout simplement combien de fois chaque séquence est retrouvée dans le listing détaillé.

Dans un mode de réalisation préférée les dosages de miARN sont réalisés par PCR quantitative (PCR en temps réel). La PCR en temps réel permet d'obtenir des valeurs, appelées Ct, correspondant au nombre de cycles à partir duquel la fluorescence émise dépasse un certain seuil, le seuil étant fixé par l'utilisateur en début de phase exponentielle. Cette valeur de Ct est proportionnelle à la quantité de cDNA (provenant de la transcription inverse des miARN en cDNA par la Reverse Transcriptase) présent initialement dans l'échantillon. En l'absence de gamme-étalon spécifique pour chaque cDNA, seule une quantification relative entre échantillons est possible. Dans un premier temps, afin de pouvoir comparer le contingent de chaque miARN pris individuellement et présent dans les glioblastomes, les oligodendrogliomes et les tissus sains, les valeurs de dosage pour chaque miARN sont normalisées avec les données obtenues pour un ARN non-codant (de la catégorie des snoRNA) appelé RNU24 (SEQ ID NO : 20). Le choix de RNU24 est dicté par une analyse des mesures de plusieurs snoRNA effectuées sur de nombreux échantillons et réalisé à l'aide du logiciel Normfinder. Cette analyse a permis de conclure que RNU24 est le normalisateur le plus fiable dans la présente étude (son taux d'expression varie en fait très peu entre tous les échantillons tissulaires utilisés dans l'invention). La quantification relative d'un miARN entre 2 types d'échantillons est obtenue ensuite grâce par exemple au logiciel REST qui prend en compte le paramètre d'efficacité de la PCR et les valeurs de Ct du miARN d'intérêt et de RNU24 pour tous les échantillons analysés. Cependant, la présente invention peut être mise en oeuvre avec d'autres normalisateurs connus de l'homme de métier.

Lorsque les niveaux d'expression des miARNs sont analysés par hybridation sur « puces à ADN », ou par Northern blot, ou par séquençage, ils peuvent être exprimés par la formule I :

$$\text{Quantité de miARNx} = \text{intensité du signal de détection pour miARNx}$$

Où « intensité de signal » signifie quantité de fluorescence, de radioactité ou de luminescence enregistrée sur les « puces à ADN » par le détecteur adapté, ou nombre de séquences identiques détectées par l'analyse à haut débit par séquençage. Les quantités sont exprimées en unités arbitraires.

Les quantités de miARN peuvent être normalisées par rapport à un autre dosage, notamment un ARN dont la concentration ne varie pas dans les différents types d'échantillons analysés. En particulier, la quantité tissulaire d'un ARN non-codant appelé RNU24 a été montrée par les inventeurs comme très stable entre les échantillons tumoraux et les échantillons de tissus sains. Cette normalisation permet de garantir que l'on compare les niveaux d'expression des miARNs détectables dans des extraits dont les concentrations en ARN sont similaires entre ces différents extraits purifiés. Dans ce cas, le niveau d'expresssion normalisé pour un miARN dans un échantillon de tissu est exprimé par la formule II :

$$\text{Quantité de miARNx normalisée} = \text{intensité du signal de détection pour miARNx} \, / $$

$$\text{intensité du signal pour RNU24.}$$

Lorsque les niveaux d'expression des miARNs sont analysés par la PCR en temps réel, ils peuvent être exprimés par la formule III, qui définit la quantité de miARN présente dans le mileu réactionnel de dosage lorsque le nombre de cycles d'amplification est égal à Ct (Quantité de miARNx à Ct) :

$$\text{Quantité de miARNx à Ct} = \text{Quantité de miARN à } t_o \text{ x Efficacité}^{-Ct}.$$

Où « Quantité de miARN à $t_o$ » désigne la quantité de miARN, ou son équivalent en cDNA, au moment où la réaction de dosage par amplification PCR est initiée. L'expression « efficacité » dans la formule (III) signifie la valeur de l'efficacité de PCR (de valeur comprise entre 1 et 2). Cette valeur dépend de divers paramètres expérimentaux, et notamment de l'appareil effectuant la PCR en temps réel mis en oeuvre. Une fois que cette valeur est mesurée pour un protocole particulier et une machine de PCR configurée, il n'est plus nécessaire de mesurer cette valeur chaque fois avant le calcul, sauf si le protocole expérimental et/ou la condition de fonctionnement de la machine ont été modifiés pour l'expérience donnée.

Dans un mode de réalisation particulier l'échantillon biologique provenant d'un patient suspecté de présenter un gliome est un tissu cérébral obtenu par exérèse, ou par biopsie.

Cette méthode comprend :

- la mesure du niveau d'expression d'au moins un miARN choisi parmi hsa-miR-126, hsa-miR-15b, hsa-miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-151, hsa-miR-26b, hsa-miR-34b*, hsa-miR-155, hsa-miR-210 ou hsa-miR-10a, dans un tissu biologique provenant d'un tissu suspecté de présenter un caractère tumoral,
- la comparaison :

  - entre le niveau d'expression d'au moins un susdit miARN dans le tissu suspecté et le niveau d'expression du susdit miARN dans un tissu sain de référence, ou
  - entre le niveau d'expression d'au moins un premier susdit miARN dans le tissu suspecté et le niveau d'expression d'au moins un deuxième susdit miARN dans le tissu suspecté.

Les comparaisons des niveaux d'expression des miARNs peuvent être faites entre un échantillon suspecté et un échantillon sain de référence. Il s'agit d'une comparaison inter-échantillon.

Dans un premier mode de réalisation, la méthode de diagnostic *in vitro* de gliomes comprend :

- la mesure du niveau d'expression d'au moins un miARN choisi parmi hsa-miR-126, hsa-miR-15b, hsa-miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-151, hsa-miR-26b, hsa-miR-34b*, hsa-miR-155, hsa-miR-210 ou hsa-miR-10a, dans un échantillon biologique provenant d'un patient suspecté de présenter un gliome,
- la comparaison entre le niveau d'expression d'au moins un susdit miARN dans le susdit échantillon et le niveau d'expression du susdit miARN dans un échantillon sain de référence, et
- la déduction que le patient suspecté présente un gliome lorsque le niveau d'expression du susdit miARN dans l'échantillon provenant d'un patient suspecté de présenter un gliome est différent du niveau d'expression du susdit miARN dans un échantillon sain de référence.

Dans un mode de réalisation avantageux cette méthode comprend la mesure du niveau d'expression d'au moins un miARN choisi parmi hsa-miR-126, hsa-miR-15b, hsa-miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-151, hsa-miR-26b ou hsa-miR-34b*.

Parmi les 16 miARNs repérés dans l'invention, il y a 10 miARNs dont le niveau d'expression est systématiquement plus élevé dans les échantillons provenant d'un patient présentant un gliome par rapport à celui dans les échantillons sains de référence, alors que le niveau d'expression de 6 autres miARNs est plus faible dans les échantillons provenant d'un patient présentant un gliome par rapport à celui dans les échantillons sains de référence.

Ainsi, un mode de réalisation particulier de la méthode de diagnostic *in vitro* de gliomes comprend:

- la mesure du niveau d'expression est effectuée pour l'un au moins des miARNs suivants : hsa-miR-126, hsa-miR-15b, hsa-miR-16, hsa-miR-17, hsa-miR-20a, hsa-let7a, hsa-let 7f, hsa-let 7d, hsa-miR-374 ou hsa-miR-26b, et
- la déduction que le patient suspecté présente un gliome est faite lorsque le niveau d'expression du susdit miARN dans l'échantillon provenant d'un patient suspecté de présenter un gliome est supérieur au niveau d'expression du susdit miARN dans un échantillon sain de référence.

Un autre mode de réalisation particulier de la méthode de diagnostic *in vitro* de gliomes comprend:

- la mesure du niveau d'expression est effectuée pour l'un au moins des miARNs suivants : hsa-miR-409, hsa-miR-134, hsa-miR-339, hsa-miR-127, hsa-miR-151 ou hsa-miR-34b*, et
- la déduction que le patient suspecté présente un gliome est faite lorsque le niveau d'expression du susdit miARN dans l'échantillon provenant d'un patient suspecté de présenter un gliome est inférieur au niveau d'expression du susdit miARN dans l'échantillon sain de référence.

Lorsque les niveaux d'expression des miARNs sont obtenus par hybridation sur « puces à ADN » ou par séquençage, la comparaison des niveaux d'expression d'un même miARN dans deux échantillons différents destinée à mettre en évidence les modifications d'expression d'un miARN entre ces échantillons (surexpression ou sous-expression) est évaluée grâce à la formule IV :

Quantité de miARNx normalisée (échantillon A) / Quantité de miARNx normalisée (échantillon B),

où A représente l'échantillon suspecté et B représente l'échantillon sain de référence.

Lorsque les niveaux d'expression des miARNs sont obtenus par la PCR en temps réel, la comparaison des niveaux d'expression d'un même miARN dans deux échantillons différents peut être exprimée par un rapport obtenu par la formule (V) suivante:

$$\text{Rapport (échantillon A/échantillon B)} = \text{Efficacité}^{-((\text{Ct hsa-miARN X} - \text{Ct RNU24})\text{échantillon A}-(\text{Ct hsa-miARNX} - \text{Ct RNU24})\text{échantillon B})}.$$

En l'occurrence, l'échantillon A est l'échantillon suspecté; l'échantillon B est l'échantillon sain de référence. miARN X représente le miARN dont le niveau d'expression est mesuré respectivement dans l'échantillon sain de référence et l'échantillon suspecté. Les valeurs de Ct pour hsa-miARN X et pour RNU24, respectivement pour l'échantillon suspecté et l'échantillon sain de référence, sont obtenues directement par la PCR en temps réel.

Les variations des niveaux d'expression entre le niveau d'expression d'un miARN dans un échantillon provenant d'un patient suspecté de présenter un gliome et celui du susdit miRNA dans un échantillon sain de référence peuvent être déduites des formules IV ou V, selon la méthode d'analyse employée.

Dans un mode de réalisation particulier la déduction que le patient suspecté présente un gliome est faite, lorsque le niveau d'expression du susdit miARN dans l'échantillon provenant d'un patient suspecté de présenter un gliome et le niveau d'expression du susdit miARN dans l'échantillon sain de référence sont différents, le niveau d'expression du susdit miARN dans l'échantillon provenant d'un patient suspecté de présenter un gliome étant au moins 3 fois supérieur ou inférieur au niveau d'expression du susdit miARN dans l'échantillon sain de référence.

Respectivement, le résultat des formules IV ou V est supérieur à 3 ou inférieur à 0,33.

Selon un mode de réalisation avantageux, le niveau d'expression du susdit miARN dans l'échantillon provenant d'un patient suspecté de présenter un gliome est au moins 3 fois supérieur au niveau d'expression du susdit miARN dans l'échantillon sain de référence.

Par l'expression «niveau d'expression du susdit miARN dans l'échantillon provenant d'un patient suspecté de présenter un gliome est au moins 3 fois supérieur au niveau d'expression du susdit miARN dans l'échantillon sain de référence », on entend que le rapport obtenu d'après les formules (IV ou V) est supérieur à une valeur de 3. Cette valeur signifie une surexpression dudit miARN dans l'échantillon provenant d'un patient suspecté de présenter un gliome par rapport à l'échantillon sain de référence. En l'occurrence, dans ces formules, l'échantillon A est l'échantillon provenant d'un patient suspecté; l'échantillon B est l'échantillon sain de référence.

Selon un autre mode de réalisation avantageux, le niveau d'expression du susdit miARN dans l'échantillon provenant d'un patient suspecté de présenter un gliome est au moins 3 fois inférieur au niveau d'expression du susdit miARN dans l'échantillon sain de référence.

Par l'expression « niveau d'expression du susdit miARN dans l'échantillon provenant d'un patient suspecté de présenter un gliome est au moins 3 fois inférieur au niveau d'expression du susdit miARN dans l'échantillon sain de référence », on entend que le rapport calculé d'après la formule (IV ou V) est inférieur à une valeur de 0, 33. Cette valeur signifie une sous-expression dudit miARN dans l'échantillon provenant d'un patient suspecté de présenter un gliome vis-à-vis de l'échantillon sain de référence. En l'occurrence, dans ces formules, l'échantillon A est l'échantillon provenant d'un patient suspecté; l'échantillon B est l'échantillon sain de référence.

Selon un mode de réalisation particulièrement avantageux, la méthode de diagnostic *in vitro* de gliomes comprend :

- la mesure du niveau d'expression d'au moins un miARN choisi parmi hsa-miR-126, hsa-miR-15b, hsa-miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-151, hsa-miR-26b, hsa-miR-34b*, dans un échantillon biologique provenant d'un patient suspecté de présenter un gliome,
- la comparaison entre le niveau d'expression du susdit miARN dans le susdit échantillon, et le niveau d'expression du susdit miARN dans un échantillon sain de référence, et,
- la déduction que le patient suspecté présente un gliome, lorsque le niveau d'expression du susdit miARN dans l'échantillon provenant d'un patient suspecté de présenter un gliome et le niveau d'expression du susdit miARN dans l'échantillon sain de référence sont différents, le niveau d'expression du susdit miARN dans l'échantillon provenant d'un patient suspecté de présenter un gliome étant 3 fois, notamment 4 fois, plus particulièrement 5 fois, supérieur ou inférieur au niveau d'expression du susdit miARN dans l'échantillon sain de référence.

Les comparaisons peuvent aussi être réalisées entre les niveaux d'expression de deux miARNs provenant d'un même échantillon provenant d'un patient suspecté présenter un gliome. Il s'agit d'une comparaison intra-échantillon, qui repose sur l'identification de signatures propres à chaque type tumoral.

Ainsi, un deuxième mode de réalisation d'une méthode de diagnostic *in vitro* de gliomes comprend:

(i) la mesure du niveau d'expression d'au moins un miARN choisi parmi hsa-miR-126, hsa-miR-15b, hsa-miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-151, hsa-miR-26b, hsa-miR-34b*, hsa-miR-155, hsa-miR-210 ou hsa-miR-10a, dans un échantillon biologique provenant d'un patient suspecté de présenter un gliome,

(ii) la comparaison entre le niveau d'expression d'au moins un premier susdit miARN dans l'échantillon provenant d'un patient suspecté de présenter un gliome et le niveau d'expression d'au moins un deuxième susdit miARN dans le susdit échantillon, et

(iii) la déduction que le patient suspecté présente un gliome, lorsque le rapport entre le niveau d'expression du susdit premier miARN et le niveau d'expression du susdit deuxième miARN est différent de celui dans un abaque préétabli pour un échantillon sain de référence, les susdits premier miARN et deuxième miARN formant un couple de miARNs.

« Le rapport entre le niveau d'expression du susdit premier miARN et le niveau d'expression du susdit deuxième miARN » est un rapport obtenu d'après la formule (VI) ci-dessous dans le cas du dosage par PCR en temps réel:

$$\text{Rapport }_{\text{(hsa-miARN X/ hsa-miARN Y)}} = \text{Efficacité}^{\text{ - (Ct hsa-miARN X - Ct hsa-miARN Y)}}.$$

En l'occurrence, le premier miARN et le deuxième miARN, dont les niveaux d'expression sont comparés, sont représentés respectivement par miARN X et miARN Y. Les valeurs de Ct pour hsa-miARN X et Ct pour hsa-miARN Y, pour l'échantillon provenant d'un patient suspecté et pour l'échantillon sain de référence, sont obtenues directement par la PCR en temps réel. La valeur de l'efficacité est établie préalablement.

Dans le cas d'un dosage réalisé par une méthode autre que la PCR en temps réel, le rapport entre le niveau d'expression du susdit premier miARN et le niveau d'expression du susdit deuxième miARN » est un rapport obtenu d'après la formule VII :

$$\text{Rapport }_{\text{(hsa-miARN X/ hsa-miARN Y)}} = \text{intensité de signal de détection de miARN X / intensité de signal de détection de miARN Y}$$

En l'occurrence, le premier miARN et le deuxième miARN, dont les niveaux d'expression sont comparés, sont représentés respectivement par miARN X et miARN Y.

Par l'expression « abaque », on entend un tableau ou un graphique établi préalablement, constitué par une série de valeurs de référence.

Par « un abaque préétabli pour un échantillon sain de référence», on entend un tableau ou un graphique constitué par une série de valeurs de référence obtenues dans un échantillon sain de référence. Plus particulièrement, une telle valeur de référence, qui sert à établir un abaque, est un rapport$_{\text{(hsa-miARN X/ hsa-miARN Y)}}$ obtenu dans un échantillon sain de référence.

L'expression « le susdit premier miARN et deuxième miARN formant un couple de miARNs » signifie que la comparaison des niveaux d'expression des miARNs est réalisée entre ces deux miARNs. En fonction du nombre de couples de miARNs, un abaque sous forme d'un graphique peut être un graphique bidimensionnel, lorsqu'il s'agit de deux couples de miARNs, ou un graphique tridimensionnel, lorsqu'il s'agit de trois couples de miARNs. En effet, un abaque sous forme d'un graphique bidimensionnel ou tridimensionnel réalise une double ou triple séparation basée sur deux ou trois rapports pour un échantillon à tester, ce qui permet d'éviter une confusion de l'interprétation éventuellement apportée à un échantillon suspecté, lorsque cette interprétation est basée sur un seul rapport$_{\text{(hsa-miARN X/ hsa-miARN Y)}}$ obtenu d'un échantillon suspecté.

Cependant, il est possible d'adopter toute autre façon de l'établissement d'un abaque pour interpréter un rapport$_{\text{(hsa-miARN X/ hsa-miARN Y)}}$ obtenu pour un échantillon provenant d'un patient suspecté.

Après la mise en place d'un abaque, le recours à des échantillons sains n'est plus requis et tout nouvel échantillon peut être typé par le dosage d'un minimum de 2 miARNs seulement.

Dans un mode de réalisation avantageux les couples de miARNs utilisés sont les suivants : hsa-let 7a/hsa-miR127, hsa-let 7a/hsa-miR15b, hsa-let 7a/hsa-miR-34b*, hsa-miR126/hsa-let 7a, hsa-miR126/hsa-miR-34b*, hsa-miR127/hsa-let 7d, hsa-miR127/hsa-miR15b, hsa-miR134/hsa-let 7a, hsa-miR134/hsa-miR10a, hsa-miR151/hsa-let 7a, hsa-miR151/hsa-let 7d, hsa-miR151/hsa-let 7f, hsa-miR151/hsa-miR15b, hsa-miR15b/hsa-miR409, hsa-miR16/hsa-miR151, hsa-miR16/hsa-miR339, hsa-miR16/hsa-miR-34b*, hsa-miR17/hsa-miR339, hsa-miR17/hsa-miR-34b*, hsa-miR17/hsa-miR409, hsa-miR20a/hsa-miR339, hsa-miR26b/hsa-miR134, hsa-miR26b/hsa-miR-34b*, hsa-miR26b/hsa-miR409, hsa-miR339/hsa-let 7f, hsa-miR-34b*/hsa-let 7f, hsa-miR374/hsa-let 7f, hsa-miR374/hsa-miR127, hsa-miR374/hsa-miR134, hsa-miR374/hsa-miR339, hsa-miR374/hsa-miR-34b*, hsa-miR374/hsa-miR409, hsa-miR409/hsa-miR10a, hsa-miR155/hsa-miR134, hsa-miR155/hsa-miR127, hsa-miR126/hsa-miR409, hsa-miR127/hsa-let 7f, hsa-miR151/hsa-miR20a, hsa-miR16/hsa-miR134, hsa-miR126/hsa-miR339, hsa-miR151/hsa-miR10a, hsa-miR16/hsa-miR127, hsa-miR20a/hsa-let 7f, hsa-miR20a/hsa-miR409, hsa-miR155/hsa-miR409, hsa-miR339/hsa-let 7d, hsa-miR151/hsa-miR374, hsa-miR127/hsa-miR10a, hsa-miR15b/hsa-let 7f, hsa-miR20a/hsa-let 7a, hsa-miR210/hsa-miR155, hsa-miR210/hsa-let 7a, hsa-miR26b/hsa-miR-339, hsa-miR26b/hsa-miR-127, hsa-miR16/hsa-miR-409, hsa-miR127/hsa-miR-17, hsa-miR151/hsa-miR126, hsa-miR26b/hsa-miR151, hsa-miR20a/hsa-miR127, hsa-miR339/hsa-let 7a, hsa-miR26b/hsa-let 7f, hsa-miR126/hsa-miR134, hsa-miR20a/hsa-miR-34b*, hsa-let 7d/hsa-miR-34b*, hsa-miR15b/hsa-miR339, hsa-let 7d/hsa let 7f, hsa-miR10a/hsa let 7d, hsa-miR126/hsa let 7f, hsa-miR126/hsa-miR10a, hsa-miR126/hsa-miR155, hsa-miR127/hsa-miR126, hsa-miR134/hsa-miR17, hsa-miR134/hsa-miR409, hsa-miR151/hsa-miR17, hsa-miR155/hsa-miR339, hsa-miR15b/hsa-miR10a, hsa-miR15b/hsa-miR155, hsa-miR15b/hsa-miR-34b*, hsa-miR16/hsa-miR10a, hsa-miR16/hsa-miR126, hsa-miR16/hsa-miR155, hsa-miR16/hsa-miR15b, hsa-miR16/hsa-miR20a, hsa-miR17/hsa let 7d, hsa-miR20a/hsa-miR10a, hsa-miR210/hsa-miR127, hsa-miR210/hsa-miR134, hsa-miR210/hsa-miR339, hsa-miR210/hsa-miR409, hsa-miR26b/hsa let 7a, hsa-miR26b/hsa-miR10a, hsa-miR26b/hsa-miR155, hsa-miR26b/hsa-miR15b, hsa-miR374/hsa-miR10a, hsa-miR10a/hsa-miR210, hsa-miR10a/hsa-miR339, hsa-miR10a/hsa-miR-34b*, hsa-miR155/hsa let 7f, hsa-miR16/hsa let 7a, hsa-miR16/hsa-miR210, hsa-miR210/hsa let 7d, hsa-miR210/hsa-miR126, hsa-miR210/hsa-miR15b, hsa-miR210/hsa-miR17, hsa-miR210/hsa-miR20a, hsa-miR210/hsa-miR374, hsa-miR26b/hsa-miR210, hsa-miR16/hsa let 7f, hsa-miR16/hsa-miR374.

Les ratios inverses de ceux cités dans la liste ci-dessus gardent la même signification pour le diagnostic, c'est-à-dire que l'on peut utiliser ces ratios ou leurs inverses.

Une déduction que le patient présente au moins une tumeur gliale est faite, lorsque le rapport du niveau d'expression de deux miARNs d'un couple est différent par rapport au susdit rapport établi dans un échantillon sain de référence.

A titre d'exemple, la colonne 2 du tableau 4 illustre un abaque préétabli sous forme d'un tableau, dans un tissu sain de référence, pour chaque couple de miARNs. Selon l'invention, il est aussi possible d'établir un graphique bidimensionnel ou tridimensionnel, utilisant deux ou plusieurs couples de miARNs choisis parmi les couples de miARNs mentionnés dans la partie ci-dessus.

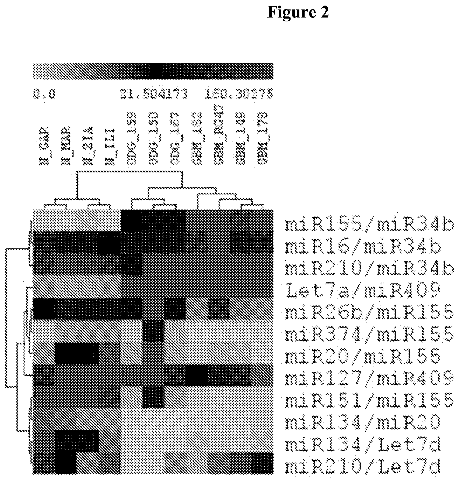

A titre d'exemple, les figures 2-6 sont des abaques sous forme de graphique bidimensionnel, dans lesquels les points losanges représentant des tissus normaux constituent une zone encadrée par le rectangle tracé en tiret-pointés. Après le dosage de miARNs concernés et le calcul des rapports tels que ceux qui sont utilisés pour l'établissement de l'abaque, la nature du tissu suspecté peut être facilement repérée sur cet abaque, et on peut déduire que le tissu suspecté est un tissu tumoral, lorsque le point, représentant le tissu suspecté, n'entre pas dans la zone encadrée par le rectangle tracé en tiret-pointés.

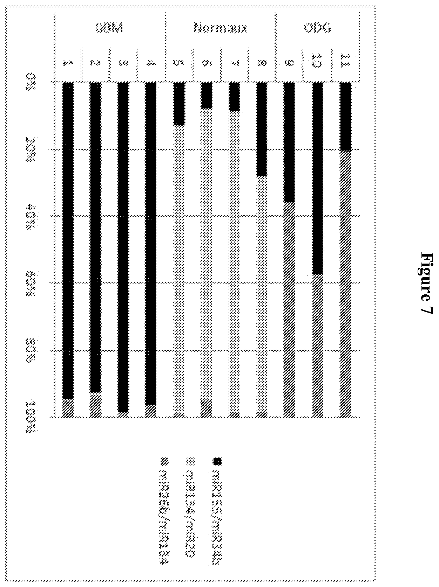

A titre d'exemple également, la figure 7 basée sur l'utilisation de 3 couples différents de miARNs dont les valeurs respectives sont exprimées en pourcentage de la somme des valeurs de ces 3 couples, permet de repérer aisément les tissus sains par rapport aux tissus tumoraux puisqu'ils se caractérisent - à la différences des tissus tumoraux - par des valeurs élevées du couple hsa-miR134/hsa-miR20a (barres en gris pâle) par rapport aux valeurs des deux autres couples dans un tissu donné.

Dans un mode de réalisation avantageux, la déduction que le patient suspecté présente un gliome est faite lorsque le rapport entre le niveau d'expression du susdit premier miARN et le niveau d'expression du susdit deuxième miARN est 2 fois, notamment 3 fois, plus particulièrement 4 fois, supérieur à la valeur dudit rapport dans un abaque préétabli pour un échantillon sain de référence.

Dans un autre mode de réalisation avantageux, la déduction que le patient suspecté présente un gliome est faite lorsque le rapport entre le niveau d'expression du susdit premier miARN et le niveau d'expression du susdit deuxième miARN est 2 fois, notamment 3 fois, plus particulièrement 4 fois, inférieur à la valeur dudit rapport dans un abaque préétabli pour un échantillon sain de référence.

Un mode de réalisation avantageux d'une méthode de diagnostic *in vitro* de gliomes selon l'invention comprend :

(i) la mesure du rapport du niveau d'expression d'au moins un couple de miARNs choisi parmi hsa-let 7a/hsa-miR127, hsa-let 7a/hsa-miR15b, hsa-let 7a/hsa-miR-34b*, hsa-miR126/hsa-let 7a, hsa-miR126/hsa-miR-34b*, hsa-miR127/hsa-let 7d, hsa-miR127/hsa-miR15b, hsa-miR134/hsa-let 7a, hsa-miR134/hsa-miR10a, hsa-miR151/hsa-let 7a, hsa-miR151/hsa-let 7d, hsa-miR151/hsa-let 7f, hsa-miR151/hsa-miR15b, hsa-miR15b/hsa-miR409, hsa-miR16/hsa-miR151, hsa-miR16/hsa-miR339, hsa-miR16/hsa-miR-34b*, hsa-miR17/hsa-miR339, hsa-miR17/hsa-miR-34b*, hsa-miR17/hsa-miR409, hsa-miR20a/hsa-miR339, hsa-miR26b/hsa-miR134, hsa-miR26b/hsa-miR-34b*, hsa-miR26b/hsa-miR409, hsa-miR339/hsa-let 7f, hsa-miR-34b*/hsa-let 7f, hsa-miR374/hsa-let 7f, hsa-miR374/hsa-miR127, hsa-miR374/hsa-miR134, hsa-miR374/hsa-miR339, hsa-miR374/hsa-miR-34b*, hsa-miR374/hsa-miR409, hsa-miR409/hsa-miR10a, hsa-miR155/hsa-miR134, hsa-miR155/hsa-miR127, hsa-miR126/hsa-miR409, hsa-miR127/hsa-let 7f, hsa-miR151/hsa-miR20a, hsa-miR16/hsa-miR134, hsa-miR126/hsa-miR339, hsa-miR151/hsa-miR10a, hsa-miR16/hsa-miR127, hsa-miR20a/hsa-let 7f, hsa-miR20a/hsa-miR409, hsa-miR155/hsa-miR409, hsa-miR339/hsa-let 7d, hsa-miR151/hsa-miR374, hsa-miR127/hsa-miR10a, hsa-miR15b/hsa-let 7f, hsa-miR20a/hsa-let 7a, hsa-miR210/hsa-miR155, hsa-miR210/hsa-let 7a, hsa-miR26b/hsa-miR-339, hsa-miR26b/hsa-miR-127, hsa-miR16/hsa-miR-409, hsa-miR127/hsa-miR-17, hsa-miR151/hsa-miR126, hsa-miR26b/hsa-miR151, hsa-miR20a/hsa-miR127, hsa-miR339/hsa-let 7a, hsa-miR26b/hsa-let 7f, hsa-miR126/hsa-miR134, hsa-miR20a/hsa-miR-34b*, hsa-let 7d/hsa-miR-34b*, hsa-miR15b/hsa-miR339, hsa-let 7d/hsa let 7f, hsa-miR10a/hsa let 7d, hsa-miR126/hsa let 7f, hsa-miR126/hsa-miR10a, hsa-miR126/hsa-miR155, hsa-miR127/hsa-miR126, hsa-miR134/hsa-miR17, hsa-miR134/hsa-miR409, hsa-miR151/hsa-miR17, hsa-miR155/hsa-miR339, hsa-miR15b/hsa-miR10a, hsa-miR15b/hsa-miR155, hsa-miR15b/hsa-miR-34b*, hsa-miR16/hsa-miR10a, hsa-miR16/hsa-miR126, hsa-miR16/hsa-miR155, hsa-miR16/hsa-miR15b, hsa-miR16/hsa-miR20a, hsa-miR17/hsa let 7d, hsa-miR20a/hsa-miR10a, hsa-miR210/hsa-miR127, hsa-miR210/hsa-miR134, hsa-miR210/hsa-miR339, hsa-miR210/hsa-miR409, hsa-miR26b/hsa let 7a, hsa-miR26b/hsa-miR10a, hsa-miR26b/hsa-miR155, hsa-miR26b/hsa-miR15b, hsa-miR374/hsa-miR10a, hsa-miR10a/hsa-miR210, hsa-miR10a/hsa-miR339, hsa-miR10a/hsa-miR-34b*, hsa-miR155/hsa let 7f, hsa-miR16/hsa let 7a, hsa-miR16/hsa-miR210, hsa-miR210/hsa let 7d, hsa-miR210/hsa-miR126, hsa-miR210/hsa-miR15b, hsa-miR210/hsa-miR17, hsa-miR210/hsa-miR20a, hsa-miR210/hsa-miR374, hsa-miR26b/hsa-miR210, hsa-miR16/hsa let 7f, hsa-miR16/hsa-miR374, dans un échantillon biologique provenant d'un patient suspecté de présenter un gliome,

(ii) la déduction que le patient suspecté présente un gliome, lorsque le susdit rapport entre le niveau d'expression du susdit premier miARN et le niveau d'expression du susdit deuxième miARN est 2 fois, notamment 3 fois, plus particulièrement 4 fois, supérieur ou inférieur à la valeur dudit rapport dans un abaque préétabli dans un échantillon sain de référence.

Dans un mode de réalisation particulier, l'échantillon biologique provenant d'un patient suspecté de présenter un gliome est un tissu cérébral obtenu par exérèse, ou par biopsie. Ladite méthode comprend :

(i) la mesure du rapport du niveau d'expression d'au moins un couple de miARNs choisi parmi hsa-let 7a/hsa-miR127, hsa-let 7a/hsa-miR15b, hsa-let 7a/hsa-miR-34b*, hsa-miR126/hsa-let 7a, hsa-miR126/hsa-miR-34b*, hsa-miR127/hsa-let 7d, hsa-miR127/hsa-miR15b, hsa-miR134/hsa-let 7a, hsa-miR134/hsa-miR10a, hsa-miR151/hsa-let 7a, hsa-miR151/hsa-let 7d, hsa-miR151/hsa-let 7f, hsa-miR151/hsa-miR15b, hsa-miR15b/hsa-miR409, hsa-miR16/hsa-miR151, hsa-miR16/hsa-miR339, hsa-miR16/hsa-miR-34b*, hsa-miR17/hsa-miR339, hsa-miR17/hsa-miR-34b*, hsa-miR17/hsa-miR409, hsa-miR20a/hsa-miR339, hsa-miR26b/hsa-miR134, hsa-miR26b/hsa-miR-34b*, hsa-miR26b/hsa-miR409, hsa-miR339/hsa-let 7f, hsa-miR-34b*/hsa-let 7f, hsa-miR374/hsa-let 7f, hsa-miR374/hsa-miR127, hsa-miR374/hsa-miR134, hsa-miR374/hsa-miR339, hsa-miR374/hsa-miR-34b*, hsa-miR374/hsa-miR409, hsa-miR409/hsa-miR10a, hsa-miR155/hsa-miR134, hsa-miR155/hsa-miR127, hsa-miR126/hsa-miR409, hsa-miR127/hsa-let 7f, hsa-miR151/hsa-miR20a, hsa-miR16/hsa-miR134, hsa-miR126/hsa-miR339, hsa-miR151/hsa-miR10a, hsa-miR16/hsa-miR127, hsa-miR20a/hsa-let 7f, hsa-miR20a/hsa-miR409, hsa-miR155/hsa-miR409, hsa-miR339/hsa-let 7d, hsa-miR151/hsa-miR374, hsa-miR127/hsa-miR10a, hsa-miR15b/hsa-let 7f, hsa-miR20a/hsa-let 7a, hsa-miR210/hsa-miR155, hsa-miR210/hsa-let 7a, hsa-miR26b/hsa-miR-339, hsa-miR26b/hsa-miR-127, hsa-miR16/hsa-miR-409, hsa-miR127/hsa-miR-17, hsa-miR151/hsa-miR126, hsa-miR26b/hsa-miR151, hsa-miR20a/hsa-miR127, hsa-miR339/hsa-let 7a, hsa-miR26b/hsa-let 7f, hsa-miR126/hsa-miR134, hsa-miR20a/hsa-miR-34b*, hsa-let 7d/hsa-miR-34b*, hsa-miR15b/hsa-miR339, hsa-let 7d/hsa let 7f, hsa-miR10a/hsa let 7d, hsa-miR126/hsa let 7f, hsa-miR126/hsa-miR10a, hsa-miR126/hsa-miR155, hsa-miR127/hsa-miR126, hsa-miR134/hsa-miR17, hsa-miR134/hsa-miR409, hsa-miR151/hsa-miR17, hsa-miR155/hsa-miR339, hsa-miR15b/hsa-miR10a, hsa-miR15b/hsa-miR155, hsa-miR15b/hsa-miR-34b*, hsa-miR16/hsa-miR10a, hsa-miR16/hsa-miR126, hsa-miR16/hsa-miR155, hsa-miR16/hsa-miR15b, hsa-miR16/hsa-miR20a, hsa-miR17/hsa let 7d, hsa-miR20a/hsa-miR10a, hsa-miR210/hsa-miR127, hsa-miR210/hsa-miR134, hsa-miR210/hsa-miR339, hsa-miR210/hsa-miR409, hsa-miR26b/hsa let 7a, hsa-miR26b/hsa-miR10a, hsa-miR26b/hsa-miR155, hsa-miR26b/hsa-miR15b, hsa-miR374/hsa-miR10a, hsa-miR10a/hsa-miR210, hsa-miR10a/hsa-miR339, hsa-miR10a/hsa-miR-34b*, hsa-miR155/hsa let 7f, hsa-miR16/hsa let 7a, hsa-miR16/hsa-miR210, hsa-miR210/hsa let 7d, hsa-miR210/hsa-miR126, hsa-miR210/hsa-miR15b, hsa-miR210/hsa-miR17, hsa-miR210/hsa-miR20a, hsa-miR210/hsa-miR374, hsa-miR26b/hsa-miR210, hsa-miR16/hsa let 7f, hsa-miR16/hsa-miR374, dans un tissu biologique provenant d'un tissu suspecté de présenter un caractère tumoral,

(ii) la déduction du caractère tumoral du tissu suspecté, lorsque le susdit rapport entre le niveau d'expression du susdit premier miARN et le niveau d'expression du susdit deuxième miARN est 2 fois, notamment 3 fois, plus particulièrement 4 fois, supérieur ou inférieur à la valeur dudit rapport dans un abaque préétabli dans un tissu sain de référence.

La description a également pour objet une méthode de diagnostic *in vitro* de gliomes, permettant de distinguer un glioblastome d'un oligodendrogliome.

Dans un premier mode de réalisation, cette méthode repose uniquement sur la mise en oeuvre de 5 miARNs repérés par les Inventeurs, à savoir hsa-miR-155, hsa-miR-210 ou hsa-miR-10a, hsa-miR-409 ou hsa-miR-134, dont les niveaux d'expression sont systématiquement plus élevés dans les glioblastomes par rapport à ceux dans les oligodendrogliomes.

Dans ce mode d'analyse, il s'agit de réaliser des comparaisons inter-échantillon.

Cette méthode comprend :

- la mesure du niveau d'expression d'au moins un miARN choisi parmi hsa-miR-155, hsa-miR-210, hsa-miR-10a, hsa-miR-409 ou hsa-miR-134, dans un échantillon biologique provenant d'un patient suspecté de présenter un glioblastome ou un oligodendrogliome,
- la comparaison entre :

    - le niveau d'expression du susdit miARN dans ledit échantillon biologique et

        * respectivement le niveau d'expression du susdit miARN dans un échantillon biologique de référence provenant d'un patient présentant un glioblastome, et
        * respectivement le niveau d'expression du susdit miARN dans un échantillon biologique de référence provenant d'un patient présentant un oligodendrogliome, et

- la déduction :

    - soit que le patient suspecté présente un glioblastome, lorsque le niveau d'expression du susdit miARN dans l'échantillon provenant d'un patient suspecté de présenter un glioblastome ou un oligodendrogliome est égal, dans la limite de 20% en plus ou 20% en moins, au niveau d'expression du susdit miARN dans l'échantillon de référence provenant d'un patient présentant un glioblastome,
    - soit que le patient suspecté présente un oligodendrogliome, lorsque le niveau d'expression du susdit miARN dans l'échantillon provenant d'un patient suspecté de présenter un glioblastome ou un oligodendrogliome est égal, dans la limite de 20% en plus ou 20% en moins, au niveau d'expression du susdit miARN dans l'échantillon de référence provenant d'un patient présentant un oligodendrogliome.

« Un échantillon biologique de référence provenant d'un patient suspecté de présenter un glioblastome ou un oligodendrogliome » est un échantillon prévelé d'un patient dont la présence d'une tumeur gliale est déjà confirmé par une méthode décrite dans la partie susmentionnée ou par toute autre méthode conventionnelle connue de l'homme du métier, telle qu'une analyse histologique, ou un scanner. Néanmoins, la nature ou la classification exacte de la tumeur gliale est encore inconnue.

Un échantillon de référence provenant d'un patient présentant un glioblastome ou un oligodendrogliome peut être un tissu de glioblastome ou un oligodendrogliome obtenu par exérèse chez un patient et dont le type de tumeur a été certifié par l'analyse anatomo-pathologique, ou un échantillon sanguin prélevé d'un patient dont la présence d'un glioblastome ou un oligodendrogliome est confirmée. Idéalement, le diagnostic peut également être confirmé par des analyses moléculaires telles que par exemple l'analyse des profils d'expression de gènes de ces tissus tumoraux. Les tumeurs sont ensuite caractérisées en utilisant la méthode de classification de Li et al (Li A, Walling J, Ahn S, Kotliarov Y, Su Q, Quezado M, Oberholtzer JC, Park J, Zenklusen JC, Fine HA. Cancer Res. 2009 Mar 1;69(5):2091-9) qui repose sur l'utilisation des niveaux d'expression d'un jeu de 54 gènes. Les glioblastomes ou oligodendrogliomes sont classés respectivement dans les groupes G et O de la publication sus-mentionnée.

Etant donné que les niveaux d'expression de 5 miARNs mis en oeuvre dans cette méthode de détermination de la nature d'une tumeur gliale sont systématiquement plus élevés dans les glioblastomes par rapport aux oligodendrogliomes, lorsque l'échantillon suspecté provient d'un patient atteint d'un glioblastome, le niveau d'expression de l'un susdit miARN dans l'échantillon suspecté est supérieur au niveau d'expression dudit miARN dans l'échantillon de référence provenant d'un patient présentant un oligodendrogliome, et proche du niveau d'expression dudit miARN dans l'échantillon de référence provenant d'un patient présentant un glioblastome.

En revanche, lorsque l'échantillon suspecté provient d'un patient atteint d'un oligodendrogliome, le niveau d'expression de l'un susdit miARN dans l'échantillon suspecté est inférieur au niveau d'expression dudit miARN dans l'échantillon de référence provenant d'un patient présentant un glioblastome, et proche du niveau d'expression dudit miARN dans l'échantillon de référence provenant d'un patient présentant un oligodendrogliome.

Les niveaux d'expression des miARNs peuvent être mesurés par hybridation sur « puces à ADN », par séquençage, ou par PCR en temps réel.

Lorsque le dosage des niveaux d'expression des miARNs est effectué par hybridation sur « puces à ADN » ou par séquençage, la comparaison entre le niveau d'expression d'un miARN dans l'échantillon suspecté et celui dans un échantillon de référence (glioblastomes ou oligodendrogliomes) est évaluée d'après la formule IV :

$$\text{Quantité de miARNx normalisée (échantillon A)} / \text{Quantité de miARNx normalisée (échantillon B)}$$

En l'occurrence, A représente l'échantillon suspecté et B représente un échantillon de référence provenant d'un patient présentant un glioblastome ou un oligodendrogliome.

Lorsque le dosage des niveaux d'expression est obtenu par PCR en temps réel, la comparaison entre le niveau d'expression d'un miARN dans l'échantillon suspecté et celui dans un échantillon de référence provenant d'un patient présentant un glioblastome ou un oligodendrogliome est évaluée selon la formule V ci-après

$$\text{Rapport (échantillon A/échantillon B)} = \text{Efficacité}^{-((Ct\ hsa\text{-}miARN\ X\ -\ Ct\ RNU24)\text{échantillon A}-(Ct\ hsa\text{-}miARNX\ -\ Ct\ RNU24)\text{échantillon B})},$$

où l'échantillon A est l'échantillon suspecté et l'échantillon B est l'échantillon de référence provenant d'un patient présentant un glioblastome ou un oligodendrogliome.

Ainsi, la déduction que le patient présente un glioblastome est faite lorsque :

- le niveau d'expression du susdit miARN dans l'échantillon provenant d'un patient suspecté de présenter un glioblastome ou un oligodendrogliome est au moins 3 fois supérieur au niveau d'expression du susdit miARN dans l'échantillon de référence provenant d'un patient présentant un oligodendrogliome, et
- le niveau d'expression du susdit miARN dans l'échantillon provenant d'un patient suspecté de présenter un glioblastome ou un oligodendrogliome est égal, dans la limite de 20% en plus ou 20% en moins, au niveau d'expression du susdit miARN dans l'échantillon de référence provenant d'un patient présentant un glioblastome.

L'expression « le niveau d'expression du susdit miARN dans l'échantillon provenant d'un patient suspecté de présenter un glioblastome ou un oligodendrogliome est au moins 3 fois supérieur au niveau d'expression du susdit miARN dans l'échantillon de référence provenant d'un patient présentant un oligodendrogliome » signifie que le rapport entre le niveau d'expression d'un miARN dans l'échantillon provenant d'un patient suspecté et le niveau d'expression du susdit miARN dans un échantillon de référence provenant d'un patient présentant un oligodendrogliome, obtenu d'après la formule IV ou V, a une valeur égale ou supérieure à 3.

En l'occurrence, dans ces formules, l'échantillon A est l'échantillon suspecté ; l'échantillon B est l'échantillon de référence provenant d'un patient présentant un oligodendrogliome.

L'expression « le niveau d'expression du susdit miARN dans l'échantillon provenant d'un patient suspecté de présenter un glioblastome ou un oligodendrogliome est égal, dans la limite de 20% en plus ou 20% en moins, au niveau d'expression du susdit miARN dans l'échantillon de référence provenant d'un patient présentant un glioblastome» signifie que le rapport entre le niveau d'expression d'un miARN dans l'échantillon suspecté et le niveau d'expression du susdit miARN dans échantillon de référence provenant d'un patient présentant un glioblastome, obtenu d'après la formule IV ou V, a une valeur égale à 1 ou comprise entre 0,8 et 1,2.

En l'occurrence, dans ces formules, l'échantillon A est l'échantillon suspecté ; l'échantillon B est l'échantillon de référence provenant d'un patient présentant un glioblastome.

La déduction que le patient présente un oligodendrogliome est faite lorsque :

- le niveau d'expression du susdit miARN dans l'échantillon provenant d'un patient suspecté de présenter un glioblastome ou un oligodendrogliome est au moins 3 fois inférieur au niveau d'expression du susdit miARN dans l'échantillon de référence provenant d'un patient présentant un glioblastome, et
- le niveau d'expression du susdit miARN dans l'échantillon provenant d'un patient suspecté de présenter un glioblastome ou un oligodendrogliome est égal, dans la limite de 20% en plus ou 20% en moins, au niveau d'expression du susdit miARN dans l'échantillon de référence provenant d'un patient présentant un oligodendrogliome.

L'expression « le niveau d'expression du susdit miARN dans l'échantillon provenant d'un patient suspecté de présenter un glioblastome ou un oligodendrogliome est au moins 3 fois inférieur au niveau d'expression du susdit miARN dans l'échantillon de référence provenant d'un patient présentant un glioblastome » signifie que le rapport entre le niveau d'expression d'un miARN dans l'échantillon suspecté et le niveau d'expression du susdit miARN dans un échantillon de référence provenant d'un patient présentant un glioblastome, obtenu d'après la formule IV ou V, a une valeur égale ou inférieure à 0,33.

En l'occurrence, dans ces formules, l'échantillon A est l'échantillon suspecté ; l'échantillon B est l'échantillon de référence provenant d'un patient présentant un glioblastome.

L'expression « le niveau d'expression du susdit miARN dans l'échantillon provenant d'un patient suspecté de présenter un glioblastome ou un oligodendrogliome est égal, dans la limite de 20% en plus ou 20% en moins, au niveau d'expression du susdit miARN dans l'échantillon de référence provenant d'un patient présentant un oligodendrogliome» signifie que le rapport entre le niveau d'expression d'un miARN dans l'échantillon suspecté et le niveau d'expression du susdit miARN dans un échantillon de référence provenant d'un patient présentant un oligodendrogliome, obtenu d'après la formule IV ou V, a une valeur égale à 1 ou comprise entre 0,8 et 1,2.

En l'occurrence, dans ces formules, l'échantillon A est l'échantillon suspecté ; l'échantillon B est l'échantillon de référence provenant d'un patient présentant un oligodendrogliome.

En résumé, lorsque l'échantillon suspecté provient d'un patient atteint d'un glioblastome, le rapport entre le niveau d'expression d'un miARN dans l'échantillon suspecté et le niveau d'expression du susdit miARN dans l'échantillon de référence provenant d'un patient présentant un glioblastome est proche de la valeur de 1, et le rapport entre le niveau d'expression d'un miARN dans l'échantillon suspecté et le niveau d'expression du susdit miARN dans l'échantillon de référence provenant d'un patient présentant un oligodendrogliome est supérieur à 3.

En revanche, lorsque l'échantillon suspecté provient d'un patient atteint d'un oligodendrogliome, le rapport entre le niveau d'expression d'un miARN dans le tissu suspecté et le niveau d'expression du susdit miARN dans l'échantillon de référence provenant d'un patient présentant un oligodendrogliome est proche de la valeur de 1, et le rapport entre le niveau d'expression d'un miARN dans l'échantillon suspecté et le niveau d'expression du susdit miARN dans l'échantillon de référence provenant d'un patient présentant un glioblastome est inférieur à 0,33.

Plus particulièrement, cette méthode comprend donc les étapes suivantes :

(i) la mesure du niveau d'expression d'au moins un miARN choisi parmi hsa-miR-155, hsa-miR-210 ou hsa-miR-10a, hsa-miR-409 ou hsa-miR-134, dans un échantillon biologique provenant d'un patient suspecté de présenter un glioblastome ou un oligodendrogliome,

(ii) la comparaison entre :

- le niveau d'expression du susdit miARN dans l'échantillon provenant d'un patient suspecté de présenter un glioblastome ou un oligodendrogliome et

  * respectivement le niveau d'expression du susdit miARN dans l'échantillon de référence provenant d'un patient présentant un glioblastome, et
  * respectivement le niveau d'expression du susdit miARN l'échantillon de référence provenant d'un patient présentant un oligodendrogliome,

(iii) la déduction :

- soit que le susdit patient présente un glioblastome,

  * lorsque le niveau d'expression du susdit miARN dans l'échantillon provenant d'un patient suspecté de présenter un glioblastome ou un oligodendrogliome est égal, dans la limite de 20% en plus ou 20% en moins, au niveau d'expression du susdit miARN dans l'échantillon de référence provenant d'un patient présentant un glioblastome, et
  * lorsque le niveau d'expression du susdit miARN dans l'échantillon provenant d'un patient suspecté de présenter un glioblastome ou un oligodendrogliome est au moins 3 fois supérieur au niveau d'expression du susdit miARN dans l'échantillon provenant d'un patient présentant un oligodendrogliome,

- soit que le patient présente un oligodendrogliome,

  * lorsque le niveau d'expression du susdit miARN dans l'échantillon provenant d'un patient suspecté de présenter un glioblastome ou un oligodendrogliome est égal, dans la limite de 20% en plus ou 20% en moins, au niveau d'expression du susdit miARN dans l'échantillon de référence provenant d'un patient présentant un oligodendrogliome, et

* lorsque le niveau d'expression du susdit miARN dans l'échantillon provenant d'un patient suspecté de présenter un glioblastome ou un oligodendrogliome est au moins 3 fois inférieur au niveau d'expression du susdit miARN dans l'échantillon provenant d'un patient présentant un glioblastome.

Dans un mode de réalisation particulier, l'échantillon biologique provenant d'un patient suspecté de présenter un gliome est un tissu cérébral obtenu par exérèse, ou par biopsie. Ladite méthode comprend :

(i) la mesure du niveau d'expression d'au moins un miARN choisi parmi hsa-miR-155, hsa-miR-210 ou hsa-miR-10a, hsa-miR-409 ou hsa-miR-134, dans un tissu biologique provenant d'un tissu tumoral suspecté d'être un tissu de glioblastome ou un tissu d'oligodendrogliome,

(ii) la comparaison entre :

- le niveau d'expression du susdit miARN dans le tissu suspecté et

  * respectivement le niveau d'expression du susdit miARN dans un tissu de glioblastome de référence, et
  * respectivement le niveau d'expression du susdit miARN dans un tissu d'oligodendrogliome de référence, et

(iii) la déduction :

- soit que le tissu suspecté est un glioblastome,

  * lorsque le niveau d'expression du susdit miARN dans le tissu suspecté est égal, dans la limite de 20% en plus ou en moins, notamment 15%, plus particulièrement 10%, au niveau d'expression du susdit miARN dans le tissu de glioblastome de référence, et
  * lorsque le niveau d'expression du susdit miARN dans le tissu suspecté est au moins 3 fois supérieur au niveau d'expression du susdit miARN dans le tissu d'oligodendrogliome de référence,

- soit que le tissu suspecté est un oligodendrogliome,

  * lorsque le niveau d'expression du susdit miARN dans le tissu suspecté est égal, dans la limite de 20% en plus ou en moins, notamment 15%, plus particulièrement 10%, au niveau d'expression du susdit miARN dans le tissu d'oligodendrogliome de référence, et
  * lorsque le niveau d'expression du susdit miARN dans le tissu suspecté est au moins 3 fois inférieur au niveau d'expression du susdit miARN dans le tissu de glioblastome de référence.

Un deuxième mode de réalisation d'une méthode de diagnostic *in vitro* de gliomes, permettant de distinguer un glioblastome d'un oligodendrogliome, consiste en des comparaisons intra-échantillons. Cette méthode est basée sur une comparaison intra-échantillon du niveau d'expression de deux miARNs, dont l'un fait partie des 5 miARNs spécifiques dont les niveaux d'expression sont plus élevés dans les glioblastomes par rapport à ceux dans les oligodendrogliomes, l'autre est choisi parmi les 14 autres miARN restant de l'ensemble de 19 miARNs définis précédemment.

Cette méthode comprend:

(i) la mesure du niveau d'expression des miARNs dans au moins un couple de miARNs constitué par:

* un miARN choisi parmi hsa-miR-155, hsa-miR-210, hsa-miR-10a, hsa-miR-409 ou hsa-miR-134, et
* un miARN choisi parmi hsa-miR-126, hsa-miR-15b, hsa-miR-16, hsa-miR-17, hsa-miR-20a, hsa-let7a, hsa-let 7f, hsa-let 7d, hsa-miR-374, , hsa-miR-339, hsa-miR-127, hsa-miR-151, hsa-miR-26b , hsa-miR-34b*,

pour un échantillon biologique provenant d'un patient suspecté de présenter un glioblastome ou un oligodendrogliome,

ladite mesure permettant d'établir le rapport du niveau d'expression pour un susdit couple de miARNs,

(ii) la comparaison entre :

- le susdit rapport du niveau d'expression pour un couple de miARNs et,
- celui représenté dans un abaque préétabli pour un échantillon sain de référence, un échantillon de référence provenant d'un patient présentant un glioblastome, et un échantillon de référence provenant d'un patient présentant un oligodendrogliome,

(iii) la déduction :

* soit que le patient présente un glioblastome, lorsque la différence entre le rapport obtenu dans l'étape (i) et le rapport dans un abaque préétabli dans un échantillon de référence provenant d'un patient présentant un glioblastome est inférieure à 20%, notamment 15%, plus particulièrement 10%, vis-à-vis du rapport dans un abaque préétabli,
* soit que le patient présente un oligodendrogliome, lorsque la différence entre le rapport obtenu dans l'étape (i) et le rapport dans un abaque préétabli dans un échantillon de référence provenant d'un patient présentant un oligodendrogliome est inférieure à 20%, notamment 15%, plus particulièrement 10%, vis-à-vis du rapport dans un abaque préétabli.

Dans cette méthode, la comparaison intra-échantillon du niveau d'expression de deux miARNs est exprimée par un $\text{rapport}_{(hsa\text{-}miARN\ X/\ hsa\text{-}miARN\ Y)}$, calculé selon les formules (VI ou VII).

miARN X est choisi parmi hsa-miR-155, hsa-miR-210, hsa-miR-10a, hsa-miR-409 ou hsa-miR-134, et miARN Y est choisi parmi l'ensemble des 14 miARNs définis précédemment. Dans cette méthode l'utilisation de l'inverse des ratios sus-mentionnés garde toute sa valeur pour l'identification des échantillons.

La différence entre ce rapport et un abaque préétabli est exprimée par une valeur donnée en pourcentage, calculée selon la formule (VIII) :

$$|\text{Rapport}_{(hsa\text{-}miARN\ X/hsa\text{-}miARN\ Y)} - \text{Rapport}_{abaque}| \times 100 / \text{Rapport}_{abaque}.$$

$\text{Rapport}_{abaque}$, qui se trouve dans un abaque préétabli, est un $\text{rapport}_{(hsa\text{-}miARN\ X/\ hsa\text{-}miARN\ Y)}$ obtenu dans un échantillon de référence provenant d'un patient présentant un glioblastome ou un oligodendrogliome.

Cette méthode utilisant un abaque préétabli permet d'éviter le recours à des échantillons sains ou tumoraux de référence. Tout nouvel échantillon peut être analysé par le dosage de seulement quelques miARNs.

A titre d'exemple, les colonnes 3 et 4 du tableau 4 donnent un abaque préétabli respectivement dans l'échantillon de référence provenant d'un patient présentant un glioblastome ou un oligodendrogliome, pour chaque couple de miARNs.

Un abaque peut également être présenté sous forme d'un graphique bidimensionnel ou tridimensionnel.

Dans un abaque à la forme d'un graphique bidimensionnel, tel que l'abaque illustré aux figures 2-6, à titre d'exemple, deux séries de rapport obtenus dans le tissu de glioblastome de référence donnent une zone encadrée par un rectangle en tracé pointillé; deux séries de rapport obtenus dans le tissu d'oligodendrogliome référence donnent une zone encadrée par un rectangle tracé avec des tirets.

On peut déduire qu'un tissu suspecté est un tissu de glioblastomes, lorsque la position fixée par deux rapports obtenus respectivement de ce tissu suspecté est incluse dans le rectangle en tracé pointillé couvrant les carrés représentant les glioblastomes de référence.

En revanche, un tissu suspecté est un tissu d'oligodendrogliomes, lorsque la position fixée par deux rapports obtenus respectivement de ce tissu suspecté entre dans le rectangle tracé avec des tirets couvrant les triangles représentant les oligodendrogliomes de référence.

Dans un mode de réalisation avantageux, les couples de miARNs utilisés sont les suivants : hsa-miR127/hsa-miR409, hsa-miR210/hsa-miR-34b*, hsa-let 7f/hsa-miR10a, hsa-miR155/hsa-miR17, hsa-miR155/hsa-miR374, hsa-let 7a/hsa-miR 409, hsa-let 7d/hsa-miR409, hsa-miR134/hsa-let 7f, hsa-let 7f/hsa-miR409, hsa-miR134/hsa-let 7d, hsa-miR151/hsa-miR155, hsa-miR155/hsa-miR-34b*, hsa-miR15b/hsa-miR134, hsa-miR134/hsa-miR20a, hsa-miR210/hsa-let 7f, hsa-miR20/hsa-miR155.

Dans un mode de réalisation particulier, l'échantillon biologique provenant d'un patient suspecté de présenter un gliome est un tissu cérébral obtenu par exérèse, ou par biopsie. Ladite méthode comprend :

(i) la mesure du niveau d'expression des miARNs d'un couple choisi parmi hsa-miR127/hsa-miR409, hsa-miR210/hsa-miR-34b*, hsa-let 7f/hsa-miR10a, hsa-miR155/hsa-miR17, hsa-miR155/hsa-miR374, hsa-let 7a/hsa-miR 409, hsa-let 7d/hsa-miR409, hsa-miR134/hsa-let 7f, hsa-let 7f/hsa-miR409, hsa-miR134/hsa-let 7d, hsa-miR151/hsa-miR155, hsa-miR155/hsa-miR-34b*, hsa-miR15b/hsa-miR134, hsa-miR134/hsa-miR20a, hsa-miR210/hsa-let 7f, hsa-miR20/hsa-miR155, pour un tissu biologique provenant d'un tissu tumoral suspecté d'être un tissu de glioblastome ou un tissu d'oligodendrogliome,

(ii) la comparaison entre :

- le susdit rapport du niveau d'expression pour un couple de miARNs et,

    * celui représenté dans un abaque préétabli pour un tissu sain de référence, un tissu glioblastome de référence, et un tissu oligodendrogliome de référence,

(iii) la déduction :

    * soit que le tissu suspecté est un glioblastome, lorsque la différence entre le rapport obtenu dans l'étape (i) et le rapport dans un abaque préétabli dans un tissu de glioblastome de référence est inférieure à 20%, notamment 15%, plus particulièrement 10%, vis-à-vis du rapport dans un abaque préétabli,
    * soit que le tissu suspecté est un oligodendrogliome, lorsque la différence entre le rapport obtenu dans l'étape (i) et le rapport dans un abaque préétabli dans un tissu d'oligodendrogliome de référence est inférieure à 20%, notamment 15%, plus particulièrement 10%, vis-à-vis du rapport dans un abaque préétabli.

La description a particulièrement pour objet une méthode de diagnostic *in vitro* de gliomes, permettant de préciser si un patient suspecté de présenter un gliome est atteint d'un glioblastome ou d'un oligodendrogliome.
Cette méthode comprend :

- i) la détermination, selon l'une des méthodes décrites ci-dessus, de la présence de gliome dans un patient suspecté de présenter un gliome,
- ii) la déduction, selon l'une des méthodes décrites ci-dessus, que ledit patient présente un glioblastome ou le patient suspecté présente un oligodendrogliome.

Le premier mode de réalisation de cette méthode consiste en des comparaisons des niveaux d'expression des miARNs entre l'échantillon suspecté et les échantillons de référence.
Plus précisément, cette méthode comprend:

- i) la mesure du niveau d'expression d'au moins un miARN choisi parmi hsa-miR-126, hsa-miR-15b, hsa-miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-151, hsa-miR-26b ou hsa-miR-34b*, dans un échantillon biologique provenant d'un patient suspecté de présenter un gliome,
- ii) la comparaison des niveaux d'expression du susdit miARN dans le susdit échantillon et dans un échantillon sain de référence,
- iii) la déduction que le patient présente un gliome, lorsque le niveau d'expression du susdit miARN dans l'échantillon provenant du susdit patient et le niveau d'expression du susdit miARN dans l'échantillon sain de référence sont différents, le niveau d'expression du susdit miARN dans l'échantillon provenant du susdit patient étant 3 fois, notamment 4 fois, plus particulièrement 5 fois, supérieur ou inférieur au niveau d'expression du susdit miARN dans l'échantillon sain de référence,

- iv) la mesure du niveau d'expression d'au moins un miARN choisi parmi hsa-miR-155, hsa-miR-210 ou hsa-miR-10a, hsa-miR-409 ou hsa-miR-134, dans ledit échantillon biologique provenant d'un patient présentant un gliome,
- v) la comparaison entre :
- le niveau d'expression du susdit miARN dans le susdit échantillon et

    * respectivement le niveau d'expression du susdit miARN dans un échantillon de référence provenant d'un patient présentant un glioblastome, et
    * respectivement le niveau d'expression du susdit miARN dans un échantillon de référence provenant d'un patient présentant un oligodendrogliome, et

- vi) la déduction :
- soit que le patient présente un glioblastome, lorsque le niveau d'expression du susdit miARN dans l'échantillon provenant du susdit patient est égal, dans la limite de 20% en plus ou 20% en moins, au niveau d'expression du susdit miARN dans l'échantillon de référence provenant d'un patient présentant un glioblastome,
- soit que le patient présente un oligodendrogliome, lorsque le niveau d'expression du susdit miARN dans l'échantillon provenant du susdit patient est égal, dans la limite de 20% en plus ou 20% en moins, au niveau d'expression du susdit miARN dans l'échantillon de référence provenant d'un patient présentant un oligodendrogliome.

Dans un mode de réalisation particulier, l'échantillon biologique provenant d'un patient suspecté de présenter un gliome est un tissu cérébral obtenu par exérèse, ou par biopsie. Ladite méthode comprend :

- (i) la mesure du niveau d'expression d'au moins un miARN choisi parmi hsa-miR-126, hsa-miR-15b, hsa-miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-151, hsa-miR-26b ou hsa-miR-34b*,
- ii) la comparaison des niveaux d'expression du susdit miARN dans le tissu suspecté et dans un tissu sain de référence,
- iii) la déduction du caractère tumoral du tissu suspecté, lorsque le niveau d'expression du susdit miARN dans le tissu suspecté et le niveau d'expression du susdit miARN dans le tissu sain de référence sont différents, le niveau d'expression du susdit miARN dans le tissu suspecté étant 3 fois, notamment 4 fois, plus particulièrement 5 fois, supérieur ou inférieur au niveau d'expression du susdit miARN dans le tissu sain de référence,
- iv) la mesure du niveau d'expression d'au moins un miARN choisi parmi hsa-miR-155, hsa-miR-210 ou hsa-miR-10a, hsa-miR-409 ou hsa-miR-134, dans ledit tissu biologique déterminé à l'étape précédente comme ayant un caractère tumoral,
- v) la comparaison entre :
- le niveau d'expression du susdit miARN dans le susdit tissu et

  * respectivement le niveau d'expression du susdit miARN dans un tissu de glioblastome de référence, et
  * respectivement le niveau d'expression du susdit miARN dans un tissu d'oligodendrogliome de référence, et

- vi) la déduction :

  - soit que le susdit tissu est un glioblastome, lorsque le niveau d'expression du susdit miARN dans le tissu suspecté est égal, dans la limite de 20% en plus ou 20% en moins, au niveau d'expression du susdit miARN dans le tissu de glioblastome de référence,
  - soit que le susdit tissu est un oligodendrogliome, lorsque le niveau d'expression du susdit miARN dans le tissu suspecté est égal, dans la limite de 20% en plus ou 20% en moins, au niveau d'expression du susdit miARN dans le tissu d'oligodendrogliome de référence.

Dans un mode avantageux, cette méthode permet de déterminer directement si un glioblastome ou un oligodendrogliome est présent dans un patient suspecté d'être atteint d'un gliome. Il n'est plus nécessaire d'effectuer une première étape relative à la détermination de la présence de gliomes dans le susdit patient. La réalisation de cette méthode fait appel à 5 miARNs spécifiques repérés dans l'invention, à savoir hsa-miR-155, hsa-miR-210, hsa-miR-10a, hsa-miR-409, et hsa-miR-134, dont les niveaux d'expression sont à la fois bien différents entre les échantillons provenant des patients présentant un gliome et les échantillons provenant d'une personne saine, et systématiquement plus élevés dans les échantillons provenant des patients présentant un glioblastome par rapport à ceux provenant des patients présentant un oligodendrogliome.

Cette méthode est réalisée selon les étapes suivantes :

(i) la mesure du niveau d'expression d'au moins un miARNs choisi parmi hsa-miR-155, hsa-miR-210, hsa-miR-10a, hsa-miR-409 ou hsa-miR-134, dans un échantillon biologique provenant d'un patient suspecté de présenter un gliome,
(ii) la comparaison entre le niveau d'expression du susdit miARN dans le susdit échantillon et

  * respectivement le niveau d'expression du susdit miARN dans un échantillon sain de référence, et
  * respectivement le niveau d'expression du susdit miARN dans un échantillon de référence provenant d'un patient présentant un glioblastome, et
  * respectivement le niveau d'expression du susdit miARN dans un échantillon de référence provenant d'un patient présentant un oligodendrogliome,

(iii) la déduction :

  * soit que le patient ne présente pas un gliome, lorsque le niveau d'expression du susdit miARN dans l'échantillon provenant d'un patient suspecté de présenter un gliome est égal, dans la limite de 20% en plus ou 20% en moins, au niveau d'expression du susdit miARN dans l'échantillon sain de référence,
  * soit que le patient présente un glioblastome,

  - lorsque le niveau d'expression du susdit miARN dans l'échantillon provenant d'un patient suspecté de présenter un gliome est égal, dans la limite de 20% en plus ou 20% en moins, au niveau d'expression du susdit miARN dans l'échantillon de référence provenant d'un patient présentant un glioblastome, et

- lorsque le niveau d'expression du susdit miARN dans l'échantillon provenant d'un patient suspecté de présenter un gliome est au moins 3 fois supérieur au niveau d'expression du susdit miARN dans l'échantillon de référence provenant d'un patient présentant un oligodendrogliome,

  * soit que le patient présente un oligodendrogliome,

- lorsque le niveau d'expression du susdit miARN dans l'échantillon provenant d'un patient suspecté de présenter un gliome est égal, dans la limite de 20% en plus ou 20% en moins, au niveau d'expression du susdit miARN dans l'échantillon de référence provenant d'un patient présentant un oligodendrogliome, et
- lorsque le niveau d'expression du susdit miARN dans l'échantillon provenant d'un patient suspecté de présenter un gliome est au moins 3 fois inférieur au niveau d'expression du susdit miARN dans l'échantillon de référence provenant d'un patient présentant un glioblastome.

Dans un mode de réalisation particulier, l'échantillon biologique provenant d'un patient suspecté de présenter un gliome est un tissu cérébral obtenu par exérèse, ou par biopsie. Ladite méthode comprend :

(i) la mesure du niveau d'expression d'au moins un miARNs choisi parmi hsa-miR-155, hsa-miR-210, hsa-miR-10a, hsa-miR-409 ou hsa-miR-134, dans un tissu biologique provenant d'un tissu suspecté de présenter un caractère tumoral,
(ii) la comparaison entre le niveau d'expression du susdit miARN dans le susdit tissu suspecté et

  * respectivement le niveau d'expression du susdit miARN dans un tissu sain de référence, et
  * respectivement le niveau d'expression du susdit miARN dans un tissu de glioblastome de référence, et
  * respectivement le niveau d'expression du susdit miARN dans un tissu d'oligodendrogliome de référence,

(iii) la déduction :

  * soit que le susdit tissu est un tissu sain, lorsque le niveau d'expression du susdit miARN dans le tissu suspecté est égal, dans la limite de 20% en plus ou 20% en moins, au niveau d'expression du susdit miARN dans le tissu sain de référence,
  * soit que le susdit tissu est un glioblastome,

    - lorsque le niveau d'expression du susdit miARN dans le tissu suspecté est égal, dans la limite de 20% en plus ou 20% en moins, au niveau d'expression du susdit miARN dans le tissu de glioblastome de référence, et
    - lorsque le niveau d'expression du susdit miARN dans le tissu suspecté est au moins 3 fois supérieur au niveau d'expression du susdit miARN dans le tissu d'oligodendrogliome de référence,

  * soit que le susdit tissu est un oligodendrogliome,

    - lorsque le niveau d'expression du susdit miARN dans le tissu suspecté est égal, dans la limite de 20% en plus ou 20% en moins, au niveau d'expression du susdit miARN dans le tissu d'oligodendrogliome de référence, et
    - lorsque le niveau d'expression du susdit miARN dans le tissu suspecté est au moins 3 fois inférieur au niveau d'expression du susdit miARN dans le tissu de glioblastome de référence.

Avantageusement, la détermination de la nature précise d'une tumeur gliale suspectée d'être présente chez un patient est réalisée selon les étapes suivantes :

(i) la mesure du niveau d'expression d'au moins un miARNs choisi parmi hsa-miR-409 ou hsa-miR-134, dans un échantillon biologique provenant d'un patient suspecté de présenter un gliome,
(ii) la comparaison entre le niveau d'expression du susdit miARN dans le susdit échantillon et

  * respectivement le niveau d'expression du susdit miARN dans un échantillon sain de référence, et
  * respectivement le niveau d'expression du susdit miARN dans un échantillon de référence provenant d'un patient présentant un glioblastome, et
  * respectivement le niveau d'expression du susdit miARN dans un échantillon de référence provenant d'un patient présentant un oligodendrogliome,

(iii) la déduction :

* soit que le patient ne présente pas un gliome, lorsque le niveau d'expression du susdit miARN dans l'échantillon provenant du susdit patient est égal, dans la limite de 20% en plus ou 20% en moins, au niveau d'expression du susdit miARN dans l'échantillon sain de référence,
* soit que le patient présente un glioblastome,

- lorsque le niveau d'expression du susdit miARN dans l'échantillon provenant d'un patient suspecté de présenter un gliome est égal, dans la limite de 20% en plus ou 20% en moins, au niveau d'expression du susdit miARN dans l'échantillon de référence provenant d'un patient présentant un glioblastome, et
- lorsque le niveau d'expression du susdit miARN dans l'échantillon provenant d'un patient suspecté de présenter un gliome est au moins 3 fois supérieur au niveau d'expression du susdit miARN dans l'échantillon de référence provenant d'un patient présentant un oligodendrogliome,

* soit que le patient présente un oligodendrogliome,

- lorsque le niveau d'expression du susdit miARN dans l'échantillon provenant d'un patient suspecté de présenter un gliome est égal, dans la limite de 20% en plus ou 20% en moins, au niveau d'expression du susdit miARN dans l'échantillon de référence provenant d'un patient présentant un oligodendrogliome, et
- lorsque le niveau d'expression du susdit miARN dans l'échantillon provenant d'un patient suspecté de présenter un gliome est au moins 3 fois inférieur au niveau d'expression du susdit miARN dans l'échantillon de référence provenant d'un patient présentant un glioblastome.

Le deuxième mode de réalisation de la méthode de diagnostic *in vitro* de gliomes, permettant de déterminer qu'un patient présente un gliome et de déterminer la nature précise du gliome, consiste en des comparaisons de niveaux d'expression des miARNs intra-échantillons.
Cette méthode comprend

(i) la mesure d'au moins un miARN choisi parmi hsa-miR-126, hsa-miR-15b, hsa-miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-151, hsa-miR-26b, hsa-miR-34b*, hsa-miR-155, hsa-miR-210 ou hsa-miR-10a, dans un échantillon biologique provenant d'un patient suspecté de présenter un gliome,
(ii) la comparaison entre le niveau d'expression d'au moins un premier susdit miARN dans l'échantillon biologique du patient suspecté de présenter un gliome et le niveau d'expression d'au moins un deuxième susdit miARN dans l'échantillon biologique du patient suspecté, les susdits premier miARN et deuxième miARN formant un couple de miARNs, et
(iii) la déduction que le patient présente un gliome, lorsque le rapport entre le niveau d'expression du susdit premier miARN et le niveau d'expression du susdit deuxième miARN est 2 fois, notamment 3 fois, plus particulièrement 4 fois, supérieur ou inférieur à celui d'un abaque préétabli dans un échantillon sain de référence,
(iv) la mesure du rapport du niveau d'expression d'au moins un couple de miARNs constitué par :

* un miARN choisi parmi hsa-miR-155, hsa-miR-210, hsa-miR-10a, hsa-miR-409 ou hsa-miR-134, et
* un miARN choisi parmi hsa-miR-126, hsa-miR-15b, hsa-miR-16, hsa-miR-17, hsa-miR-20a, hsa-let7a, hsa-let 7f, hsa-let 7d, hsa-miR-374, hsa-miR-339, hsa-miR-127, hsa-miR-151, hsa-miR-26b, hsa-miR-34b* pour un échantillon provenant du susdit patient,

ladite mesure permettant d'établir le rapport du niveau d'expression pour un susdit couple de miARNs,
(v) la comparaison entre :

- le susdit rapport du niveau d'expression pour un couple de miARNs et,
- le rapport dans un abaque préétabli dans un échantillon sain de référence, l'échantillon de référence provenant d'un patient présentant un glioblastome, et l'échantillon de référence provenant d'un patient présentant un oligodendrogliome,

(vi) la déduction :

* soit que le patient présente un glioblastome, lorsque la différence entre le rapport obtenu dans l'étape (iv) et le rapport dans un abaque préétabli dans un échantillon de référence provenant d'un patient présentant un glioblastome est inférieure à 20%, notamment 15%, plus particulièrement 10%, vis-à-vis du rapport dans un abaque préétabli dans un échantillon de référence provenant d'un patient présentant un glioblastome,

* soit que le patient présente un oligodendrogliome, lorsque la différence entre le rapport obtenu dans l'étape (iv) et le rapport dans un abaque préétabli dans un échantillon de référence provenant d'un patient présentant un oligodendrogliome est inférieure à 20%, notamment 15%, plus particulièrement 10%, vis-à-vis du rapport dans un abaque préétabli dans un échantillon de référence provenant d'un patient présentant un oligodendrogliome.

Dans un mode de réalisation particulier, l'échantillon biologique provenant d'un patient suspecté de présenter un gliome est un tissu cérébral obtenu par exérèse, ou par biopsie. Ladite méthode comprend :

(i) la mesure d'au moins un miARN choisi parmi hsa-miR-126, hsa-miR-15b, hsa-miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-151, hsa-miR-26b, hsa-miR-34b*, hsa-miR-155, hsa-miR-210 ou hsa-miR-10a, dans un tissu biologique provenant d'un tissu suspecté d'avoir un caractère tumoral,

(ii) la comparaison entre le niveau d'expression d'au moins un premier susdit miARN dans le tissu suspecté et le niveau d'expression d'au moins un deuxième susdit miARN dans le tissu suspecté, les susdits premier miARN et deuxième miARN formant un couple de miARNs, et

(iii) la déduction du caractère tumoral du tissu suspecté, lorsque le rapport entre le niveau d'expression du susdit premier miARN et le niveau d'expression du susdit deuxième miARN est 2 fois, notamment 3 fois, plus particulièrement 4 fois, supérieur ou inférieur à celui d'un abaque préétabli dans un tissu sain de référence,

(iv) la mesure du rapport du niveau d'expression d'au moins un couple de miARNs constitué par :

* un miARN choisi parmi hsa-miR-155, hsa-miR-210, hsa-miR-10a, hsa-miR-409 ou hsa-miR-134, et

* un miARN choisi parmi hsa-miR-126, hsa-miR-15b, hsa-miR-16, hsa-miR-17, hsa-miR-20a, hsa-let7a, hsa-let 7f, hsa-let 7d, hsa-miR-374, hsa-miR-339, hsa-miR-127, hsa-miR-151, hsa-miR-26b, hsa-miR-34b* pour un tissu biologique provenant d'un tissu tumoral suspecté d'être un tissu de glioblastome ou un tissu d'oligodendrogliome,

ladite mesure permettant d'établir le rapport du niveau d'expression pour un susdit couple de miARNs,

(v) la comparaison entre :

* le susdit rapport du niveau d'expression pour un couple de miARNs et,

* le rapport dans un abaque préétabli dans un tissu sain de référence, un tissu glioblastome de référence, et un tissu oligodendrogliome de référence,

(vi) la déduction :

* soit que le tissu suspecté est un glioblastome, lorsque la différence entre le rapport obtenu dans l'étape (iv) et le rapport dans un abaque préétabli dans un tissu de glioblastome de référence est inférieure à 20%, notamment 15%, plus particulièrement 10%, vis-à-vis du rapport dans un abaque préétabli dans un tissu de glioblastome de référence.

* soit que le tissu suspecté est un oligodendrogliome, lorsque la différence entre le rapport obtenu dans l'étape (iv) et le rapport dans un abaque préétabli dans un tissu d'oligodendrogliome de référence est inférieure à 20%, notamment 15%, plus particulièrement 10%, vis-à-vis du rapport dans un abaque préétabli dans un tissu d'oligodendrogliome de référence.

Dans un mode avantageux, cette méthode permet de déterminer directement la nature précise d'un tissu suspecté de présenter un caractère tumoral, sans besoin d'effectuer une étape de la détermination de la présence de gliomes dans un tissu suspecté. Cette méthode est réalisée selon les étapes suivantes :

(i) la mesure du rapport du niveau d'expression d'au moins un couple de miARNs choisi parmi hsa-let 7a/hsa-miR 409, hsa-let 7d/hsa-miR409, hsa-miR134/hsa-let 7f, hsa-let 7f/hsa-miR409, hsa-miR134/hsa-let 7d, hsa-miR151/hsa-miR155, hsa-miR155/hsa-miR-34b*, hsa-miR15b/hsa-miR134, hsa-miR134/hsa-miR20a, hsa-miR210/hsa-let 7f, (tableau 7) dans un échantillon biologique provenant d'un patient suspecté de présenter un gliome,

(ii) la comparaison entre : ,

le susdit rapport du niveau d'expression pour un couple de miARNs, et

- le rapport dans un abaque préétabli respectivement dans un échantillon sain de référence, l'échantillon de référence provenant d'un patient présentant un glioblastome, et l'échantillon de référence provenant d'un patient présentant un oligodendrogliome,

(iii) la déduction :

\* soit que le patient ne présente pas un gliome, lorsque la différence entre le rapport obtenu dans l'étape (i) et le rapport dans un abaque préétabli dans un échantillon sain de référence est inférieure à 20%, notamment 15%, plus particulièrement 10%, vis-à-vis du rapport dans un abaque préétabli dans un échantillon sain de référence.

\* soit que le patient présente un glioblastome, lorsque la différence entre le rapport obtenu dans l'étape (i) et le rapport dans un abaque préétabli dans un échantillon de référence provenant d'un patient présentant un glioblastome est inférieure à 20%, notamment 15%, plus particulièrement 10%, vis-à-vis du rapport dans un abaque préétabli dans un échantillon de référence provenant d'un patient présentant un glioblastome,

\* soit que le patient présente un oligodendrogliome, lorsque la différence entre le rapport obtenu dans l'étape (i) est le rapport dans un abaque préétabli dans un échantillon de référence provenant d'un patient présentant un oligodendrogliome est inférieure à 20%, notamment 15%, plus particulièrement 10%, vis-à-vis du rapport dans un abaque préétabli dans un échantillon de référence provenant d'un patient présentant un oligodendrogliome.

Dans un mode de réalisaiton particulier, l'échantillon biologique provenant d'un patient suspecté de présenter un gliome est un tissu cérébral obtenu par exérèse, ou par biopsie. Ladite méthode comprend :

(i) la mesure du rapport du niveau d'expression d'au moins un couple de miARNs choisi parmi hsa-let 7a/hsa-miR 409, hsa-let 7d/hsa-miR409, hsa-miR134/hsa-let 7f, hsa-let 7f/hsa-miR409, hsa-miR134/hsa-let 7d, hsa-miR151/hsa-miR155, hsa-miR155/hsa-miR-34b\*, hsa-miR15b/hsa-miR134, hsa-miR134/hsa-miR20a, hsa-miR210/hsa-let 7f, (tableau 7) dans un tissu biologique provenant d'un tissu suspecté d'avoir un caractère tumoral,

(ii) la comparaison entre :

- le susdit rapport du niveau d'expression pour un couple de miARNs, et
- le rapport dans un abaque préétabli respectivement dans un tissu sain de référence, un tissu glioblastome de référence, et un tissu oligodendrogliome de référence

(iii) la déduction :

\* soit que le susdit tissu est un tissu sain, lorsque la différence entre le rapport obtenu dans l'étape (i) et le rapport dans un abaque préétabli dans un tissu sain de référence est inférieure à 20%, notamment 15%, plus particulièrement 10%, vis-à-vis du rapport dans un abaque préétabli dans un tissu sain de référence.

\* soit que le susdit tissu est un tissu de glioblastome, lorsque la différence entre le rapport obtenu dans l'étape (i) et le rapport dans un abaque préétabli dans un tissu de glioblastome de référence est inférieure à 20%, notamment 15%, plus particulièrement 10%, vis-à-vis du rapport dans un abaque préétabli dans un tissu glioblastome de référence.

\* soit que le susdit tissu est un tissu d'oligodendrogliome, lorsque la différence entre le rapport obtenu dans l'étape (i) est le rapport dans un abaque préétabli dans un tissu d'oligodendrogliome de référence est inférieure à 20%, notamment 15%, plus particulièrement 10%, vis-à-vis du rapport dans un abaque préétabli dans un tissu d'oligodendrogliome de référence.

Dans un mode de réalisation, le niveau d'expression des miARNs est dosé par PCR en temps réel.

Dans un autre mode de réalisation, le niveau d'expression des miARNs est dosé par hybridation sur « puces à ADN » ou par séquençage à haut débit, ou par Northern blot.

Dans un mode de réalisation, un échantillon biologique est choisi parmi un échantillon tissulaire, notamment un tissu cérébral obtenu par exérèse, ou un échantillon de liquide biologique, notamment un échantillon sanguin.

La description concerne également une trousse de diagnostic *in vitro* de gliomes, cette trousse comprenant les moyens de dosage d'au moins un miARN choisi parmi hsa-miR-126, hsa-miR-15b, hsa-miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-151, hsa-miR-26b, hsa-miR-34b\*, hsa-miR-155, hsa-miR-210 ou hsa-miR-10a.

Dans un mode particulier, cette trousse comprend les moyens de dosage d'au moins un miARN choisi parmi hsa-miR-155, hsa-miR-210 ou hsa-miR-10a, hsa-miR-134 ou hsa-miR-409.

Cette trousse de diagnostic *in vitro* de gliomes peut comprendre à titre d'exemple, la Reverse Transcriptase, la DNA polymérase, telle que Hot start, le tampon, un inhibiteur de RNase, des dNTPs, les oligonucléotides amorces d'amplification, les sondes spécifiques, telles que la sonde de Taqman@, ou les marqueurs aspécifiques comme le SYBR Green.

La description a également pour objet une méthode d'évaluation d'efficacité d'une méthode de traitement de gliomes.

Cette méthode comprend :

- la mesure du niveau d'expression d'au moins un miARN choisi parmi hsa-miR-126, hsa-miR-15b, hsa-miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-151, hsa-miR-26b, hsa-miR-34b*, hsa-miR-155, hsa-miR-210 ou hsa-miR-10a dans un échantillon biologique provenant d'un patient soumis à un traitement du gliome, et éventuellement
- la comparaison entre
- le niveau d'expression du susdit miARN dans l'échantillon biologique provenant dudit patient, et
- respectivement le niveau d'expression du susdit miARN un échantillon de référence provenant d'un patient présentant un glioblastome,
- et respectivement le niveau d'expression du susdit miARN dans un échantillon de référence provenant d'un patient présentant un oligodendrogliome.

Dans un mode de réalisation avantageux, cette méthode permet d'évaluer l'efficacité d'une méthode de traitement de gliomes, par exemple, une intervention chirurgicale, une chimiothérapie ou une radiothérapie.

Dans un mode de réalisation particulièrement avantageux, la méthode concerne une méthode de diagnostic *in vitro* d'une tumeur cérébrale appartenant au groupe constitué de 2 types de tumeurs : ODG, GBM, et d'identification du type de la tumeur, comprenant :

(i) la mesure du niveau d'expression d'au moins deux miARNs dans un échantillon biologique provenant d'un patient suspecté de présenter un gliome, dans lesquels :

- un premier miARN est choisi parmi hsa-miR-155, hsa-miR-210, hsa-miR-10a, hsa-miR-409 ou hsa-miR-134 ;
- un deuxième miARN est choisi parmi hsa-miR-126, hsa-miR-15b, hsa-miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-151, hsa-miR-26b, hsa-miR-34b*, hsa-miR-155, hsa-miR-210 ou hsa-miR-10a, ledit deuxième miARN choisi étant différent du susdit premier miARN choisi ;

(ii) la comparaison :

- entre les niveaux d'expression des susdits miARNs dans le susdit échantillon et les niveaux d'expression des susdits miARNs dans un échantillon sain de référence, un échantillon de GBM de référence, un échantillon d'ODG de référence, ou
- entre le niveau d'expression d'au moins un premier susdit miARN dans l'échantillon provenant d'un patient suspecté de présenter un gliome et le niveau d'expression d'au moins un deuxième susdit miARN dans le même échantillon provenant du patient suspecté de présenter un gliome.

Plus particulièrement, cette méthode comprend :

(i) la mesure du niveau d'expression dans un échantillon biologique provenant d'un patient suspecté de présenter un gliome,

- d'un premier miARN choisi parmi hsa-miR-155, hsa-miR-210, hsa-miR-10a, hsa-miR-409 ou hsa-miR-134, et
- d'un deuxième miARN choisi parmi hsa-miR-126, hsa-miR-15b, hsa-miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-151, hsa-miR-26b, hsa-miR-34b*, hsa-miR-155, hsa-miR-210 ou hsa-miR-10a, ledit deuxième miARN choisi étant différent du susdit premier miARN choisi,

(ii) la comparaison entre les niveaux d'expression des susdits miARNs dans le susdit échantillon et les niveaux d'expression des susdits miARNs dans un échantillon sain de référence, un échantillon de GBM de référence, un échantillon d'ODG de référence.

Dans un mode de réalisaiton particulier, la déduction de la présence de GBM dans l'échantillon provenant du patient suspecté de présenter un gliome est faite, lorsque :

(i) le niveau d'expression du premier miARN mesuré dans ledit échantillon est au moins 3 fois supérieur ou inférieur au niveau d'expression du susdit miARN mesuré dans l'échantillon sain de référence, et

(ii) le niveau d'expression du premier miARN mesuré dans ledit échantillon est au moins 3 fois supérieur au niveau d'expression du susdit miARN mesuré dans l'échantillon d'ODG de référence, et

(iii) le niveau d'expression du deuxième miARN mesuré dans ledit échantillon est au moins 3 fois supérieur ou inférieur au niveau d'expression du susdit miARN mesuré dans l'échantillon sain de référence, et

(iv) le niveau d'expression du deuxième miARN mesuré dans ledit échantillon est égal, dans la limite de 20% en plus ou 20% en moins, au niveau d'expression du susdit miARN dans l'échantillon de GBM de référence.

Dans un autre mode de réalisation particulier, la déduction de la présence d'ODG dans l'échantillon provenant du patient suspecté de présenter un gliome est faite, lorsque :

(i) le niveau d'expression du premier miARN mesuré dans ledit échantillon est au moins 3 fois supérieur ou inférieur au niveau d'expression du susdit miARN mesuré dans l'échantillon sain de référence, et

(ii) le niveau d'expression du premier miARN mesuré dans ledit échantillon est au moins 3 fois inférieur au niveau d'expression du susdit miARN mesuré dans l'échantillon de GBM de référence, et

(iii) le niveau d'expression du deuxième miARN mesuré dans ledit échantillon est au moins 3 fois supérieur ou inférieur au niveau d'expression du susdit miARN mesuré dans l'échantillon sain de référence, et

(iv) le niveau d'expression du deuxième miARN mesuré dans ledit échantillon est égal, dans la limite de 20% en plus ou 20% en moins, au niveau d'expression du susdit miARN dans l'échantillon d'ODG de référence.

Dans un mode de réalisation avantageux, la méthode de diagnostic *in vitro* d'une tumeur cérébrale appartenant au groupe constitué de 2 types de tumeurs : ODG, GBM, et d'identification du type de la tumeur comprend la mesure:

- du premier miARN : hsa-miR-155, et
- du deuxième miARN choisi parmi hsa- miR-126, hsa-miR-15b, hsa- miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-151, hsa-miR-26b, hsa-miR-34b*, hsa-miR-210 ou hsa-miR-10a.

Dans un mode de réalisation particulièrement avantageux, la méthode de diagnostic *in vitro* d'une tumeur cérébrale appartenant au groupe constitué de 2 types de tumeurs : ODG, GBM, et d'identification du type de la tumeur comprend la mesure:

- du premier miARN : hsa-miR-155, et
- du deuxième miARN choisi parmi hsa-miR-126, hsa-miR-15b, hsa-miR-16, hsa-miR-17, hsa-miR-20a, hsa-let 7f, hsa-miR-374, hsa-miR-409, hsa-miR-134, hsa-miR-339, hsa-miR-151, hsa-miR-26b, hsa-miR-34b* ou hsa-miR-210.

Plus particulièrement, la méthode de diagnostic *in vitro* mesure le premier miARN hsa-miR-155 et le deuxième miARN hsa-miR-126.

Plus particulièrement, la méthode de diagnostic *in vitro* mesure le premier miARN hsa-miR-155 et le deuxième miARN hsa-miR-15b.

Plus particulièrement, la méthode de diagnostic *in vitro* mesure le premier miARN hsa-miR-155 et le deuxième miARN hsa-miR-16.

Plus particulièrement, la méthode de diagnostic *in vitro* mesure le premier miARN hsa-miR-155 et le deuxième miARN hsa-miR-17.

Plus particulièrement, la méthode de diagnostic *in vitro* mesure le premier miARN hsa-miR-155 et le deuxième miARN hsa-miR-20a.

Plus particulièrement, la méthode de diagnostic *in vitro* mesure le premier miARN hsa-miR-155 et le deuxième miARN hsa-let 7f.

Plus particulièrement, la méthode de diagnostic *in vitro* mesure le premier miARN hsa-miR-155 et le deuxième miARN hsa-miR-374.

Plus particulièrement, la méthode de diagnostic *in vitro* mesure le premier miARN hsa-miR-155 et le deuxième miARN hsa-miR-409.

Plus particulièrement, la méthode de diagnostic *in vitro* mesure le premier miARN hsa-miR-155 et le deuxième miARN hsa-miR-134.

Plus particulièrement, la méthode de diagnostic *in vitro* mesure le premier miARN hsa-miR-155 et le deuxième miARN hsa-miR-339.

Plus particulièrement, la méthode de diagnostic *in vitro* mesure le premier miARN hsa-miR-155 et le deuxième miARN hsa-miR-151.

Plus particulièrement, la méthode de diagnostic *in vitro* mesure le premier miARN hsa-miR-155 et le deuxième miARN hsa-miR-26b.

Plus particulièrement, la méthode de diagnostic *in vitro* mesure le premier miARN hsa-miR-155 et le deuxième miARN hsa-miR-34b*.

Plus particulièrement, la méthode de diagnostic *in vitro* mesure le premier miARN hsa-miR-155 et le deuxième miARN ou hsa-miR-210.

Dans un mode de réalisation avantageux, la méthode de diagnostic *in vitro* d'une tumeur cérébrale appartenant au groupe constitué de 2 types de tumeurs : ODG, GBM, et d'identification du type de la tumeur comprend la mesure:

- du premier miARN : hsa-miR-210, et
- du deuxième miARN choisi parmi hsa- miR-126, hsa-miR-15b, hsa- miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-151, hsa-miR-26b, hsa-miR-34b*, hsa-miR-155 ou hsa-miR-10a.

Dans un mode de réalisation particulièrement avantageux, la méthode de diagnostic *in vitro* comprend la mesure:

- du premier miARN : hsa-miR-210, et
- du deuxième miARN choisi parmi hsa-miR-126, hsa-miR-15b, hsa-miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-26b, hsa-miR-34b*, hsa-miR-155, ou hsa-miR-10a.

Plus particulièrement, la méthode de diagnostic *in vitro* mesure le premier miARN hsa-miR-210 et le deuxième miARN hsa-miR-126.

Plus particulièrement, la méthode de diagnostic *in vitro* mesure le premier miARN hsa-miR-210 et le deuxième miARN hsa-miR-15b.

Plus particulièrement, la méthode de diagnostic *in vitro* mesure le premier miARN hsa-miR-210 et le deuxième miARN hsa-miR-16.

Plus particulièrement, la méthode de diagnostic *in vitro* mesure le premier miARN hsa-miR-210 et le deuxième miARN hsa-miR-17.

Plus particulièrement, la méthode de diagnostic *in vitro* mesure le premier miARN hsa-miR-210 et le deuxième miARN hsa-miR-20a.

Plus particulièrement, la méthode de diagnostic *in vitro* mesure le premier miARN hsa-miR-210 et le deuxième miARN hsa-let7a.

Plus particulièrement, la méthode de diagnostic *in vitro* mesure le premier miARN hsa-miR-210 et le deuxième miARN hsa-let 7f.

Plus particulièrement, la méthode de diagnostic *in vitro* mesure le premier miARN hsa-miR-210 et le deuxième miARN hsa-let 7d.

Plus particulièrement, la méthode de diagnostic *in vitro* mesure le premier miARN hsa-miR-210 et le deuxième miARN hsa-miR-374.

Plus particulièrement, la méthode de diagnostic *in vitro* mesure le premier miARN hsa-miR-210 et le deuxième miARN hsa-miR-409.

Plus particulièrement, la méthode de diagnostic *in vitro* mesure le premier miARN hsa-miR-210 et le deuxième miARN hsa-miR-134.

Plus particulièrement, la méthode de diagnostic *in vitro* mesure le premier miARN hsa-miR-210 et le deuxième miARN hsa-miR-339.

Plus particulièrement, la méthode de diagnostic *in vitro* mesure le premier miARN hsa-miR-210 et le deuxième hsa-miR-127.

Plus particulièrement, la méthode de diagnostic *in vitro* mesure le premier miARN hsa-miR-210 et le deuxième miARN hsa-miR-26b.

Plus particulièrement, la méthode de diagnostic *in vitro* mesure le premier miARN hsa-miR-210 et le deuxième miARN hsa-miR-34b*.

Plus particulièrement, la méthode de diagnostic *in vitro* mesure le premier miARN hsa-miR-210 et le deuxième miARN hsa-miR-155.

Plus particulièrement, la méthode de diagnostic *in vitro* mesure le premier miARN hsa-miR-210 et le deuxième miARN hsa-miR-10a.

Dans un mode de réalisation avantageux, la méthode de diagnostic *in vitro* d'une tumeur cérébrale appartenant au groupe constitué de 2 types de tumeurs : ODG, GBM, et d'identification du type de la tumeur comprend la mesure:

- du premier miARN : hsa-miR-10a ; et
- du deuxième miARN choisi parmi hsa- miR-126, hsa-miR-15b, hsa- miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-151, hsa-miR-26b, hsa-miR-34b*, hsa-miR-210 ou hsa-miR-155.

Dans un mode de réalisation particulièrement avantageux, la méthode de diagnostic *in vitro* comprend la mesure:

- du premier miARN : hsa-miR-10a ; et
- le deuxième miARN choisi parmi hsa-miR-126, hsa-miR-15b, hsa-miR-16, hsa-miR-20a, hsa-let 7f, hsa-let 7d, hsa-miR-374, hsa-miR-409, hsa-miR-134, hsa-miR-339, hsa-miR-127, hsa-miR-151, hsa-miR-26b, hsa-miR-34b* ou hsa-miR-210.

Plus particulièrement, la méthode de diagnostic *in vitro* mesure le premier hsa-miR-10a et le deuxième miARN hsa-miR-126.

Plus particulièrement, la méthode de diagnostic *in vitro* mesure le premier hsa-miR-10a et le deuxième miARN hsa-miR-15b.

Plus particulièrement, la méthode de diagnostic *in vitro* mesure le premier hsa-miR-10a et le deuxième miARN hsa-miR-16.

Plus particulièrement, la méthode de diagnostic *in vitro* mesure le premier hsa-miR-10a et le deuxième miARN hsa-miR-20a.

Plus particulièrement, la méthode de diagnostic *in vitro* mesure le premier hsa-miR-10a et le deuxième hsa-let 7f.

Plus particulièrement, la méthode de diagnostic *in vitro* mesure le premier hsa-miR-10a et le deuxième miARN hsa-let 7d.

Plus particulièrement, la méthode de diagnostic *in vitro* mesure le premier hsa-miR-10a et le deuxième miARN hsa-miR-374.

Plus particulièrement, la méthode de diagnostic *in vitro* mesure le premier hsa-miR-10a et le deuxième miARN hsa-miR-409.

Plus particulièrement, la méthode de diagnostic *in vitro* mesure le premier hsa-miR-10a et le deuxième miARN hsa-miR-134.

Plus particulièrement, la méthode de diagnostic *in vitro* mesure le premier hsa-miR-10a et le deuxième miARN hsa-miR-339.

Plus particulièrement, la méthode de diagnostic *in vitro* mesure le premier hsa-miR-10a et le deuxième miARN hsa-miR-127.

Plus particulièrement, la méthode de diagnostic *in vitro* mesure le premier hsa-miR-10a et le deuxième miARN hsa-miR-151.

Plus particulièrement, la méthode de diagnostic *in vitro* mesure le premier hsa-miR-10a et le deuxième miARN hsa-miR-26b.

Plus particulièrement, la méthode de diagnostic *in vitro* mesure le premier hsa-miR-10a et le deuxième miARN hsa-miR-34b*.

Plus particulièrement, la méthode de diagnostic *in vitro* mesure le premier hsa-miR-10a et le deuxième miARN hsa-miR-210.

Dans un mode de réalisation avantageux, la méthode de diagnostic *in vitro* d'une tumeur cérébrale appartenant au groupe constitué de 2 types de tumeurs : ODG, GBM, et d'identification du type de la tumeur comprend la mesure:

- du premier miARN : hsa-miR-409 ; et
- du deuxième miARN choisi parmi parmi hsa- miR-126, hsa-miR-15b, hsa- miR-16, hsa-miR-17, hsa-miR-20a, hsa-let7a, hsa-let 7f, hsa-let 7d, hsa-miR-374, hsa-miR-10a, hsa-miR-134, hsa-miR-339, hsa-miR-127, hsa-miR-151, hsa-miR-26b, hsa-miR-34b*, hsa-miR-210 ou hsa-miR-155.

Dans un mode de réalisation particulièrement avantageux, la méthode de diagnostic *in vitro* comprend la mesure:

- du premier miARN : hsa-miR-409 ; et
- du deuxième miARN choisi parmi hsa-miR-126, hsa-miR-15b, hsa-miR-16, hsa-miR-17, hsa-miR-20a, hsa-let7a, hsa-let 7f, hsa-let 7d, hsa-miR-374, hsa-miR-134, hsa-miR-127, hsa-miR-26b, hsa-miR-155, hsa-miR-210 ou hsa-miR-10a.

Plus particulièrment, la méthode de diagnostic *in vitro* mesure le premier hsa-miR-409 et le deuxième miARN hsa-miR-126.

Plus particulièrment, la méthode de diagnostic *in vitro* mesure le premier hsa-miR-409 et le deuxième miARN hsa-miR-15b.

Plus particulièrment, la méthode de diagnostic *in vitro* mesure le premier hsa-miR-409 et le deuxième miARN hsa-miR-16.

Plus particulièrment, la méthode de diagnostic *in vitro* mesure le premier hsa-miR-409 et le deuxième miARN hsa-miR-17.

Plus particulièrment, la méthode de diagnostic *in vitro* mesure le premier hsa-miR-409 et le deuxième miARN hsa-miR-20a.

Plus particulièrment, la méthode de diagnostic *in vitro* mesure le premier hsa-miR-409 et le deuxième miARN hsa-let7a.

Plus particulièrment, la méthode de diagnostic *in vitro* mesure le premier hsa-miR-409 et le deuxième miARN hsa-let 7f.

Plus particulièrment, la méthode de diagnostic *in vitro* mesure le premier hsa-miR-409 et le deuxième miARN hsa-let 7d.

Plus particulièrment, la méthode de diagnostic *in vitro* mesure le premier hsa-miR-409 et le deuxième miARN hsa-miR-374.

Plus particulièrment, la méthode de diagnostic *in vitro* mesure le premier hsa-miR-409 et le deuxième miARN hsa-miR-134.

Plus particulièrment, la méthode de diagnostic *in vitro* mesure le premier hsa-miR-409 et le deuxième miARN hsa-miR-127.

Plus particulièrment, la méthode de diagnostic *in vitro* mesure le premier hsa-miR-409 et le deuxième miARN hsa-miR-26b.

Plus particulièrment, la méthode de diagnostic *in vitro* mesure le premier hsa-miR-409 et le deuxième miARN hsa-miR-155.

Plus particulièrment, la méthode de diagnostic *in vitro* mesure le premier hsa-miR-409 et le deuxième miARN hsa-miR-210.

Plus particulièrment, la méthode de diagnostic *in vitro* mesure le premier hsa-miR-409 et le deuxième miARN hsa-miR-10a.

Dans un mode de réalisation avantageux, la méthode de diagnostic *in vitro* d'une tumeur cérébrale appartenant au groupe constitué de 2 types de tumeurs : ODG, GBM, et d'identification du type de la tumeur comprend la mesure:

- du premier miARN : hsa-miR-134 ;
- du deuxième miARN choisi parmi hsa- miR-126, hsa-miR-15b, hsa- miR-16, hsa-miR-17, hsa-miR-20a, hsa-let7a, hsa-let 7f, hsa-let 7d, hsa-miR-374, hsa-miR-409, hsa-miR-339, hsa-miR-127, hsa-miR-151, hsa-miR-26b, hsa-miR-34b*, hsa-miR-155, hsa-miR-210 ou hsa-miR-10a.

Dans un mode de réalisation particulièrement avantageux, la méthode de diagnostic *in vitro* d'une tumeur cérébrale appartenant au groupe constitué de 2 types de tumeurs : ODG, GBM, et d'identification du type de la tumeur comprend la mesure:

- du premier miARN : hsa-miR-134 ;
- du deuxième miARN choisi parmi hsa-miR-126, hsa-miR-15b, hsa-miR-17, hsa-miR-20a, hsa-let7a, hsa-let 7f, hsa-let 7d, hsa-miR-374, hsa-miR-409, hsa-miR-26b, hsa-miR-155, hsa-miR-210 ou hsa-miR-10a.

Plus particulièrment, la méthode de diagnostic *in vitro* mesure le premier hsa-miR-134 et le deuxième miARN hsa-miR-126.

Plus particulièrment, la méthode de diagnostic *in vitro* mesure le premier hsa-miR-134 et le deuxième miARN hsa-miR-15b.

Plus particulièrment, la méthode de diagnostic *in vitro* mesure le premier hsa-miR-134 et le deuxième miARN hsa-miR-17.

Plus particulièrment, la méthode de diagnostic *in vitro* mesure le premier hsa-miR-134 et le deuxième miARN hsa-miR-20a.

Plus particulièrment, la méthode de diagnostic *in vitro* mesure le premier hsa-miR-134 et le deuxième miARN hsa-let7a.

Plus particulièrment, la méthode de diagnostic *in vitro* mesure le premier hsa-miR-134 et le deuxième miARN hsa-let 7f.

Plus particulièrment, la méthode de diagnostic *in vitro* mesure le premier hsa-miR-134 et le deuxième miARN hsa-let 7d.

Plus particulièrment, la méthode de diagnostic *in vitro* mesure le premier hsa-miR-134 et le deuxième miARN hsa-miR-374.

Plus particulièrment, la méthode de diagnostic *in vitro* mesure le premier hsa-miR-134 et le deuxième miARN hsa-miR-409.

Plus particulièrment, la méthode de diagnostic *in vitro* mesure le premier hsa-miR-134 et le deuxième miARN hsa-miR-26b.

Plus particulièrment, la méthode de diagnostic *in vitro* mesure le premier hsa-miR-134 et le deuxième miARN hsa-miR-155.

Plus particulièrment, la méthode de diagnostic *in vitro* mesure le premier hsa-miR-134 et le deuxième miARN hsa-miR-210.

Plus particulièrment, la méthode de diagnostic *in vitro* mesure le premier hsa-miR-134 et le deuxième hsa-miR-10a.

Dans un autre mode de réalisation, la description concerne une méthode de diagnostic *in vitro* d'une tumeur cérébrale appartenant au groupe constitué de 2 types de tumeurs : ODG, GBM, et d'identification du type de la tumeur, comprenant :

(i) la mesure du niveau d'expression d'au moins deux couples de miARNs dans un échantillon biologique provenant d'un patient suspecté de présenter un gliome,

\*chaque susdit couple de miARNs étant formé par

- un premier miARN choisi parmi hsa-miR-155, hsa-miR-210, hsa-miR-10a, hsa-miR-409 ou hsa-miR-134 ;
- un deuxième miARN choisi parmi hsa-miR-126, hsa-miR-15b, hsa-miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-151, hsa-miR-26b, hsa-miR-34b\*, hsa-miR-155, hsa-miR-210 ou hsa-miR-10a, ledit deuxième miARN choisi étant différent du susdit premier miARN choisi, et

\* lesdits couples de miARNs étant différents l'un de l'autre,

ladite mesure permettant d'établir les rapports des niveaux d'expression pour les susdits couples de miARNs,
(ii) la comparaison entre:

- les rapports des niveaux d'expression des susdits couples de miARNs, et
- ceux représentés dans un abaque préétabli pour un échantillon sain de référence, un échantillon de GBM de référence et un échantillon d'ODG de référence.

Dans un mode de réalisation avantageux, la déduction de la présence de GBM dans l'échantillon provenant du patient suspecté de présenter un gliome est faite, lorsque :

(i) la différence entre le premier rapport obtenu dans un échantillon provenant d'un patient suspecté de présenter un gliome et le rapport dans un abaque préétabli dans un échantillon de GBM de référence est inférieure à 20%, notamment 15%, plus particulièrement 10%, vis-à-vis du susdit premier rapport dans l'abaque préétabli, et
(ii) la différence entre le deuxième rapport obtenu dans un échantillon provenant d'un patient suspecté de présenter un gliome et le rapport dans un abaque préétabli dans un échantillon de GBM de référence est inférieure à 20%, notamment 15%, plus particulièrement 10%, vis-à-vis du susdit deuxième rapport dans l'abaque préétabli.

Dans un autre mode de réalisation particulier, la déduction de la présence d'ODG dans l'échantillon provenant du patient suspecté de présenter un gliome est faite, lorsque :

(i) la différence entre le premier rapport obtenu dans un échantillon provenant d'un patient suspecté de présenter un gliome et le rapport dans un abaque préétabli dans un échantillon d'ODG de référence est inférieure à 20%, notamment 15%, plus particulièrement 10%, vis-à-vis du susdit premier rapport dans l'abaque préétabli, et

(ii) la différence entre le deuxième rapport obtenu dans un échantillon provenant d'un patient suspecté de présenter un gliome et le rapport dans un abaque préétabli dans un échantillon d'ODG de référence est inférieure à 20%, notamment 15%, plus particulièrement 10%, vis-à-vis du susdit deuxième rapport dans l'abaque préétabli.

Selon un mode de réalisation particulièrement avantageux, dans la susdite méthode de diagnostic *in vitro* d'une tumeur cérébrale appartenant au groupe constitué de 2 types de tumeurs : ODG, GBM, et d'identification du type de la tumeur, le premier couple de miARNs est formé par un premier miARN : hsa-miR-155, et un deuxième miARN choisi parmi : hsa-miR-126, hsa-miR-15b, hsa-miR-16, hsa-miR-17, hsa-miR-20a, hsa-let 7f, hsa-miR-374, hsa-miR-409, hsa-miR-134, hsa-miR-339, hsa-miR-151, hsa-miR-26b, hsa-miR-34b* ou hsa-miR-210.

Selon un mode de réalisation particulièrement avantageux, dans la susdite méthode, le premier couple de miARNs est formé par un premier miARN : hsa-miR-210, et un deuxième miARN choisi parmi : hsa-miR-126, hsa-miR-15b, hsa-miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-26b, hsa-miR-34b*, hsa-miR-155, ou hsa-miR-10a.

Selon un mode de réalisation particulièrement avantageux, dans la susdite méthode, le premier couple de miARNs est formé par un premier miARN : hsa-miR-134, et un deuxième miARN choisi parmi : hsa-miR-126, hsa-miR-15b, hsa-miR-17, hsa-miR-20a, hsa-let7a, hsa-let 7f, hsa-let 7d, hsa-miR-374, hsa-miR-409, hsa-miR-26b, hsa-miR-155, hsa-miR-210 ou hsa-miR-10a.

Selon un mode de réalisation particulièrement avantageux, dans la susdite méthode, le premier couple de miARNs est formé par un premier miARN : hsa-miR-409, et un deuxième miARN choisi parmi : hsa-miR-126, hsa-miR-15b, hsa-miR-16, hsa-miR-17, hsa-miR-20a, hsa-let7a, hsa-let 7f, hsa-let 7d, hsa-miR-374, hsa-miR-134, hsa-miR-127, hsa-miR-26b, hsa-miR-155, hsa-miR-210 ou hsa-miR-10a.

Selon un mode de réalisation particulièrement avantageux, dans la susdite méthode, le premier couple de miARNs est formé par un premier miARN : hsa-miR-10a, et un deuxième miARN choisi parmi : hsa-miR-126, hsa-miR-15b, hsa-miR-16, hsa-miR-20a, hsa-let 7f, hsa-let 7d, hsa-miR-374, hsa-miR-409, hsa-miR-134, hsa-miR-339, hsa-miR-127, hsa-miR-151, hsa-miR-26b, hsa-miR-34b* ou hsa-miR-210.

Dans un mode de réalisation particulièrement avantageux, le premier couple de miARNs utilisé dans la susdite méthode est choisi parmi hsa-miR126/hsa-miR134, hsa-miR126/hsa-miR409, hsa-miR127/hsa-miR10a, hsa-miR134/hsa-let 7a, hsa-miR134/hsa-miR10a, hsa-miR151/hsa-miR10a, hsa-miR155/hsa-miR134, has-miR155/has-miR409, hsa-miR15b/hsa-miR409, hsa-miR16/hsa-miR-409, hsa-miR17/hsa-miR409, hsa-miR20a/hsa-miR409, hsa-miR210/hsa-let 7a, hsa-miR210/hsa-miR155, hsa-miR26b/hsa-miR134, hsa-miR26b/hsa-miR409, hsa-miR374/hsa-miR134, hsa-miR374/hsa-miR409, hsa-miR409/hsa-miR10a, hsa-miR10a/hsa let 7d, hsa-miR126/hsa-miR10a, hsa-miR126/hsa-miR155, hsa-miR134/hsa-miR17, hsa-miR134/hsa-miR409, hsa-miR155/hsa-miR339, hsa-miR15b/hsa-miR10a, hsa-miR15b/hsa-miR155, hsa-miR16/hsa-miR10a, hsa-miR16/hsa-miR155, hsa-miR20a/hsa-miR10a, hsa-miR210/hsa-miR127, hsa-miR210/hsa-miR134, hsa-miR210/hsa-miR339, hsa-miR210/hsa-miR409, hsa-miR26b/hsa-miR10a, hsa-miR26b/hsa-miR155, hsa-miR374/hsa-miR10a, hsa-miR10a/hsa-miR210, hsa-miR10a/hsa-miR339, hsa-miR10a/hsa-miR-34b*, hsa-miR155/hsa let 7f, hsa-miR16/hsa-miR210, hsa-miR210/hsa let 7d, hsa-miR210/hsa-miR126, hsa-miR210/hsa-miR15b, hsa-miR210/hsa-miR17, hsa-miR210/hsa-miR20a, hsa-miR210/hsa-miR374, hsa-miR26b/hsa-miR210, hsa-miR127/hsa-miR409, hsa-miR210/hsa-miR-34b*, hsa-let 7f/hsa-miR10a, hsa-miR155/hsa-miR17, hsa-miR155/hsa-miR374, hsa-miR20a/hsa-miR155, hsa-let 7a/hsa-miR 409, hsa-let 7d/hsa-miR409, hsa-miR134/hsa-let 7f, hsa-let 7f/hsa-miR409, hsa-miR134/hsa-let 7d, hsa-miR151/hsa-miR155, hsa-miR155/hsa-miR-34b*, hsa-miR15b/hsa-miR134, hsa-miR134/hsa-miR20a, hsa-miR210/hsa-let 7f.

Dans un autre mode de réalisation particulièrement avantageux, le deuxième couple de miARNs utilisé dans la susdite méthode est choisi parmi : hsa-miR127/hsa-miR409, hsa-miR210/hsa-miR-34b*, hsa-let 7f/hsa-miR10a, hsa-miR155/hsa-miR17, hsa-miR155/hsa-miR374, hsa-let 7a/hsa-miR 409, hsa-let 7d/hsa-miR409, hsa-miR134/hsa-let 7f, hsa-let 7f/hsa-miR409, hsa-miR134/hsa-let 7d, hsa-miR151/hsa-miR155, hsa-miR155/hsa-miR-34b*, hsa-miR15b/hsa-miR134, hsa-miR134/hsa-miR20a, hsa-miR210/hsa-let 7f, hsa-miR20a/has-miR155.

Selon un mode de réalisation particulièrement avantageux, dans la susdite méthode de diagnostic *in vitro* d'une tumeur cérébrale appartenant au groupe constitué de 2 types de tumeurs : ODG, GBM, et d'identification du type de la tumeur, le premier couple de miARNs est hsa-miR155/hsa-miR-34b*, le deuxième couple de miARNs est hsa-let 7a/hsa-miR 409.

Selon un mode de réalisation particulièrement avantageux, dans la susdite méthode, le premier couple de miARNs est hsa-miR151/hsa-miR155, le deuxième couple de miARNs est hsa-miR155/hsa-miR-34b* .

Selon un mode de réalisation particulièrement avantageux, dans la susdite méthode, le premier couple de miARNs est hsa-let 7d/hsa-miR409, le deuxième couple de miARNs est hsa-miR134/hsa-miR20a.

Selon un mode de réalisation particulièrement avantageux, dans la susdite méthode, le premier couple de miARNs est hsa-let 7a/hsa-miR 409, le deuxième couple de miARNs est hsa-miR134/hsa-let 7f.

Un mode de réalisation avantageux concerne une méthode de diagnotic *in vitro* comprenant la mesure de trois couples de miARNs respectivement dans un tissu sain de référence, un tissu d'ODG de référence et un tissu de GBM de référence.

Dans un mode de réalisation particulier, les trois couples de miARNs choisis dans cette méthode sont miR155/miR-34b*, miR134/miR20 et miR26b/miR134.

La présente invention est illustrée par les figures et les exemples suivants.

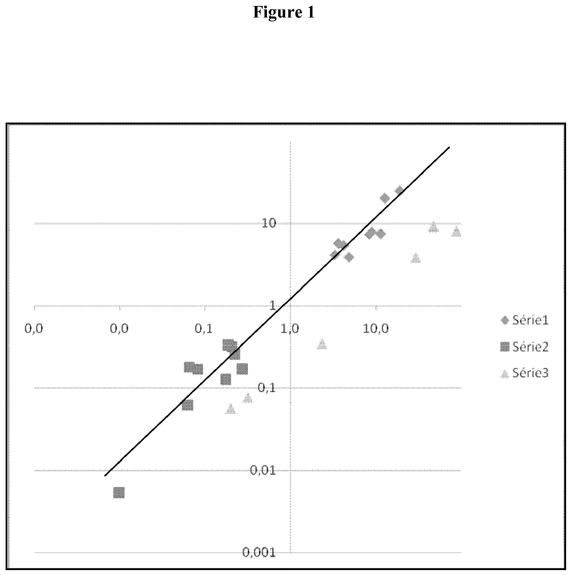

Figure 1 : La figure1 illustre les catégories de microARN surexprimés dans les gliomes par rapport au tissu sain (points losanges) ; les microARN sous-exprimés dans les gliomes par rapport au tissu sain (carrés); et les microARN surexprimés dans les glioblastomes (GBM) par rapport aux oligodendrogliomes (ODG) (triangles). L'axe des ordonnées représente les valeurs des ratios des quantités de chaque miARN dans les oligodendrogliomes par rapport à la quantité de ces miARN dans le tissu sain (ODG/N). L'axe des abscisses représente les valeurs des ratios des quantités de chaque miARN dans les glioblastomes par rapport à la quantité de ces miARN dans le tissu sain (GBM/N).

Figure 2 : La figure 2 illustre un clustering permettant de distinguer GBM, ODG, tissus sains sur la base des 12 ratios présents respectivement dans les tableaux 4, 5, 6, 7. Les valeurs des ratios pour 4 échantillons de tissu sain, 3 oligodendrogliomes et 4 glioblastomes permettent de construire un tri des tissus en trois groupes bien distincts et cohérents en termed'identité tissulaire (dendrogramme en haut du cluster-logiciel MeV-Multiexperiment viewer v4.4-TM4 software suite MeV team- http://www.TM4.org).

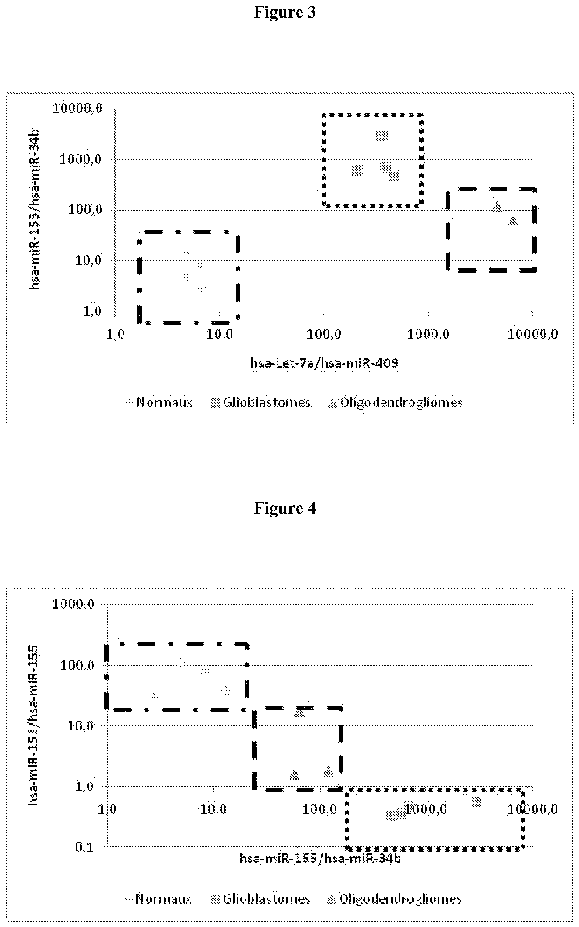

Figure 3 : La figure 3 illustre l'identification de tissus normaux (points losanges dans le rectangle tracé en tiret-pointés), glioblastomes (points carrés dans le rectangle en tracé pointillé) et des oligodendrogliomes (points triangles dans le rectangle tracé avec des tirets), par dosage de 4 miARNs (let 7a, miR409, miR155 et miR-34b*), et sur la base des valeurs des ratios let7a/miR409 et miR155/miR-34b*.

Figure 4 : La figure 4 illustre l'identification de tissus normaux (points losanges dans le rectangle tracé en tiret-pointés), des glioblastomes (points carrés dans le rectangle en tracé pointillé) et des oligodendrogliomes (points triangles dans le rectangle tracé avec des tirets), par dosage de 3 miARNs (miR155, miR-34b* et miR151), et sur la base des valeurs des ratios miR155/miR-34b* et miR151/miR155.

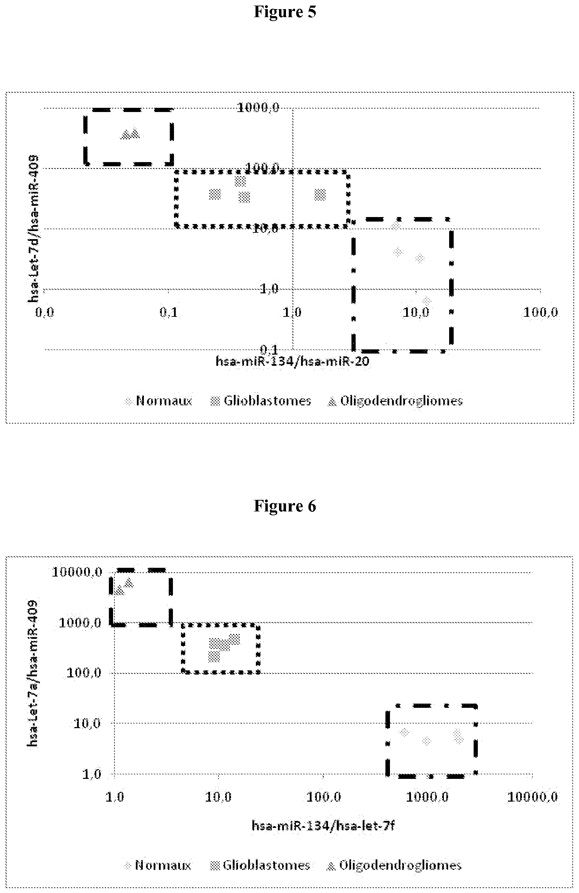

Figure 5 : La figure 5 illustre l'identification de tissus normaux (points losanges dans le rectangle tracé en tiret-pointés), des glioblastomes (points carrés dans le rectangle en tracé pointillé) et des oligodendrogliomes (points triangles dans le rectangle tracé avec des tirets), par dosage de 4 miARNs (let 7d, miR409, miR134 et miR20), et sur la base des valeurs des ratios let7d/miR409 et miR134/miR20.

Figure 6 : La figure 6 illustre l'identification de tissus normaux (points losanges dans le rectangle tracé en tiret-pointés), glioblastomes (points carrés dans le rectangle en tracé pointillé) et des oligodendrogliomes (points triangles dans le rectangle tracé avec des tirets), par dosage de 4 miARNs (let 7a, miR409, miR134 et let-7f), et sur la base des valeurs des ratios let7a/miR409 et miR134/let-7f.

Figure 7 : La figure 7 illustre les valeurs des ratios miR155/miR-34b* (série 1), miR134/miR20 (série 2) et miR26b/miR134 (série 3), exprimées en pourcentage (barres empilées 100%). Ces valeurs permettent de distinguer visuellement les oligodendrogliomes (lignes 9-11), les tissus normaux (lignes 5-8), et les glioblastomes (lignes 1-4).

Exemples

Exemple 1 : Matériels et Méthodes

Les échantillons tissulaires

Les échantillons tumoraux (8 glioblastomes et 4 oligodendrogliomes) ainsi que les échantillons de tissu sain (cortectomies) sont obtenus après exérèse par les neurochirurgiens au bloc opératoire et sont immédiatement congelés à -80°C. Des coupes de tissu d'environ $40\mu$m d'épaisseur sont ensuite réalisées à l'aide d'un cryotome en nombre suffisant afin d'obtenir environ 80mg de tissu et conservées à -80°C jusqu'à extraction des ARN.

Les liquides biologiques

Pour la purification des microARNs circulants dans le sang, des échantillons sanguins sont prélevés chez les patients sur des tubes de prélèvement de type PAXGene Blood RNA (PreAnalytix- Qiagen - BD company). La lyse totale des cellules circulantes est réalisée et les ARN sont collectés par centrifugation et purifiés selon les indications du fournisseur.

En ce qui concerne les fractions de microARN contenus dans les microvésicules produites par les tumeurs et présentes dans le sang, la méthode utilisée est directement basée sur celle décrite par Skog et coll (2008).

Les microvésicules sont purifiées à partir des sérums par centrifugations et microfiltration. Les sérums sont centrifugés une première fois pendant 10 min à 300g, puis les surnageants sont ensuite centrifugés pendant 20 min à 17000g et filtrés sur filtre de 0,22 microns. Les microvésicules sont obtenues par ultracentrifugation du filtrat à 110000g pendant 70 min. Le culot est enfin resuspendu en tampon phosphate (PBS) et les ARN sont extraits selon le protocole décrit ci-après.

L'extraction des ARN

Les ARNs sont extraits à l'aide du kit hsa-miRVana™ (Ambion, ABI) permettant de séparer les ARNs longs (taille> 250nt) des ARNs courts (taille < 250nt) en suivant les recommandations du fournisseur. Le tissu est tout d'abord lysé, les ARNs sont ensuite précipités après ajout d'acétate de sodium et d'éthanol, extraits en présence d'un mélange phénol/chloroforme, séparés selon leur taille sur colonne chromatographique, puis élués. Les ARNs élués sont ensuite quantifiés par mesure de la DO à 260nm à l'aide du spectrophotomètre Nanodrop ND-1000 et un contrôle qualité de ces ARN est réalisé par migration électrophorétique dans un gel de polymère sur le BioAnalyser 2100 grâce au kit RNA 6000 nano LabChip@(Agilent).

Le dosage des miARNs par PCR en temps réel

L'expression de miARNs est quantifiée par la technique de PCR quantitative à l'aide des kits distribués par Applied Biosystems, spécifiques des miARNs matures. Dans un premier temps, 80ng d'ARNs courts sont inversement transcrits (en cDNA monobrin) en présence d'amorces en boucle, qui confèrent la spécificité pour la quantification de l'expression des miARNs matures. La PCR en temps réel est ensuite réalisée à l'aide des amorces fournies dans les kits. Une des amorces comporte des groupements fluorescents (sonde dite Taqman@) ce qui permet de réaliser une mesure quantitative en utilisant un fluorimètre adapté tel que la station Stratagene Mx3005.Le seuil de détection est déterminé dans un premier temps par l'utilisateur en début de phase exponentielle. La valeur de Ct correspond au nombre de cycles à partir duquel la fluorescence dépasse ce seuil de détection. Cette valeur de Ct est proportionnelle à la quantité de cDNA présent initialement dans l'échantillon. En l'absence de gamme-étalon spécifique pour chaque cDNA, seule une quantification relative entre échantillons est possible. Les valeurs de dosage pour chaque miARN sont normalisées avec les données obtenues pour un ARN non-codant, RNU24.

Interprétation des résultats

Un niveau d'expression aberrant d'un miARN dans un tissu testé peut être un indicateur du caractère tumoral dudit tissu. Afin de déterminer si un niveau d'expression d'un miARN testé est normal, la comparaison peut être faite soit entre le tissu suspecté et les tissus de référence (la comparaison inter-tissus), soit entre le niveau d'expression du susdit miARN et le niveau d'expression d'un autre miARN dans le même tissu (la comparaison intra-tissu).

La comparaison inter-tissulaire du niveau d'expression d'un miARN X dans le tissu A et le tissu B est exprimée par le rapport$_{(échantillon\ A/échantillon\ B)}$, calculé d'après la formule (V) :

$$\text{Rapport}_{(\text{échantillon A/échantillon B})} = \text{Efficacité}^{-((\text{Ct hsa-miARNX} - \text{Ct RNU24})\text{échantillon A} - (\text{Ct hsa-miARNX} - \text{Ct RNU24})\text{échantillon B})}.$$

La comparaison intra-tissulaire entre le niveau d'expression d'un miARN X et celui d'un miARN Y est exprimée par le rapport$_{(\text{hsa-miARN X/ hsa-miARN Y})}$, calculé d'après la formule (VI) : $\text{Rapport}_{(\text{hsa-miARN X/ hsa-miARN Y})} = \text{Efficacité}^{-(\text{Ct hsa-miARN X} - \text{Ct hsa-miARN Y})}$.

Exemple 2 : Comparaison des niveaux d'expression des miARNs dans les tissus de tumeurs gliales et dans les tissus normaux de référence

La nature des tissus utilisés dans le présent exemple est préalablement confirmée par l'analyse histologique.

Les niveaux d'expression de 282 miARNs dans les tissus de glioblastomes, les tissus d'oligodendrogliome, les tissus sains de référence sont quantifiés respectivement par la PCR en temps réel selon la méthode décrite dans l'exemple 1.

Parmi 282 miARNs testés, l'expression de 19 miARNs est modifiée dans les glioblastomes et dans les oligodendrogliomes, par rapport à celle dans du tissu sain.

Le tableau 3 ci-dessous présente respectivement : les comparaisons inter-tissulaires pour les 19 miARN réalisées entre les glioblastomes de référence (GBM) et les tissus sains de référence (N), exprimées par le rapport$_{(GMB/N)}$; les comparaisons entre les oligodendrogliomes de référence (ODG) et les tissus sains de référence (N), exprimées par le rapport$_{(ODG/N)}$; et les comparaisons entre les glioblastomes de référence (GBM) et les oligodendrogliomes de référence (ODG), exprimées par le rapport$_{(GBM/ODG)}$.

Tableau 3

|  | GBM/N | ODG/N | GBM/ODG |
|---|---|---|---|
| hsa-miR-155 | 34,46 | 4,27 | 8,07 |
| **hsa-miR-210** | 2,35 | 0,29 | 8,1 |
| hsa-miR-10a | 47,00 | 9,20 | 5,1 |
| hsa-miR-134 | 0,20 | 0,06 | 3,63 |
| hsa-miR-409 | 0,321 | 0,065 | 4,94 |
| hsa-miR-374 | 7,76 | 7,35 | 1,06 |
| hsa-miR-126 | 4,84 | 3,90 | 1,24 |
| hsa-miR-15b | 3,31 | 4,16 | 0,80 |
| hsa-miR-16 | 11,43 | 6,31 | 1,81 |
| hsa-miR-17 | 12,87 | 20,32 | 0,63 |
| hsa-miR-20a | 3,63 | 5,73 | 0,63 |
| hsa-miR-26b | 8,95 | 7,86 | 1,14 |
| hsa-let 7a | 19,25 | 24,92 | 0,77 |
| hsa-let 7d | 4,19 | 4,57 | 0,92 |
| hsa- let 7f | 23,18 | 32,46 | 0,71 |
| hsa-miR-34b* | 0,21 | 0,32 | 0,67 |
| hsa-miR-127 | 0,06 | 0,05 | 1,21 |
| hsa-miR-151 | 0,23 | 0,26 | 0,89 |
| hsa-miR-339 | 0,07 | 0,18 | 0,39 |

12 miARNs, marqués en gris dans le tableau 3, sont surexprimés dans les GBM et les ODG.

6 autres miARNs, sans aucun marquage dans le tableau 3, sont sous-exprimés dans les GBM et les ODG.

Une exception est hsa-miR-210, marqué en gras dans le tableau 3, qui est surexprimé dans les GBM, mais sous-exprimé dans les ODG.

Parmi ces 19 miARNs repérés dans l'invention en raison de leur profil d'expression modifié dans les tissus de gliomes par rapport aux tissus normaux, 5 miARNs, dont hsa-miR-155, hsa-miR-210, hsa-miR-10a, hsa-miR-134, hsa-miR-409, montrent également un profil d'expression distinct entre les glioblastomes et les oligodendrogliomes. Ces 5 miARNs sont soulignés dans le tableau 3.

Exemple 3 : Comparaisons intra-tissulaires des niveaux d'expression des miARNs

La nature des tissus utilisés dans le présent exemple est préalablement confirmée par l'analyse histologique.

La comparaison est réalisée entre les niveaux d'expression de 2 miARNs (miARN X et miARN Y) issus d'un même tissu. Le résultat de cette comparaison est exprimé sous forme d'un rapport, qui est calculé selon la formule (VI). Les tableaux 4, 5, 6, 7 ci-dessous représentent le rapport $_{(hsa-miARN X/ hsa-miARN Y)}$ obtenu respectivement dans un tissu de glioblastome de référence (GBM), un tissu d'oligodendrogliome de référence (ODG), ou un tissu sain de référence (N). Les couples de miARNs sont sélectionnés sur la base de p-values inférieures à 0,05. (La p-value indique la significativité de chaque couple de miARNs (miARN X et miARN Y) pour les distinctions entre catégories de tissus. Une p-value inférieure à 0,05 est significative).

Tableau 4

| Couple de miARNs | Rapport dans N | Rapport dans GBM | Rapport dans ODG |
|---|---|---|---|
| hsa-let 7a/hsa-miR127 | 0,04 | 13,39 | 26,93 |
| hsa-let 7a/hsa-miR15b | 1,00 | 6,57 | 5,58 |
| hsa-let 7a/hsa-miR-34b* | 11,77 | 1108,71 | 5664,24 |
| hsa-let 7d/hsa-miR-34b* | 8,32 | 137,03 | 341,47 |
| hsa-miR126/hsa-let 7a | 7,02 | 2,30 | 2,08 |
| hsa-miR126/hsa-miR134 | 0,32 | 8,48 | 39,09 |
| hsa-miR126/hsa-miR339 | 2,37 | 948,81 | 283,44 |
| hsa-miR126/hsa-miR-34b* | 92,64 | 2446,73 | 1047,16 |
| hsa-miR126/hsa-miR409 | 38,66 | 699,88 | 6908,75 |
| hsa-miR127/hsa-let 7d | 68,45 | 1,09 | 0,99 |
| hsa-miR127/hsa-let 7f | 2065,79 | 9,19 | 6,29 |
| hsa-miR127/hsa-miR10a | 10839,87 | 28,13 | 72,27 |
| hsa-miR27/hsa-miR15b | 30,35 | 1,04 | 1,31 |
| hsa-miR127/hsa-miR-17 | 405,56 | 2,82 | 2,95 |
| hsa-miR134/hsa-let 7a | 21,26 | 0,21 | 0,08 |
| hsa-miR134/hsa-miR10a | 9255,37 | 31,37 | 31,47 |
| hsa-miR151/hsa-let 7a | 35,92 | 0,56 | 0,53 |
| hsa-miR151/hsa-let 7d | 181,76 | 5,35 | 4,57 |
| hsa-miR151/hsa-let 7f | 2941,11 | 25,08 | 14,37 |
| hsa-miR151/hsa-miR10a | 21981,20 | 78,57 | 333,08 |
| hsa-miR151/hsa-miR126 | 6,28 | 0,30 | 0,39 |
| hsa-miR151/hsa-miR15b | 42,05 | 2,42 | 2,62 |
| hsa-miR151/hsa-miR20a | 19,14 | 1,94 | 0,75 |
| hsa-miR151/hsa-miR374 | 73,06 | 2,11 | 1,72 |
| hsa-miR155/hsa-miR127 | 0,02 | 21,72 | 9,02 |
| hsa-miR155/hsa-miR134 | 0,03 | 4,95 | 4,62 |
| has-miR155/has-miR409 | 3,68 | 412,41 | 798,39 |
| hsa-miR15b/hsa-let 7f | 78,80 | 9,77 | 8,62 |
| hsa-miR15b/hsa-miR339 | 0,38 | 94,27 | 16,51 |
| hsa-miR15b/hsa-miR409 | 6,94 | 74,86 | 638,97 |
| hsa-miR16/hsa-miR127 | 1,24 | 409,04 | 529,94 |
| hsa-miR16/hsa-miR134 | 1,76 | 111,42 | 327,36 |
| hsa-miR16/hsa-miR151 | 1,31 | 67,29 | 33,17 |
| hsa-miR16/hsa-miR339 | 12,38 | 16977,61 | 1904,10 |
| hsa-miR16/hsa-miR-34b* | 489,53 | 34822,63 | 20260,67 |
| hsa-miR16/hsa-miR-409 | 211,30 | 9357,42 | 50575,30 |
| hsa-miR17/hsa-miR339 | 0,04 | 47,85 | 8,30 |

(suite)

| Couple de miARNs | Rapport dans N | Rapport dans GBM | Rapport dans ODG |
|---|---|---|---|
| hsa-miR17/hsa-miR-34b* | 1,40 | 106,08 | 185,81 |
| hsa-miR17/hsa-miR409 | 0,76 | 27,18 | 250,77 |
| hsa-miR20a/hsa-let 7a | 2,53 | 0,59 | 0,60 |
| hsa-miR20a/hsa-let 7f | 141,24 | 28,07 | 21,61 |
| hsa-miR20a/hsa-miR127 | 0,08 | 6,21 | 16,98 |
| hsa-miR20a/hsa-miR339 | 0,78 | 308,04 | 58,84 |
| hsa-miR20a/hsa-miR-34b* | 32,41 | 783,28 | 1528,75 |
| hsa-miR20a/hsa-miR409 | 13,92 | 188,77 | 1844,61 |
| hsa-miR210/hsa-let 7a | 14,30 | 2,24 | 0,31 |
| hsa-miR210/hsa-miR155 | 27,01 | 2,90 | 3,84 |
| hsa-miR26b/hsa-let 7f | 379,42 | 148,58 | 83,66 |
| hsa-miR26b/hsa-miR-127 | 0,20 | 54,39 | 103,49 |
| hsa-miR26b/hsa-miR134 | 0,31 | 13,96 | 59,85 |
| hsa-miR26b/hsa-miR151 | 0,23 | 8,54 | 5,76 |
| hsa-miR26b/hsa-miR-339 | 2,01 | 1777,46 | 375,39 |
| hsa-miR26b/hsa-miR-34b* | 73,80 | 3751,16 | 2921,07 |
| hsa-miR26b/hsa-miR409 | 36,68 | 1151,30 | 9627,57 |
| hsa-miR339/hsa-let 7a | 3,44 | 0,06 | 0,05 |
| hsa-miR339/hsa-let 7d | 8,08 | 0,41 | 0,25 |
| hsa-miR339/hsa-let 7f | 225,84 | 2,49 | 1,56 |
| hsa-miR-34b*/hsa-let 7f | 6,74 | 0,06 | 0,08 |
| hsa-miR374/hsa-let 7f | 41,18 | 15,54 | 8,72 |
| hsa-miR374/hsa-miR127 | 0,02 | 5,64 | 10,35 |
| hsa-miR374/hsa-miR134 | 0,04 | 1,37 | 6,35 |
| hsa-miR374/hsa-miR339 | 0,24 | 227,47 | 37,20 |
| hsa-miR374/hsa-miR-34b* | 9,42 | 374,55 | 387,50 |
| hsa-miR374/hsa-miR409 | 4,57 | 114,05 | 985,51 |
| hsa-miR409/hsa-miR10a | 78,10 | 0,48 | 0,40 |

Les couples de miARNs du tableau 4 permettent d'indiquer que le tissu suspecté est un tissu tumoral, lorsque le rapport du niveau d'expression entre les deux miARNs d'un couple est différent du susdit rapport établi dans un tissu sain de référence.

Tableau 5

| Couple de miARNs | Rapport dans N | Rapport dans GBM | Rapport dans ODG |
|---|---|---|---|
| hsa-let 7d/hsa let 7f | 39,38 | 7,44 | 5,17 |
| hsa-miR10a/hsa let 7d | 0,01 | 0,19 | 0,02 |
| hsa-miR126/hsa let 7f | 409,60 | 86,77 | 52,65 |
| hsa-miR126/hsa-miR10a | 3309,62 | 212,01 | 1322,35 |

(suite)

| Couple de miARNs | Rapport dans N | Rapport dans GBM | Rapport dans ODG |
|---|---|---|---|
| hsa-miR126/hsa-miR155 | 11,23 | 2,56 | 14,01 |
| hsa-miR127/hsa-miR126 | 6,28 | 0,18 | 0,25 |
| hsa-miR134/hsa-miR17 | 308,44 | 5,14 | 0,92 |
| hsa-miR134/hsa-miR409 | 117,50 | 76,90 | 105,39 |
| hsa-miR151/hsa-miR7 | 786,64 | 12,17 | 5,49 |
| hsa-miR155/hsa-miR339 | 0,23 | 658,78 | 33,40 |
| hsa-miR15b/hsa-miR10a | 480,59 | 28,27 | 216,27 |
| hsa-miR15b/hsa-miR155 | 2,49 | 0,26 | 12,60 |
| hsa-miR15b/hsa-miR-34b* | 17,58 | 237,79 | 813,26 |
| hsa-miR16/hsa-miR10a | 16307,76 | 2830,58 | 11326,64 |
| hsa-miR16/hsa-miR126 | 5,72 | 13,65 | 14,07 |
| hsa-miR16/hsa-miR155 | 63,55 | 27,68 | 301,21 |
| hsa-miR16/hsa-miR15b | 33,54 | 118,49 | 74,32 |
| hsa-miR16/hsa-miR20a | 15,54 | 66,32 | 21,88 |
| hsa-miR16/hsa-let 7f | 2208 | 1195 | 450 |
| hsa-miR16/hsa-miR374 | 54 | 80 | 52 |
| hsa-miR17/hsa let 7d | 0,18 | 0,66 | 0,74 |
| hsa-miR20a/hsa-miR10a | 973,95 | 63,63 | 520,31 |
| hsa-miR210/hsa-miR127 | 0,47 | 41,36 | 11,83 |
| hsa-miR210/hsa-miR134 | 0,76 | 11,49 | 6,43 |
| hsa-miR210/hsa-miR339 | 4,34 | 577,38 | 42,97 |
| hsa-miR210/hsa-miR409 | 85,51 | 825,50 | 1082,17 |
| hsa-miR26b/hsa let 7a | 6,38 | 3,46 | 3,02 |
| hsa-miR26b/hsa-miR10a | 2395,15 | 423,06 | 2043,29 |
| hsa-miR26b/hsa-miR155 | 11,10 | 3,44 | 42,63 |
| hsa-miR26b/hsa-miR15b | 5,92 | 14,88 | 14,85 |
| hsa-miR374/hsa-miR10a | 260,02 | 47,05 | 219,75 |
| hsa-miR10a/hsa-miR210 | 0,00 | 0,02 | 0,01 |
| hsa-miR10a/hsa-miR339 | 0,00 | 4,79 | 0,11 |
| hsa-miR10a/hsa-miR-34b* | 0,05 | 30,18 | 2,24 |
| hsa-miR155/hsa let 7f | 41,80 | 53,26 | 6,99 |
| hsa-miR16/hsa let 7a | 38,15 | 28,63 | 14,98 |
| hsa-miR16/hsa-miR210 | 3,45 | 41,60 | 60,43 |
| hsa-miR210/hsa let 7d | 40,01 | 20,29 | 3,03 |
| hsa-miR210/hsa-miR126 | 2,72 | 2,30 | 0,21 |
| hsa-miR210/hsa-miR15b | 14,39 | 13,77 | 1,53 |
| hsa-miR210/hsa-miR17 | 207,54 | 67,24 | 3,14 |
| hsa-miR210/hsa-miR20a | 6,74 | 10,76 | 0,43 |

(suite)

| Couple de miARNs | Rapport dans N | Rapport dans GBM | Rapport dans ODG |
|---|---|---|---|
| hsa-miR210/hsa-miR374 | 22,64 | 13,26 | 0,92 |
| hsa-miR26b/hsa-miR210 | 0,52 | 4,13 | 10,62 |

Les couples de miARNs du tableau 5 peuvent indiquer que le tissu suspecté est un des deux types de tissu tumoral, lorsque le rapport du niveau d'expression entre les deux miARNs d'un couple est différent du susdit rapport établi dans un tissu sain de référence.

Tableau 6

| Couple de miARNs | Rapport dans N | Rapport dans GBM | Rapport dans ODG |
|---|---|---|---|
| hsa-miR127/hsa-miR409 | 205,31 | 46,96 | 159,82 |
| hsa-miR210/hsa-miR-34b* | 147,47 | 1752,14 | 268,48 |
| hsa-let 7f/hsa-miR10a | 6,57 | 2,81 | 21,53 |
| hsa-miR155/hsa-miR17 | 10,56 | 26,58 | 2,85 |
| hsa-miR155/hsa-miR374 | 1,05 | 4,62 | 0,83 |
| Hsa-miR20a/hsa-miR155 | 4,34 | 0,74 | 23,47 |

Les couples de miARNs du tableau 6 permettent de distinguer un glioblastome d'un oligodendrogliome, lorsque le caractère tumoral d'un tissu suspecté est déjà confirmé par une méthode décrite dans la présente invention, ou une méthode conventionnelle.

Tableau 7

| Couple de miARNs | Rapport dans N | Rapport dans GBM | Rapport dans ODG |
|---|---|---|---|
| hsa-let 7a/hsa-miR 409 | 5,77 | 356,43 | 3726,97 |
| hsa-let 7d/hsa-miR409 | 4,75 | 41,99 | 275,42 |
| hsa-miR134/hsa-let 7f | 1067,08 | 10,55 | 1,58 |
| hsa-let 7f/hsa-miR409 | 0,11 | 7,47 | 81,66 |
| hsa-miR134/hsa-let 7d | 62,34 | 2,01 | 0,43 |
| hsa-miR151/hsa-miR155 | 62,89 | 0,44 | 6,93 |
| hsa-miR155/hsa-miR-34b* | 7,27 | 1186,37 | 80,50 |
| hsa-miR15b/hsa-miR134 | 0,06 | 0,93 | 5,98 |
| hsa-miR134/hsa-miR20a | 8,61 | 0,40 | 0,07 |
| hsa-miR210/hsa-let 7f | 944,34 | 129,36 | 8,61 |

Les couples de miARNs du tableau 7 ci-dessus permettent de préciser directement la nature d'un tissu suspecté de présenter un caractère tumoral et de distinguer qu'il s'agit d'un glioblastome ou d'un oligodendrogliome, sans besoin d'effectuer une étape de détermination de la présence de gliomes dans ce tissu suspecté.

Les figures 3 à 6 illustrent que 3 groupes d'échantillons de nature différente, dont les tissus sains, les tissus de glioblastomes, ou les tissus d'oligodendrogliomes, sont distingués sur un graphique à deux dimensions établi à l'aide de deux couples de miARNs.

La figure 7 montre que 3 groupes d'échantillons de nature différente, dont les tissus sains, les tissus de glioblastomes, ou les tissus d'oligodendrogliomes, sont bien différenciés sur un graphique à trois dimensions, établi à l'aide de trois couples de miARNs.

Le tableau 8 ci-après regroupe les couples de ratios de microARNs utilisables pour le diagnostic des tumeurs.

A partir des tableaux 5, 6 et 7 - qui listent des ratios entre les 19 miARNs faisant partie de l'invention, lesquels ratios ont un potentiel de distinction entre les types de tissus - l'intégralité des couples de ratios a été créée et listée dans le tableau 8. On obtient 1753 couples de ratios différents mentionnés selon la représentation suivante : miRA/miRB|miRC/miRD (soit le couple associant le ratio des miRA et miRB au ratio des miRC et miRD).

Ces couples de ratios permettent de réaliser une projection sur un graphique à deux dimensions des valeurs mesurées dans chaque tissu à analyser ou chaque tissu de référence selon le principe suivant, et à titre d'exemple :

Les valeurs des ratios miRA/miRB sont portées en abscisse du graphique et les valeurs des ratios miRC/miRD sont portées en ordonnées.

Il est entendu que pour ces couples de ratios, leurs représentations, et leurs significations diagnostiques restent inchangées que l'on utilise les ratios ou leurs inverses (exemple miRA/miRB ou miRB/miRA) et que l'on reporte l'un des ratios en abscisse et le deuxième en ordonnée ou l'inverse.

Des exemples de ces représentations sont montrés dans les figures 3 à 6.

A partir du listing complet (Tableau 8), il est possible d'évaluer la performance de ces différents couples de ratios pour une démarche diagnostique en procédant de la manière suivante :

Les ratios sont calculés pour un lot d'échantilllon de tissu de glioblastome et on calcule la moyenne de la valeur de chaque ratio pour ce lot d'échantillon.

Les ratios sont calculés pour un lot d'échantilllon de tissu d'oligodendrogliome et on calcule la moyenne de la valeur de chaque ratio pour ce lot d'échantillon.

Les ratios sont calculés pour un lot d'échantilllon de tissu sains et on calcule la moyenne de la valeur de chaque ratio pour ce lot d'échantillon.

Pour le premier ratio de microARNs, on calcule le rapport (A) entre la valeur du ratio mesuré dans chaque échantillon individuel de tissu de glioblastome et la valeur moyenne de ce ratio pour les échantillons sains.

On calcule le rapport (B) entre la valeur du ratio mesuré dans chaque échantillon individuel de tissu de glioblastomes et la valeur moyenne de ce ratio pour les échantillons de tissu d'oligodendrogliome.

On calcule le rapport (C) entre la valeur du ratio mesuré dans chaque échantillon individuel de tissu sain et la valeur moyenne de ce ratio pour les échantillons de tissu d'oligodendrogliome.

Pour le deuxième ratio de microARNs, on calcule le rapport (W) entre la valeur du ratio mesuré dans chaque échantillon individuel de tissu de glioblastome et la valeur moyenne de ce ratio pour les échantillons sains.

On calcule le rapport (X) entre la valeur du ratio mesuré dans chaque échantillon individuel de tissu de glioblastomes et la valeur moyenne de ce ratio pour les échantillons de tissu d'oligodendrogliome.

On calcule le rapport (Y) entre la valeur du ratio mesuré dans chaque échantillon individuel de tissu sain et la valeur moyenne de ce ratio pour les échantillons de tissu d'oligodendrogliome.

On sélectionne ensuite les couples de ratios pour lesquels les valeurs des ratios A, B, C, W, X et Y sont pour tous les échantillons toujours supérieurs à une valeur seuil, par exemple 40, ou toujours inférieurs à une valeur seuil, par exemple 0,025. Dans cet exemple, les couples de ratios qui répondent à ces critères sont au nombre de 11. Ils sont représentés en soulignés dans le tableau 8.

Ce sont les couples de ratios dont les valeurs des ratios A, B, C, W, X et Y sont supérieurs à des valeurs seuils les plus grandes possibles ou inférieures à des valeurs seuils les plus faibles possibles qui présentent la meilleure capacité à permettre la distinction graphique entre les échantillons de tissus sains, de tissus de glioblastome et de tissus d'oligodendrogliome.

Tableau 8

| | | |
|---|---|---|
| miR15b/miR134\|miR134/Let7f | miR26b/miR155\|miR210/Let7f | miR210/miR134\|miR210/miR127 |
| miR15b/miR134\|miR134/Let7d | miR210/Let7d\|miR151/miR17 | miR15b/miR34b\|Let7f/miR10a |
| miR15b/miR134\|miR134/miR20a | miR16/miR210\|miR16/miR20a | miR20a/miR155\|miR127/miR126 |
| miR134/miR20a\|Let7a/miR409 | miR26b/miR210\|miR26b/miR15b | miR210/miR339\|miR126/miR155 |
| miR134/miR17\|Let7a/miR409 | miR210/miR34b\|miR151/miR17 | miR210/miR4091miR155/Let7f |
| miR134/miR20a\|Let7f/miR409 | miR20a/miR155\|miR16/miR15b | miR16/miR126\|miR15b/miR34b |
| miR134/Let7f\|Let7a/miR409 | miR20a/miR10a\|miR16/miR126 | miR155/Let7f\|miR134/miR409 |
| miR134/Let7d\|Let7f/miR409 | miR26b/miR155\|miR134/miR409 | miR210/miR339\|Let7f/miR10a |
| miR134/Let7f\|Let7d/miR409 | miR15b/miR134\|Let7d/miR409 | miR26b/miR155\|Let7a/miR409 |
| miR134/miR17\|Let7f/miR409 | miR26b/miR210\|miR210/miR20a | miR210/miR126\|miR127/miR126 |
| miR134/Let7f\|Let7f/miR409 | miR26b/Let7a\|miR127/miR409 | miR210/miR409\|miR127/miR409 |
| miR210/Let7f\|Let7a/miR409 | miR16/miR126\|miR155/Let7f | miR15b/miR34b\|Let7a/miR409 |

(suite)

| | | |
|---|---|---|
| miR210/Let7f\|Let7f/miR409<br>miR15b/miR134\|miR126/miR10a | miR16/miR155\|miR126/Let7f<br>miR15b/miR34b\|miR134/miR409 | miR210/miR409\|miR17/Let7d<br>miR151/miR155\|miR127/miR409 |

| | | |
|---|---|---|
| miR10a/miR339\|Let7d/miR10a | miR210/miR374\|miR134/miR17 | miR210/miR339\|Let7a/miR409 |
| miR17/Let7d\|miR16/miR126 | miR210/miR126\|miR16/miR15b | miR210/miR409\|Let7d/miR10a |
| miR15b/miR134\|miR151/miR17 | miR26b/Let7a\|miR17/Let7d | miR16/Let7a\|miR155/miR17 |
| miR26b/miR210\|miR16/miR126 | miR210/miR3391miR210/Let7f | miR16/miR374\|miR155/Let7f |
| miR210/miR134\|miR16/miR126 | miR26b/miR210\|miR126/miR155 | miR210/miR17\|miR15b/miR10a |
| miR210/miR127\|miR16/miR126 | miR16/miR126\|miR127/miR409 | miR134/miR17\|miR126/Let7f |
| miR15b/miR134\|miR126/Let7f | miR210/miR134\|miR134/miR20a | miR26b/miR155\|miR210/miR339 |
| miR15b/miR134\|miR134/miR17 | miR210/miR339\|miR134/miR409 | miR26b/miR210\|miR155/miR339 |
| miR17/Let7d\|miR126/miR10a | miR210/Let7f\|miR16/miR126 | miR210/miR409\|miR151/miR155 |
| miR10a/miR339\|Let7d/Let7f | miR26b/Let7a\|Let7d/miR10a | miR210/miR374\|miR16/miR15b |
| miR15b/miR134\|miR15b/miR10a | miR16/miR155\|miR134/miR17 | miR26b/Let7a\|miR16/miR155 |
| miR20a/miR155\|miR16/miR126 | miR26b/miR210\|Let7f/miR10a | miR210/miR20a\|miR155/miR34b |
| miR210/miR126\|miR16/miR126 | miR374/miR10a\|miR126/miR10a | miR210/miR134\|miR15b/miR155 |
| miR26b/miR210\|miR126/miR10a | miR15b/miR155\|miR134/miR20a | miR16/miR374\|miR127/miR409 |
| miR17/Let7d\|miR16/Let7f | miR26b/Let7a\|miR151/miR155 | miR151/miR155\|Let7d/miR10a |
| miR155/miR339\|miR126/miR10a | miR20a/miR155\|miR155/miR17 | miR26b/miR15b\|miR155/miR34b |
| miR10a/miR339\|Let7f/miR10a | miR16/miR126\|Let7d/miR10a | miR26b/miR155\|miR15b/miR34b |
| miR10a/miR339\|Let7a/miR409 | miR134/miR409\|miR134/Let7f | miR210/miR15b\|miR16/miR210 |
| miR16/miR210\|miR16/miR126 | miR155/miR339\|miR126/miR155 | miR210/miR34b\|miR210/Let7f |
| miR26b/miR210\|miR26b/miR10a | Let7d/miR409\|Let7d/Let7f | miR210/miR134\|Let7d/miR409 |
| miR17/Let7d\|miR151/miR17 | miR210/Let7d\|miR134/Let7f | miR210/Let7d\|miR134/miR409 |
| miR26b/miR210\|miR16/Let7f | miR210/miR134\|Let7d/Let7f | miR26b/miR15b\|miR210/miR20a |
| miR210/miR134\|miR126/miR10a | miR17/Let7d\|miR16/miR155 | miR126/miR155\|Let7f/miR10a |
| miR15b/miR155\|miR126/miR10a | miR16/miR126\|miR151/miR155 | miR16/miR374\|Let7d/miR10a |
| miR17/Let7d\|miR134/Let7f | miR155/miR339\|Let7f/miR10a | miR210/miR34b\|miR134/miR409 |
| miR10a/miR34b\|Let7d/miR10a | miR210/miR34b\|miR134/Let7f | miR155/Let7f\|miR127/miR409 |
| miR210/miR127\|miR126/miR10a | miR26b/Let7a\|miR210/miR409 | miR210/miR20a\|miR126/miR155 |
| miR26b/miR210\|miR151/miR17 | miR26b/miR210\|Let7a/miR409 | miR210/miR126\|miR210/Let7d |
| miR155/miR339\|miR151/miR17 | miR210/miR126\|miR155/miR17 | miR16/miR210\|miR155/miR374 |
| miR26b/Let7a\|miR16/miR126 | miR16/Let7a\|miR15b/miR10a | miR210/miR339\|miR15b/miR34b |
| miR16/miR126\|miR16/miR126 | miR26b/miR155\|miR155/Let7f | miR15b/miR155\|Let7d/miR409 |
| miR210/miR134\|miR16/Let7f | miR15b/miR155\|Let7d/Let7f | miR16/miR374\|miR151/miR155 |
| miR26b/miR210\|miR134/Let7f | miR15b/miR34b\|miR155/Let7f | miR127/miR409\|Let7d/miR10a |
| miR155/miR339\|miR134/Let7f | miR20a/miR155\|miR16/Let7a | miR26b/miR15b\|miR126/miR155 |
| miR26b/miR155\|miR16/miR126 | miR210/miR409\|miR20a/miR155 | Let7f/miR10a\|Let7a/miR409 |
| miR210/miR339\|miR16/miR126 | miR26b/miR155\|miR127/miR409 | miR155/miR374\|miR127/miR126 |
| miR210/miR127\|miR16/Let7f | miR155/miR339\|Let7a/miR409 | miR210/miR374\|miR155/miR17 |
| miR210/miR134\|miR151/miR17 | miR15b/miR34b\|miR127/miR409 | miR210/miR20a\|Let7f/miR10a |
| miR15b/miR134\|miR134/miR409 | miR210/miR339\|miR155/Let7f | miR210/Let7f\|miR20a/miR155 |
| miR15b/miR155\|miR151/miR17 | miR210/miR126\|miR16/Let7a | miR155/miR34b\|miR126/miR155 |
| miR210/miR134\|miR134/Let7f | miR26b/miR155\|miR17/Let7d | miR134/miR20a\|Let7d/Let7f |
| miR210/miR20a\|miR16/miR126 | miR26b/miR210\|miR210/miR339 | miR26b/miR15b\|Let7f/miR10a |
| miR20a/miR155\|miR126/miR10a | miR210/miR127\|miR134/miR20a | miR126/miR155\|Let7a/miR409 |
| miR26b/miR210\|miR20a/miR155 | miR26b/miR155\|Let7d/miR10a | miR155/Let7f\|Let7d/miR10a |
| miR15b/miR134\|miR155/Let7f | miR210/miR339\|miR127/miR409 | miR155/miR34b\|Let7f/miR10a |
| miR15b/miR155\|miR134/Let7f | miR134/miR20a\|miR134/Let7d | miR16/miR210\|miR127/miR126 |
| miR26b/miR15b\|miR16/miR126 | miR210/miR20a\|miR210/Let7f | miR16/miR155\|miR155/miR17 |
| miR210/miR127\|miR151/miR17 | miR15b/miR34b\|Let7d/miR10a | miR26b/miR10a\|miR16/Let7f |

(suite)

| | | |
|---|---|---|
| miR210/miR126\|miR126/miR10a | miR26b/miR15b\|miR210/miR34b | miR155/Let7f\|miR151/miR155 |
| miR15b/miR134\|miR127/miR409 | miR26b/miR210\|miR15b/miR34b | miR210/miR20a\|Let7a/miR409 |
| miR17/Let7d\|miR126/Let7f | miR210/miR20a\|miR134/miR409 | miR210/miR374\|miR16/Let7a |
| miR15b/miR134\|Let7d/miR10a | miR26b/miR155\|miR151/miR155 | miR26b/miR155\|miR16/miR155 |
| miR15b/miR134\|miR151/miR155 | miR26b/miR15b\|miR210/Let7f | miR151/miR17\|miR134/Let7f |
| miR17/Let7d\|miR134/miR17 | miR210/miR339\|miR17/Let7d | miR26b/miR15b\|Let7a/miR409 |

| | | |
|---|---|---|
| miR127/miR126\|miR126/miR10a | miR16/miR210\|miR16/miR15b | miR210/miR127\|miR15b/miR155 |
| miR210/miR127\|miR134/Let7f | miR26b/miR210\|miR26b/miR155 | miR155/miR34b\|Let7a/miR409 |
| miR155/miR374\|miR126/miR10a | miR210/miR374\|miR15b/miR10a | miR210/Let7d\|miR155/Let7f |
| miR16/miR210\|miR126/miR10a | miR15b/miR34b\|miR151/miR155 | miR210/miR34b\|miR155/Let7f |
| miR20a/miR155\|miR16/Let7f | miR15b/miR134\|miR155/miR339 | miR26b/miR155\|miR26b/Let7a |
| miR210/miR134\|miR20a/miR155 | miR26b/miR15b\|miR134/miR409 | miR16/miR155\|miR16/Let7a |
| miR210/miR126\|miR16/Let7f | miR16/miR15b\|miR126/Let7f | miR134/miR409\|miR127/miR409 |
| miR26b/miR210\|miR126/Let7f | miR210/miR339\|Let7d/miR10a | miR210/miR339\|miR16/miR155 |
| miR17/Let7d\|miR15b/miR10a | miR155/miR34b\|miR134/miR409 | miR210/miR127\|Let7d/miR409 |
| miR155/miR339\|miR126/Let7f | miR374/miR10a\|miR26b/miR10a | miR210/Let7d\|miR127/miR409 |
| miR26b/miR210\|miR134/miR17 | miR210/miR127\|Let7d/Let7f | miR210/miR34b\|miR127/miR409 |
| miR155/miR339\|miR134/miR17 | miR26b/miR155\|miR210/miR409 | miR16/Let7f\|miR151/miR17 |
| Let7f/miR409\|Let7d/miR10a | miR16/miR155\|miR15b/miR10a | miR26b/miR15b\|miR210/miR339 |
| miR210/miR127\|miR20a/miR155 | miR210/miR339\|miR151/miR155 | miR210/Let7d\|miR17/Let7d |
| miR20a/miR155\|miR151/miR17 | miR210/miR134\|miR155/miR34b | miR134/miR409\|Let7d/miR10a |
| miR210/miR126\|miR151/miR17 | miR155/miR374\|miR155/miR17 | miR210/miR34b\|miR17/Let7d |
| miR16/miR210\|miR16/Let7f | miR210/miR374\|miR210/miR17 | miR210/miR20a\|miR15b/miR34b |
| miR16/miR20a\|miR16/miR126 | miR16/miR15b\|miR134/miR17 | miR210/miR409\|miR16/miR20a |
| miR26b/Let7a\|miR126/miR10a | miR15b/miR155\|miR155/miR34b | miR210/Let7d\|Let7d/miR10a |
| miR210/miR15b\|miR16/miR126 | miR210/miR17\|miR126/miR10a | miR210/miR34b\|Let7d/miR10a |
| miR26b/miR210\|miR15b/miR10a | miR16/miR210\|miR155/miR17 | miR26b/miR15b\|miR15b/miR34b |
| miR20a/miR155\|miR134/Let7f | miR26b/Let7a\|miR16/miR20a | miR26b/miR210\|miR20a/miR10a |
| miR16/miR126\|miR126/miR10a | miR20a/miR155\|miR16/miR210 | miR210/Let7d\|miR151/miR155 |
| miR210/miR134\|miR126/Let7f | miR210/miR134\|miR126/miR155 | miR26b/miR15b\|miR26b/miR155 |
| miR210/miR126\|miR134/Let7f | miR26b/miR210\|miR16/miR155 | miR16/miR15b\|miR16/miR15b |
| miR26b/miR210\|miR210/miR17 | miR374/miR10a\|miR16/Let7f | miR210/miR34b\|miR151/miR155 |
| miR15b/miR155\|miR126/Let7f | miR210/miR20a\|miR155/Let7f | miR210/miR134\|miR155/miR339 |
| miR155/miR374\|miR151/miR17 | miR210/miR134\|Let7f/miR10a | miR15b/miR155\|miR155/miR339 |
| miR210/miR134\|miR134/miR17 | miR15b/miR155\|miR126/miR155 | miR26b/miR10a\|miR151/miR17 |
| miR10a/miR339\|Let7d/miR409 | miR15b/miR10a\|miR126/miR10a | miR374/miR10a\|miR134/Let7d |
| miR15b/miR134\|miR155/miR17 | miR26b/miR15b\|miR155/Let7f | miR134/miR20a\|miR126/miR155 |
| miR15b/miR155\|miR134/miR17 | miR210/miR126\|miR16/miR210 | miR210/miR374\|miR210/miR15b |
| miR16/miR210\|miR151/miR17 | Let7f/miR10a\|Let7d/miR10a | miR16/miR374\|miR16/miR20a |
| miR26b/miR155\|miR126/miR10a | Let7f/miR409\|Let7d/miR409 | miR210/miR374\|miR16/miR210 |
| miR15b/miR34b\|miR126/miR10a | miR155/miR34b\|miR155/Let7f | Let7d/Let7f\|Let7a/miR409 |
| miR10a/miR34b\|Let7d/Let7f | miR26b/miR210\|miR26b/Let7a | miR16/Let7f\|miR134/Let7f |
| miR210/miR339\|miR126/miR10a | miR16/miR210\|miR16/Let7a | miR16/miR20a\|miR134/miR20a |
| miR210/miR127\|miR126/Let7f | miR210/miR20a\|miR127/miR409 | miR210/miR15b\|miR134/miR20a |
| miR155/miR374\|miR134/Let7f | miR15b/miR155\|Let7f/miR10a | miR20a/miR10a\|miR126/Let7f |
| miR16/miR210\|miR134/Let7f | miR126/miR155\|Let7d/miR10a | miR134/miR20a\|Let7f/miR10a |
| miR210/miR127\|miR134/miR17 | miR26b/miR15b\|miR127/miR409 | miR16/miR15b\|miR155/miR17 |
| miR26b/Let7a\|miR16/Let7f | Let7d/miR409\|Let7a/miR409 | miR210/miR20a\|miR16/miR155 |
| miR210/miR374\|miR16/miR126 | miR210/miR20a\|miR17/Let7d | miR26b/Let7a\|miR16/miR374 |
| miR210/miR134\|miR15b/miR10a | miR155/miR34b\|miR127/miR409 | miR26b/miR15b\|miR16/miR155 |

(suite)

| | | |
|---|---|---|
| miR16/miR126\|miR16/Let7f | miR210/miR134\|Let7a/miR409 | miR20a/miR10a\|miR134/miR17 |
| miR15b/miR155\|miR15b/miR10a | miR26b/miR15b\|miR17/Let7d | miR210/Let7f\|miR126/Let7f |
| miR126/miR155\|miR126/miR10a | miR210/miR20a\|Let7d/miR10a | miR210/miR10a\|miR126/miR10a |
| miR20a/miR155\|miR20a/miR155 | miR210/Let7d\|miR20a/miR155 | miR16/miR20a\|Let7d/Let7f |
| miR16/miR155\|miR16/miR126 | miR210/miR127\|miR155/miR34b | miR26b/Let7a\|miR155/miR374 |
| miR26b/miR155\|miR26b/miR10a | miR210/miR34b\|miR20a/miR155 | miR210/miR15b\|Let7d/Let7f |
| miR17/Let7d\|miR134/Let7d | miR15b/miR155\|Let7a/miR409 | miR26b/miR15b\|miR26b/Let7a |
| miR210/miR20a\|miR126/miR10a | miR26b/miR15b\|Let7d/miR10a | miR16/miR15b\|miR16/Let7a |
| miR210/miR126\|miR20a/miR155 | miR210/miR20a\|miR151/miR155 | miR26b/miR10a\|miR134/Let7f |
| miR26b/miR15b\|miR126/miR10a | miR155/miR34b\|Let7d/miR10a | miR210/miR127\|miR155/miR339 |
| miR155/miR34b\|miR126/miR10a | miR210/miR409\|miR126/Let7f | miR16/miR126\|miR155/miR374 |
| miR10a/miR34b\|Let7f/miR10a | miR26b/miR15b\|miR151/miR155 | miR210/Let7f\|miR134/miR17 |
| miR26b/miR155\|miR16/Let7f | miR155/miR34b\|miR151/miR155 | miR26b/Let7a\|miR127/miR126 |
| miR210/miR127\|miR15b/miR10a | miR26b/miR155\|miR16/miR20a | miR134/Let7d\|miR127/miR409 |
| miR26b/Let7a\|miR151/miR17 | miR17/Let7d\|miR16/miR374 | miR210/miR409\|miR16/miR15b |
| miR10a/miR34b\|Let7a/miR409 | miR210/miR127\|miR126/miR155 | miR16/miR126\|miR127/miR126 |
| miR210/miR339\|miR16/Let7f | miR151/miR155\|miR126/Let7f | miR134/Let7d\|Let7d/miR10a |
| miR16/miR126\|miR151/miR17 | miR26b/miR15b\|miR210/miR409 | miR26b/miR155\|miR16/miR374 |
| miR26b/miR15b\|miR26b/miR10a | miR16/miR15b\|miR15b/miR10a | miR210/miR409\|miR210/miR374 |
| miR16/miR15b\|miR16/miR126 | miR210/miR409\|miR134/miR17 | miR210/Let7d\|miR16/miR20a |
| miR26b/Let7a\|miR134/Let7f | miR17/Let7d\|miR155/miR374 | miR210/miR34b\|miR16/miR20a |
| miR26b/miR210\|miR134/Let7d | miR210/miR134\|miR15b/miR34b | miR16/Let7a\|miR134/miR20a |
| miR26b/miR155\|miR151/miR17 | miR210/miR127\|Let7f/miR10a | miR26b/miR155\|miR155/miR374 |
| miR16/miR126\|miR134/Let7f | miR16/miR20a\|miR134/Let7d | miR15b/miR34b\|miR155/miR374 |
| miR15b/miR34b\|miR151/miR17 | miR210/miR15b\|miR134/Let7d | miR210/miR17\|miR134/Let7d |
| miR134/miR20a\|miR126/miR10a | miR210/miR339\|miR16/miR20a | miR210/miR134\|miR20a/miR10a |
| miR17/Let7d\|miR134/miR409 | miR374/miR10a\|miR151/miR17 | miR210/miR339\|miR16/miR374 |
| miR155/miR3391miR134/Let7d | miR16/miR374\|miR126/Let7f | miR16/miR374\|miR16/miR15b |
| miR210/miR20a\|miR16/Let7f | miR151/miR155\|miR134/miR17 | miR26b/Let7a\|miR210/miR126 |
| miR20a/miR155\|miR126/Let7f | miR16/miR210\|miR16/miR210 | miR374/miR10a\|miR210/miR34b |
| miR210/miR339\|miR151/miR17 | miR210/miR127\|Let7a/miR409 | miR210/miR409\|miR155/miR17 |
| miR26b/miR15b\|miR16/Let7f | miR26b/Let7a\|miR16/miR15b | miR16/miR20a\|miR155/miR34b |
| miR210/miR126\|miR126/Let7f | miR127/miR409\|miR126/Let7f | miR210/miR15b\|miR155/miR34b |
| miR20a/miR155\|miR134/miR17 | miR17/Let7d\|miR127/miR126 | miR374/miR10a\|miR210/Let7f |
| miR210/miR126\|miR134/miR17 | miR134/miR17\|miR126/miR10a | miR155/miR17\|miR134/miR20a |
| miR26b/miR155\|miR134/Let7f | miR210/miR17\|miR16/Let7f | miR16/Let7a\|Let7d/Let7f |
| miR15b/miR134\|Let7d/Let7f | miR16/miR374\|miR134/miR17 | miR374/miR10a\|miR134/miR409 |
| miR15b/miR34b\|miR134/Let7f | miR155/Let7f\|miR126/Let7f | miR210/miR339\|miR155/miR374 |
| miR17/Let7d\|miR155/Let7f | miR26b/Let7a\|miR210/miR374 | miR26b/Let7a\|miR210/Let7d |
| miR210/miR339\|miR134/Let7f | miR134/miR20a\|miR127/miR409 | miR20a/miR10a\|miR15b/miR10a |
| miR210/miR409\|miR16/miR126 | miR210/miR134\|miR16/miR155 | miR20a/miR155\|miR15b/miR155 |
| miR127/miR126\|miR126/Let7f | miR20a/miR155\|miR134/miR20a | miR15b/miR134\|miR10a/miR34b |
| miR17/Let7d\|miR127/miR409 | miR155/Let7f\|miR134/miR17 | miR26b/miR155\|miR127/miR126 |
| miR17/Let7d\|miR17/Let7d | miR374/miR10a\|miR134/Let7f | miR15b/miR10a\|miR134/Let7d |
| miR17/Let7d\|Let7d/miR10a | miR26b/Let7a\|miR155/miR17 | miR15b/miR34b\|miR127/miR126 |
| miR210/miR20a\|miR151/miR17 | miR210/miR127\|miR15b/miR34b | miR210/miR409\|miR16/Let7a |
| miR155/miR374\|miR126/Let7f | miR210/miR126\|miR134/miR20a | miR16/miR20a\|miR126/miR155 |
| miR26b/miR210\|miR210/miR34b | miR134/miR20a\|Let7d/miR10a | miR210/miR15b\|miR126/miR155 |
| miR26b/miR15b\|miR151/miR17 | miR16/miR126\|miR155/miR17 | miR210/miR126\|miR15b/miR155 |
| miR26b/miR210\|miR210/Let7f | miR210/miR20a\|miR16/miR20a | miR210/miR374\|miR134/miR20a |

| | | |
|---|---|---|
| miR17/Let7d\|miR151/miR155 | miR20a/miR155\|Let7d/Let7f | miR20a/miR155\|Let7d/miR409 |
| miR155/miR34b\|miR151/miR17 | miR16/Let7a\|miR134/Let7d | miR155/miR17\|Let7d/Let7f |
| miR16/miR374\|miR16/miR126 | miR26b/miR15b\|miR16/miR20a | miR210/miR339\|miR127/miR126 |
| miR26b/miR210\|miR134/miR409 | miR26b/miR10a\|miR16/miR126 | miR16/miR374\|miR155/miR17 |
| miR16/miR210\|miR126/Let7f | miR26b/miR155\|miR16/miR15b | miR16/miR20a\|Let7f/miR10a |
| miR210/miR134\|miR134/Let7d | miR26b/miR210\|miR16/miR374 | miR210/Let7f\|miR15b/miR10a |
| miR155/miR374\|miR134/miR17 | miR26b/Let7a\|miR16/Let7a | miR210/miR15b\|Let7f/miR10a |
| miR26b/Let7a\|miR20a/miR155 | miR210/miR409\|miR15b/miR10a | miR134/Let7f\|miR126/Let7f |
| miR20a/miR155\|miR15b/miR10a | miR134/miR409\|miR126/Let7f | miR16/miR155\|miR134/miR20a |
| miR15b/miR134\|miR155/miR34b | miR210/Let7d\|miR126/Let7f | miR210/miR126\|Let7d/miR409 |
| miR15b/miR155\|miR134/Let7d | miR210/miR126\|Let7d/Let7f | miR210/miR10a\|miR16/Let7f |
| miR155/miR339\|miR134/miR409 | miR210/miR34b\|miR126/Let7f | miR210/miR374\|Let7d/Let7f |
| miR16/miR210\|miR134/miR17 | miR16/miR126\|miR16/Let7a | miR16/miR20a\|Let7a/miR409 |
| miR16/miR20a\|miR126/miR10a | miR26b/miR210\|miR155/miR374 | miR16/miR374\|miR16/Let7a |

| | | |
|---|---|---|
| miR210/miR126\|miR15b/miR10a | miR26b/miR155\|miR210/miR374 | miR210/miR15b\|Let7a/miR409 |
| miR210/miR15b\|miR126/miR10a | miR155/miR17\|miR134/Let7d | miR26b/miR155\|miR210/miR126 |
| miR210/miR20a\|miR134/Let7f | miR210/miR339\|miR16/miR15b | miR374/miR10a\|miR155/Let7f |
| miR15b/miR134\|miR126/miR155 | miR134/miR409\|miR134/miR17 | miR210/miR127\|miR20a/miR10a |
| miR26b/miR15b\|miR134/Let7f | miR210/miR409\|miR210/miR17 | miR210/miR20a\|miR16/miR374 |
| miR155/miR34b\|miR134/Let7f | miR210/miR17\|miR151/miR17 | miR16/miR155\|Let7d/Let7f |
| miR15b/miR134\|Let7f/miR10a | miR210/miR15b\|miR210/Let7f | miR374/miR10a\|miR127/miR409 |
| miR26b/miR210\|miR155/Let7f | miR210/Let7d\|miR134/miR17 | miR26b/miR10a\|miR20a/miR155 |
| miR26b/miR210\|miR127/miR409 | miR210/miR127\|miR16/miR155 | miR26b/miR155\|miR210/Let7d |
| miR155/miR339\|miR155/Let7f | miR210/miR34b\|miR134/miR17 | miR26b/miR15b\|miR16/miR374 |
| miR210/miR127\|miR134/Let7d | miR16/miR20a\|miR134/miR409 | miR210/Let7d\|miR16/miR15b |
| miR15b/miR134\|Let7a/miR409 | miR210/miR15b\|miR134/miR409 | miR16/Let7a\|miR155/miR34b |
| miR26b/miR210\|miR17/Let7d | miR26b/miR210\|miR127/miR126 | miR210/miR339\|miR210/miR126 |
| miR26b/miR155\|miR20a/miR155 | miR26b/miR155\|miR155/miR17 | miR210/miR34b\|miR16/miR15b |
| miR210/Let7d\|miR16/miR126 | miR16/miR374\|miR15b/miR10a | miR374/miR10a\|miR17/Let7d |
| miR210/miR34b\|miR16/miR126 | miR15b/miR34b\|miR155/miR17 | miR210/miR20a\|miR155/miR374 |
| miR26b/miR210\|Let7d/miR10a | miR210/miR374\|miR134/Let7d | miR151/miR17\|miR126/Let7f |
| miR155/miR339\|miR127/miR409 | miR15b/miR10a\|miR151/miR17 | miR127/miR126\|Let7d/miR409 |
| miR26b/miR210\|miR151/miR155 | miR155/miR374\|miR134/miR20a | miR26b/miR15b\|miR155/miR374 |
| Let7f/miR409\|Let7d/Let7f | miR127/miR126\|Let7d/Let7f | miR374/miR10a\|Let7d/miR10a |
| miR210/miR339\|miR20a/miR155 | miR155/miR339\|miR127/miR126 | miR210/miR339\|miR210/Let7d |
| miR17/Let7d\|miR16/miR20a | miR210/miR339\|miR155/miR17 | miR16/miR20a\|miR15b/miR34b |
| miR155/miR339\|Let7d/miR10a | miR16/miR155\|miR134/Let7d | miR210/miR409\|miR210/miR15b |
| miR16/Let7a\|miR126/miR10a | miR26b/miR155\|miR16/Let7a | miR374/miR10a\|miR151/miR155 |
| miR26b/miR210\|miR210/miR409 | miR20a/miR155\|miR155/miR34b | miR210/miR15b\|miR15b/miR34b |
| miR16/miR210\|miR15b/miR10a | miR16/miR210\|miR134/miR20a | miR210/miR409\|miR16/miR210 |
| miR155/miR339\|miR151/miR155 | miR26b/Let7a\|miR210/miR15b | miR16/Let7a\|miR126/miR155 |
| miR210/miR134\|miR210/Let7f | miR210/miR17\|miR134/Let7f | miR134/miR17\|miR134/Let7d |
| miR210/miR134\|miR134/miR409 | miR155/miR374\|Let7d/Let7f | miR210/miR17\|miR210/Let7f |
| miR155/miR17\|miR126/miR10a | miR26b/Let7a\|miR16/miR210 | miR210/miR20a\|miR127/miR126 |
| miR15b/miR155\|miR134/miR409 | miR10a/miR34b\|Let7f/miR409 | miR151/miR17\|miR134/miR17 |
| miR134/miR20a\|miR134/Let7f | miR210/miR126\|miR155/miR34b | miR16/miR210\|miR15b/miR155 |
| miR16/miR20a\|miR16/Let7f | miR210/miR339\|miR16/Let7a | miR210/miR17\|miR134/miR409 |
| miR26b/Let7a\|miR126/Let7f | miR26b/miR210\|miR210/miR126 | miR155/miR374\|Let7d/miR409 |
| miR210/miR15b\|miR16/Let7f | miR16/miR20a\|miR155/Let7f | miR26b/miR15b\|miR127/miR126 |
| miR210/miR374\|miR126/miR10a | miR210/miR15b\|miR155/Let7f | miR16/Let7a\|Let7f/miR10a |

(suite)

| | | |
|---|---|---|
| miR16/miR126\|miR126/Let7f | miR210/miR20a\|miR16/miR15b | miR210/Let7d\|miR155/miR17 |
| miR26b/Let7a\|miR134/miR17 | miR20a/miR10a\|miR126/miR10a | miR374/miR10a\|miR210/miR409 |
| miR210/miR20a\|miR20a/miR155 | miR20a/miR155\|miR126/miR155 | miR155/miR34b\|miR127/miR126 |
| miR16/miR155\|miR126/miR10a | miR16/miR210\|Let7d/Let7f | miR210/miR34b\|miR155/miR17 |
| miR26b/miR15b\|miR20a/miR155 | miR26b/miR210\|miR210/Let7d | miR210/miR10a\|miR151/miR17 |
| miR210/miR134\|miR155/Let7f | miR16/miR20a\|miR127/miR409 | miR210/miR374\|miR155/miR34b |
| miR16/miR126\|miR134/miR17 | miR15b/miR10a\|miR134/Let7f | miR20a/miR155\|miR155/miR339 |
| miR210/miR127\|miR210/Let7f | miR26b/miR15b\|miR16/miR15b | miR16/miR210\|Let7d/miR409 |
| miR15b/miR155\|miR155/Let7f | miR210/miR15b\|miR127/miR409 | miR155/miR17\|miR126/miR155 |
| miR210/miR134\|miR127/miR409 | miR20a/miR155\|Let7f/miR10a | miR15b/miR10a\|miR134/miR409 |
| miR210/miR127\|miR134/miR409 | miR374/miR10a\|miR20a/miR155 | miR16/Let7a\|Let7a/miR409 |
| miR210/miR134\|miR17/Let7d | miR210/miR126\|miR126/miR155 | miR210/miR374\|miR210/miR20a |
| miR26b/miR210\|miR16/miR20a | miR210/miR15b\|miR17/Let7d | miR16/miR374\|miR16/miR210 |
| miR15b/miR155\|miR127/miR409 | miR16/Let7a\|miR134/miR409 | miR155/miR17\|Let7f/miR10a |
| miR17/Let7d\|miR16/miR15b | miR26b/miR15b\|miR210/miR374 | miR210/Let7d\|miR16/Let7a |
| Let7d/miR409\|Let7d/miR10a | miR210/miR126\|Let7f/miR10a | miR16/miR155\|miR155/miR34b |
| Let7f/miR409\|Let7f/miR10a | miR210/Let7f\|miR126/miR10a | miR210/miR126\|miR155/miR339 |
| miR26b/miR155\|miR126/Let7f | miR16/miR20a\|Let7d/miR10a | miR210/miR34b\|miR16/Let7a |
| miR210/miR134\|Let7d/miR10a | miR210/Let7d\|miR15b/miR10a | miR16/miR15b\|miR134/miR20a |
| miR15b/miR34b\|miR126/Let7f | miR210/miR15b\|Let7d/miR10a | miR16/miR20a\|miR16/miR155 |
| miR210/miR134\|miR151/miR155 | miR134/Let7d\|miR126/Let7f | miR210/miR20a\|miR210/miR126 |
| miR15b/miR155\|Let7d/miR10a | miR210/miR34b\|miR15b/miR10a | miR210/miR374\|miR126/miR155 |
| miR16/miR20a\|miR151/miR17 | miR210/miR134\|miR16/miR374 | miR210/miR15b\|miR16/miR155 |
| Let7f/miR409\|Let7a/miR409 | miR20a/miR155\|Let7a/miR409 | miR26b/miR15b\|miR210/miR126 |
| miR210/miR15b\|miR151/miR17 | miR16/miR20a\|miR151/miR155 | miR155/miR17\|Let7a/miR409 |
| miR15b/miR155\|miR151/miR155 | miR210/miR15b\|miR151/miR155 | miR210/miR374\|Let7f/miR10a |
| miR210/miR339\|miR126/Let7f | miR210/miR20a\|miR155/miR17 | miR210/miR17\|miR155/Let7f |
| miR26b/miR155\|miR134/miR17 | miR155/miR17\|miR134/miR409 | miR210/miR20a\|miR210/Let7d |
| miR15b/miR34b\|miR134/miR17 | miR26b/miR15b\|miR155/miR17 | miR16/Let7f\|miR126/Let7f |
| miR17/Let7d\|miR155/miR17 | miR210/miR134\|miR155/miR374 | miR16/miR10a\|miR126/miR10a |
| miR210/miR127\|miR155/Let7f | miR210/miR126\|Let7a/miR409 | miR16/miR155\|miR126/miR155 |
| miR210/miR339\|miR134/miR17 | miR26b/miR155\|miR210/miR15b | miR16/miR15b\|Let7d/Let7f |
| miR210/miR127\|miR127/miR409 | miR26b/miR155\|miR16/miR210 | miR26b/miR15b\|miR210/Let7d |
| miR26b/Let7a\|miR15b/miR10a | miR155/miR34b\|miR155/miR17 | miR17/Let7d\|Let7f/miR409 |
| miR20a/miR155\|miR134/Let7d | miR210/miR34b\|miR210/miR17 | miR210/miR10a\|miR134/Let7f |
| miR17/Let7d\|miR16/Let7a | miR15b/miR155\|miR155/miR374 | miR16/miR155\|Let7f/miR10a |
| miR210/miR127\|miR17/Let7d | miR210/miR374\|miR210/miR34b | miR210/miR17\|miR127/miR409 |
| miR16/miR126\|miR15b/miR10a | miR155/miR374\|miR155/miR34b | miR16/Let7a\|miR15b/miR34b |
| miR16/miR15b\|miR126/miR10a | miR127/miR126\|miR126/miR155 | miR210/miR374\|Let7a/miR409 |
| miR210/miR374\|miR16/Let7f | miR210/miR374\|miR210/Let7f | miR15b/miR10a\|miR155/Let7f |
| miR210/miR127\|Let7d/miR10a | miR210/miR20a\|miR16/Let7a | miR210/miR17\|miR17/Let7d |
| miR210/miR126\|miR134/Let7d | miR16/miR15b\|miR134/Let7d | miR16/Let7f\|miR134/miR17 |
| miR16/miR20a\|miR134/Let7f | miR210/miR339\|miR210/miR15b | miR210/miR17\|Let7d/miR10a |
| miR26b/Let7a\|miR210/miR17 | miR210/miR374\|miR134/miR409 | miR15b/miR10a\|miR127/miR409 |
| miR210/miR15b\|miR134/Let7f | miR26b/miR15b\|miR16/Let7a | miR26b/Let7a\|miR210/miR127 |
| miR210/miR127\|miR151/miR155 | miR210/miR339\|miR16/miR210 | miR16/miR155\|Let7a/miR409 |
| miR126/miR155\|miR126/Let7f | miR127/miR126\|Let7f/miR10a | miR210/miR17\|miR151/miR155 |
| miR16/miR155\|miR16/Let7f | miR210/miR134\|miR127/miR126 | miR26b/miR10a\|miR126/Let7f |
| miR10a/miR339\|Let7f/miR409 | miR16/Let7a\|miR155/Let7f | miR374/miR10a\|miR16/miR20a |
| miR26b/miR210\|miR16/miR15b | miR16/miR210\|miR155/miR34b | miR210/miR374\|miR210/miR339 |

44

(suite)

| | | |
|---|---|---|
| miR210/miR20a\|miR126/Let7f | miR20a/miR155\|miR15b/miR34b | miR155/miR374\|miR155/miR339 |
| miR16/Let7a\|miR151/miR17 | miR15b/miR155\|miR127/miR126 | miR134/miR20a\|miR127/miR126 |
| miR26b/miR15b\|miR126/Let7f | miR16/miR155\|miR134/miR409 | miR15b/miR10a\|Let7d/miR10a |
| miR374/miR10a\|miR16/miR126 | miR155/miR374\|miR126/miR155 | miR210/Let7d\|miR16/miR210 |
| miR155/miR34b\|miR126/Let7f | miR16/Let7a\|miR127/miR409 | miR210/miR34b\|miR210/miR15b |
| miR26b/miR210\|miR210/miR374 | miR210/miR126\|miR15b/miR34b | miR210/miR34b\|miR16/miR210 |
| miR26b/miR155\|miR15b/miR10a | miR127/miR126\|Let7a/miR409 | miR16/miR210\|miR155/miR339 |
| miR210/miR20a\|miR134/miR17 | miR155/miR374\|Let7f/miR10a | miR26b/miR10a\|miR134/miR17 |
| miR15b/miR34b\|miR15b/miR10a | miR134/miR17\|miR134/Let7f | miR210/miR374\|miR15b/miR34b |
| miR210/miR134\|miR16/miR20a | miR155/miR17\|miR155/Let7f | miR15b/miR10a\|miR151/miR155 |
| miR26b/miR15b\|miR134/miR17 | miR210/miR127\|miR16/miR374 | miR210/miR409\|miR134/miR20a |
| miR155/miR17\|miR151/miR17 | miR16/miR210\|miR126/miR155 | miR16/miR15b\|miR155/miR34b |
| miR155/miR34b\|miR134/miR17 | mir16/Let7a\|Let7d/miR10a | miR16/miR155\|miR15b/miR34b |
| miR210/miR339\|miR15b/miR10a | miR16/miR210\|Let7f/miR10a | miR20a/miR155\|miR20a/miR10a |
| miR26b/miR210\|miR155/miR17 | miR155/miR17\|miR127/miR409 | miR151/miR155\|miR134/miR20a |
| miR26b/miR155\|miR210/miR17 | miR210/miR127\|miR155/miR374 | miR26b/Let7a\|miR15b/miR155 |
| miR15b/miR134\|miR155/miR374 | miR16/Let7a\|miR151/miR155 | miR20a/miR10a\|miR134/Let7d |
| miR17/Let7d\|miR16/miR210 | miR210/miR134\|miR210/miR126 | miR210/miR409\|Let7d/Let7f |
| miR155/miR374\|miR134/Let7d | miR155/miR374\|Let7a/miR409 | miR26b/miR155\|miR210/miR127 |
| miR210/miR374\|miR151/miR17 | miR26b/Let7a\|miR134/miR20a | miR210/miR126\|miR20a/miR10a |
| miR16/Let7a\|miR134/Let7f | miR20a/miR10a\|miR16/Let7f | miR26b/Let7a\|miR210/miR134 |
| miR155/miR339\|miR155/miR17 | miR210/miR374\|miR155/Let7f | miR16/miR126\|miR15b/miR155 |

| | | |
|---|---|---|
| miR16/miR210\|miR134/Let7d | miR210/miR17\|miR20a/miR155 | miR16/miR15b\|miR126/miR155 |
| miR210/miR339\|miR210/miR17 | miR20a/miR155\|miR16/miR155 | miR16/miR374\|miR134/miR20a |
| miR210/miR409\|miR126/miR10a | miR155/miR17\|Let7d/miR10a | miR26b/Let7a\|Let7d/miR409 |
| miR26b/miR210\|miR16/Let7a | miR210/miR134\|miR210/Let7d | miR26b/miR210\|Let7f/miR409 |
| miR15b/miR134\|miR127/miR126 | miR16/miR126\|miR134/miR20a | miR151/miR155\|Let7d/Let7f |
| miR16/miR155\|miR151/miR17 | miR16/miR210\|Let7a/miR409 | miR16/Let7f\|miR15b/miR10a |
| miR151/miR155\|miR126/miR10a | miR210/miR374\|miR127/miR409 | miR16/miR15b\|Let7f/miR10a |
| miR20a/miR155\|miR134/miR409 | miR16/miR155\|miR155/Let7f | miR210/miR374\|miR16/miR155 |
| miR155/miR17\|miR134/Let7f | miR134/Let7f\|miR126/miR10a | miR210/miR339\|miR210/miR127 |
| miR210/miR126\|miR210/Let7f | miR210/miR127\|miR127/miR126 | miR210/Let7f\|miR134/Let7d |
| miR16/miR15b\|miR16/Let7f | miR155/miR17\|miR151/miR155 | miR16/miR126\|Let7d/miR409 |
| miR210/miR127\|miR16/miR20a | miR16/miR20a\|miR16/miR20a | miR126/miR10a\|miR126/Let7f |
| miR210/miR126\|miR134/miR409 | miR210/miR20a\|miR210/miR15b | miR16/miR10a\|miR16/Let7f |
| miR16/miR374\|miR126/miR10a | miR210/miR374\|miR17/Let7d | Let7d/miR10a\|Let7d/Let7f |
| miR210/miR374\|miR134/Let7f | miR210/miR15b\|miR16/miR20a | miR210/miR10a\|miR210/miR10a |
| miR127/miR409\|miR126/miR10a | miR210/miR20a\|miR16/miR210 | miR134/miR17\|miR127/miR409 |
| miR210/miR20a\|miR15b/miR10a | miR210/miR126\|miR16/miR155 | miR155/Let7f\|miR134/miR20a |
| miR210/miR15b\|miR20a/miR155 | miR210/Let7f\|miR16/Let7f | miR155/miR339\|Let7f/miR409 |
| miR134/miR20a\|miR126/Let7f | miR210/miR10a\|miR16/miR126 | miR16/miR155\|miR16/miR155 |
| miR210/miR17\|miR16/miR126 | miR26b/Let7a\|Let7d/Let7f | miR16/miR374\|Let7d/Let7f |
| miR16/miR155\|miR134/Let7f | miR26b/miR15b\|miR210/miR15b | miR16/miR15b\|Let7a/miR409 |
| miR155/Let7f\|miR126/miR10a | miR16/miR155\|miR127/miR409 | miR127/miR409\|Let7d/Let7f |
| miR26b/miR15b\|miR15b/miR10a | miR374/miR10a\|miR126/Let7f | miR134/miR17\|Let7d/miR10a |
| miR210/miR134\|miR16/miR15b | miR17/Let7d\|miR15b/miR155 | miR374/miR10a\|miR16/miR15b |
| miR20a/miR155\|miR155/Let7f | miR26b/miR15b\|miR16/miR210 | miR210/miR10a\|miR20a/miR155 |
| miR210/miR20a\|miR210/miR17 | miR210/miR374\|Let7d/miR10a | miR210/miR15b\|miR16/miR374 |
| miR134/miR20a\|miR134/miR17 | miR210/miR409\|miR134/Let7d | miR210/miR17\|miR16/miR20a |
| miR26b/miR15b\|miR210/miR17 | miR16/miR126\|Let7d/Let7f | miR26b/miR10a\|miR15b/miR10a |

(suite)

| | | |
|---|---|---|
| miR10a/miR34b\|Let7d/miR409 | miR210/miR374\|miR151/miR155 | miR26b/miR155\|miR15b/miR155 |
| miR26b/miR210\|miR210/miR15b | miR17/Let7d\|Let7d/miR409 | miR155/Let7f\|Let7d/Let7f |
| miR20a/miR155\|miR127/miR409 | miR16/miR155\|Let7d/miR10a | miR15b/miR34b\|miR15b/miR155 |
| miR26b/miR210\|miR16/miR210 | miR374/miR10a\|miR134/miR17 | miR374/miR10a\|miR210/miR374 |
| miR210/miR126\|miR155/Let7f | miR151/miR155\|miR134/Let7d | miR16/miR20a\|miR155/miR374 |
| miR20a/miR155\|miR17/Let7d | miR16/miR210\|miR15b/miR34b | miR210/miR15b\|miR155/miR374 |
| miR210/miR126\|miR127/miR409 | miR16/miR155\|miR151/miR155 | miR26b/miR155\|miR210/miR134 |
| miR155/miR374\|miR134/miR409 | miR26b/miR155\|miR134/miR20a | miR17/Let7d\|miR15b/miR134 |
| miR20a/miR155\|Let7d/miR10a | miR210/miR127\|miR210/miR126 | miR126/Let7f\|Let7d/miR10a |
| miR210/miR134\|miR155/miR17 | miR16/miR15b\|miR134/miR409 | miR210/miR409\|miR155/miR34b |
| miR210/miR126\|miR17/Let7d | miR15b/miR34b\|miR134/miR20a | miR26b/miR155\|Let7d/miR409 |
| miR16/miR15b\|miR151/miR17 | miR151/miR17\|miR126/miR10a | miR210/miR339\|miR15b/miR155 |
| miR134/miR409\|miR126/miR10a | miR16/miR374\|miR134/Let7d | miR26b/miR10a\|miR210/miR17 |
| miR20a/miR155\|miR151/miR155 | miR26b/miR210\|miR210/miR127 | miR15b/miR34b\|Let7d/miR409 |
| miR17/Let7d\|miR134/miR20a | miR210/miR127\|miR210/Let7d | miR16/miR15b\|miR15b/miR34b |
| miR15b/miR155\|miR155/miR17 | miR20a/miR10a\|miR151/miR17 | miR134/miR409\|miR134/miR20a |
| miR16/miR210\|miR134/miR409 | miR210/miR339\|miR134/miR20a | miR210/miR409\|miR210/miR20a |
| miR210/Let7d\|miR126/miR10a | miR26b/miR155\|Let7d/Let7f | miR210/miR339\|miR210/miR134 |
| miR210/miR126\|Let7d/miR10a | miR15b/miR34b\|Let7d/Let7f | miR374/miR10a\|miR155/miR17 |
| miR210/miR409\|miR16/Let7f | miR155/Let7f\|miR134/Let7d | miR16/miR20a\|miR127/miR126 |
| miR210/miR34b\|miR126/miR10a | miR26b/Let7a\|miR155/miR34b | miR210/miR15b\|miR127/miR126 |
| miR210/miR126\|miR151/miR155 | miR210/Let7f\|miR151/miR17 | miR210/Let7d\|miR134/miR20a |
| miR210/miR134\|miR16/Let7a | miR210/miR339\|Let7d/Let7f | miR210/miR34b\|miR134/miR20a |
| miR210/miR127\|miR16/miR15b | miR16/miR210\|miR16/miR155 | miR210/miR339\|Let7d/miR409 |
| miR17/Let7d\|Let7d/Let7f | miR26b/Let7a\|miR210/miR20a | miR210/miR20a\|miR210/miR127 |
| miR26b/Let7a\|miR134/Let7d | miR16/miR126\|miR155/miR34b | miR210/miR409\|miR126/miR155 |
| miR155/miR374\|miR155/Let7f | miR15b/miR134\|Let7f/miR409 | miR26b/miR15b\|miR210/miR127 |
| miR16/miR374\|miR16/Let7f | miR16/miR15b\|miR155/Let7f | miR16/miR10a\|miR151/miR17 |
| miR16/miR126\|miR134/Let7d | miR16/miR20a\|miR16/miR15b | miR16/miR374\|miR155/miR34b |
| miR16/miR15b\|miR134/Let7f | miR26b/Let7a\|miR126/miR155 | miR210/miR409\|Let7f/miR10a |
| miR127/miR126\|Let7d/miR10a | miR210/miR15b\|miR16/miR15b | miR26b/Let7a\|miR155/miR339 |
| miR155/miR374\|miR127/miR409 | miR26b/miR210\|miR15b/miR155 | miR134/miR409\|Let7d/Let7f |
| miR16/miR210\|miR155/Let7f | miR16/miR15b\|miR127/miR409 | miR374/miR10a\|miR16/Let7a |
| miR210/miR127\|miR155/miR17 | miR20a/miR10a\|miR134/Let7f | miR151/miR155\|miR126/miR155 |
| miR210/miR374\|miR20a/miR155 | miR26b/Let7a\|Let7f/miR10a | miR20a/miR10a\|miR134/miR409 |
| miR16/miR210\|miR127/miR409 | miR210/miR17\|miR126/Let7f | miR210/Let7d\|Let7d/Let7f |
| miR16/miR20a\|miR126/Let7f | miR16/miR126\|miR126/miR155 | miR210/miR34b\|Let7d/Let7f |
| miR210/miR15b\|miR126/Let7f | miR374/miR10a\|miR15b/miR10a | miR16/miR126\|miR155/miR339 |
| miR155/miR374\|Let7d/miR10a | miR26b/miR210\|miR210/miR134 | miR151/miR155\|Let7f/miR10a |
| miR210/miR127\|miR16/Let7a | miR17/Let7d\|miR155/miR339 | miR210/miR409\|Let7a/miR409 |
| miR155/miR374\|miR151/miR155 | miR210/miR409\|miR210/miR34b | miR134/Let7f\|miR134/Let7d |
| miR210/miR409\|miR151/miR17 | miR26b/miR210\|Let7d/miR409 | miR210/Let7f\|miR210/Let7f |
| miR16/miR210\|Let7d/miR10a | miR16/Let7f\|miR126/miR10a | miR16/miR374\|miR126/miR155 |
| miR16/miR20a\|miR134/miR17 | miR16/miR126\|Let7f/miR10a | miR210/miR15b\|miR210/miR126 |
| miR26b/miR155\|miR134/Let7d | miR134/miR409\|miR134/Let7d | miR16/miR15b\|miR16/miR155 |
| miR210/miR15b\|miR134/miR17 | miR210/miR409\|miR210/Let7f | miR210/miR134\|Let7f/miR409 |
| miR210/miR134\|miR16/miR210 | miR210/miR374\|miR16/miR20a | miR210/Let7f\|miR134/miR409 |
| miR15b/miR34b\|miR134/Let7d | miR16/miR15b\|Let7d/miR10a | Let7f/miR10a\|Let7d/miR409 |
| miR16/miR210\|miR151/miR155 | miR210/Let7d\|miR134/Let7d | miR16/miR374\|Let7f/miR10a |
| miR17/Let7d\|miR155/miR34b | miR210/miR20a\|miR134/miR20a | miR127/miR409\|miR126/miR155 |

(suite)

| | | |
|---|---|---|
| miR26b/miR210\|miR134/miR20a | miR210/Let7f\|miR134/Let7f | miR16/Let7a\|miR155/miR374 |
| miR20a/miR155\|miR16/miR20a | miR210/miR17\|miR134/miR17 | miR151/miR155\|Let7a/miR409 |
| miR210/miR339\|miR134/Let7d | miR210/miR409\|miR134/miR409 | miR210/miR20a\|miR15b/miR155 |
| miR134/Let7d\|miR126/miR10a | miR15b/miR10a\|miR126/Let7f | miR15b/miR155\|Let7f/miR409 |
| miR210/miR126\|miR16/miR20a | miR26b/Let7a\|Let7a/miR409 | miR210/miR15b\|miR210/Let7d |
| miR155/miR339\|miR134/miR20a | Let7f/miR10a\|Let7d/Let7f | miR126/miR155\|Let7d/miR409 |
| miR16/miR374\|miR151/miR17 | miR210/miR34b\|miR134/Let7d | miR127/miR409\|Let7f/miR10a |
| miR210/Let7d\|miR16/Let7f | miR16/miR15b\|miR151/miR155 | miR210/miR17\|miR16/miR15b |
| miR210/miR34b\|miR16/Let7f | miR26b/miR155\|miR155/miR34b | Let7d/miR10a\|Let7a/miR409 |
| miR17/Let7d\|miR126/miR155 | miR16/miR20a\|miR155/miR17 | miR16/miR10a\|miR134/Let7f |
| miR26b/miR210\|Let7d/Let7f | miR26b/miR15b\|miR134/miR20a | miR155/Let7f\|miR126/miR155 |
| miR17/Let7d\|Let7f/miR10a | miR210/miR15b\|miR155/miR17 | miR210/miR409\|miR210/miR339 |
| miR210/miR409\|miR134/Let7f | miR15b/miR34b\|miR155/miR34b | miR26b/miR15b\|miR15b/miR155 |
| miR26b/Let7a\|miR210/miR34b | miR155/miR34b\|miR134/miR20a | miR210/miR20a\|miR210/miR134 |
| miR26b/Let7a\|miR210/Let7f | miR20a/miR155\|miR16/miR374 | miR16/miR374\|Let7a/miR409 |
| miR155/Let7f\|miR151/miR17 | miR155/miR339\|Let7d/miR409 | miR155/Let7f\|Let7f/miR10a |
| miR26b/Let7a\|miR134/miR409 | miR126/miR155\|Let7d/Let7f | miR210/miR20a\|Let7d/miR409 |
| miR155/miR339\|Let7d/Let7f | miR16/miR126\|Let7a/miR409 | miR26b/miR15b\|miR210/miR134 |
| miR16/Let7a\|miR126/Let7f | miR151/miR155\|miR134/miR409 | miR26b/miR210\|miR15b/miR134 |
| miR151/miR155\|miR134/Let7f | miR26b/miR155\|miR210/miR20a | miR20a/miR10a\|miR155/Let7f |
| miR17/Let7d\|Let7a/miR409 | miR15b/miR10a\|miR134/miR17 | miR16/Let7a\|miR127/miR126 |
| miR210/miR127\|miR16/miR210 | miR210/miR339\|miR155/miR34b | miR26b/miR155\|miR155/miR339 |
| miR16/miR126\|miR134/miR409 | miR210/miR126\|miR16/miR374 | miR210/miR409\|miR15b/miR34b |
| miR16/Let7a\|miR134/miR17 | miR210/miR20a\|Let7d/Let7f | miR26b/miR15b\|Let7d/miR409 |
| miR16/miR374\|miR134/Let7f | miR20a/miR155\|miR155/miR374 | miR127/miR409\|Let7a/miR409 |
| miR155/miR17\|miR126/Let7f | miR26b/Let7a\|mR210/miR339 | miR15b/miR34b\|miR155/miR339 |
| miR16/miR20a\|miR15b/miR10a | miR16/miR20a\|miR16/Let7a | miR210/miR374\|miR16/miR374 |
| miR210/miR15b\|miR15b/miR10a | miR26b/miR10a\|miR126/miR10a | miR155/miR34b\|Let7d/miR409 |
| miR210/miR20a\|miR134/Let7d | miR26b/miR155\|miR126/miR155 | miR20a/miR10a\|miR127/miR409 |
| miR26b/miR15b\|miR134/Let7d | miR210/miR15b\|miR16/Let7a | miR210/miR10a\|miR126/Let7f |

| | | |
|---|---|---|
| miR155/Let7f\|miR134/Let7f | miR26b/miR15b\|Let7d/Let7f | miR151/miR17\|miR134/Let7d |
| miR155/miR17\|miR134/miR17 | miR16/miR374\|miR134/miR409 | miR155/Let7f\|Let7a/miR409 |
| miR155/miR34b\|miR134/Let7d | miR15b/miR34b\|miR126/miR155 | miR210/Let7d\|miR155/miR34b |
| miR210/miR374\|miR126/Let7f | miR210/miR339\|miR210/miR20a | miR374/miR10a\|miR210/miR15b |
| miR26b/Let7a\|miR155/Let7f | miR155/miR34b\|Let7d/Let7f | miR20a/miR10a\|miR17/Let7d |
| miR26b/miR210\|miR155/miR34b | miR26b/miR155\|Let7f/miR10a | miR374/miR10a\|miR16/miR210 |
| miR26b/miR155\|miR210/miR34b | miR210/miR126\|miR155/miR374 | miR210/Let7f\|miR155/Let7f |
| miR17/Let7d\|miR15b/miR34b | miR26b/Let7a\|miR15b/miR34b | miR210/miR17\|miR155/miR17 |
| miR210/Let7d\|Let7f/miR409 | miR16/Let7f\|miR155/miR339 | miR210/Let7f\|miR10a/miR34b |
| miR210/Let7f\|Let7d/miR409 | miR15b/miR10a\|Let7f/miR409 | miR210/miR409\|miR10a/miR339 |
| miR210/miR10a\|miR16/miR155 | miR126/miR10a\|Let7d/miR409 | miR26b/miR10a\|miR15b/miR134 |
| miR210/miR34b\|Let7f/miR409 | miR26b/miR10a\|miR155/miR339 | miR151/miR155\|miR10a/miR339 |
| miR26b/miR10a\|miR210/miR126 | miR26b/miR155\|miR10a/miR339 | miR16/miR374\|miR10a/miR339 |
| miR16/miR10a\|Let7d/Let7f | miR15b/miR34b\|miR10a/miR339 | miR127/miR409\|miR10a/miR339 |
| miR26b/miR10a\|miR210/Let7d | miR374/miR10a\|miR15b/miR134 | miR155/Let7f\|miR10a/miR339 |
| miR16/Let7a\|miR10a/miR34b | miR134/miR409\|miR10a/miR34b | miR134/Let7f\|miR10a/miR34b |
| miR210/miR409\|miR15b/miR134 | miR210/Let7d\|miR10a/miR34b | miR210/miR10a\|Let7f/miR409 |
| miR155/miR17\|miR10a/miR34b | miR210/miR34b\|miR10a/miR34b | miR134/miR409\|miR10a/miR339 |
| miR16/miR374\|miR15b/miR134 | miR210/miR339\|miR10a/miR339 | miR210/Let7d\|miR10a/miR339 |
| miR210/miR374\|miR10a/miR34b | miR16/miR10a\|miR155/miR374 | miR210/miR34b\|miR10a/miR339 |

(suite)

| | | |
|---|---|---|
| miR20a/miR10a\|miR155/miR339 | miR16/miR10a\|miR127/miR126 | miR151/miR17\|miR10a/miR34b |
| miR20a/miR155\|miR10a/miR339 | miR126/miR155\|miR10a/miR339 | miR16/Let7f\|miR10a/miR34b |
| miR16/miR10a\|miR155/miR34b | miR126/Let7f\|Let7f/miR409 | miR134/Let7d\|miR10a/miR339 |
| miR16/miR155\|miR10a/miR34b | miR210/miR20a\|miR10a/miR339 | miR210/miR10a\|miR15b/miR134 |
| miR210/miR126\|miR10a/miR339 | miR26b/miR15b\|miR10a/miR339 | miR26b/miR10a\|miR10a/miR34b |
| miR210/Let7f\|miR155/miR339 | miR155/miR34b\|miR10a/miR339 | miR126/miR10a\|miR10a/miR34b |
| miR16/miR10a\|miR126/miR155 | miR210/miR17\|miR15b/miR134 | miR16/miR10a\|Let7f/miR409 |
| miR16/miR10a\|Let7f/miR10a | miR26b/miR10a\|miR20a/miR10a | miR374/miR10a\|miR10a/miR339 |
| miR127/miR126\|miR10a/miR339 | miR210/miR10a\|miR15b/miR155 | miR210/miR17\|miR10a/miR339 |
| miR16/miR10a\|Let7a/miR409 | miR134/Let7d\|miR10a/miR34b | miR15b/miR10a\|miR10a/miR339 |
| miR210/Let7d\|miR15b/miR134 | miR210/miR10a\|Let7d/miR409 | miR16/miR10a\|miR15b/miR134 |
| miR210/miR34b\|miR15b/miR134 | miR134/miR20a\|miR10a/miR339 | miR210/miR10a\|miR10a/miR34b |
| miR210/miR10a\|miR16/miR374 | miR20a/miR10a\|Let7f/miR409 | miR134/miR17\|miR10a/miR339 |
| miR155/miR374\|miR10a/miR339 | miR210/miR10a\|miR155/miR339 | miR126/Let7f\|miR10a/miR339 |
| miR151/miR17\|Let7d/miR409 | miR374/miR10a\|miR10a/miR34b | miR20a/miR10a\|miR10a/miR339 |
| miR210/miR10a\|miR155/miR374 | miR16/miR20a\|miR10a/miR339 | miR16/miR10a\|miR10a/miR34b |
| miR16/miR210\|miR10a/miR339 | miR210/miR15b\|miR10a/miR339 | miR210/Let7f\|miR10a/miR339 |
| miR16/miR10a\|miR15b/miR34b | miR210/miR17\|miR10a/miR34b | miR134/Let7f\|miR10a/miR339 |
| miR16/miR15b\|miR10a/miR34b | miR210/miR10a\|miR20a/miR10a | miR151/miR17\|miR10a/miR339 |
| miR20a/miR10a\|miR20a/miR10a | miR16/miR10a\|miR15b/miR155 | miR16/Let7f\|miR10a/miR339 |
| miR210/miR10a\|miR127/miR126 | miR20a/miR10a\|miR15b/miR134 | miR26b/miR10a\|miR10a/miR339 |
| miR26b/miR10a\|miR210/miR127 | miR15b/miR10a\|miR10a/miR34b | miR126/miR10a\|miR10a/miR339 |
| miR210/Let7f\|miR20a/miR10a | miR16/miR10a\|Let7d/miR409 | miR210/miR10a\|miR10a/miR339 |
| miR16/Let7f\|miR15b/miR155 | miR210/Let7f\|miR15b/miR134 | miR16/miR10a\|miR10a/miR339 |
| miR374/miR10a\|Let7f/miR409 | miR151/miR17\|Let7f/miR409 | miR210/miR126\|miR210/miR10a |
| miR16/Let7f\|Let7d/miR409 | miR16/Let7a\|miR10a/miR339 | miR210/miR127\|miR210/miR10a |
| miR210/miR10a\|miR210/Let7d | miR155/miR17\|miR10a/miR339 | miR210/miR134\|miR210/miR10a |
| miR26b/miR10a\|miR15b/miR155 | miR210/miR374\|miR10a/miR339 | miR210/miR15b\|miR210/miR10a |
| miR26b/miR10a\|miR210/miR134 | miR134/miR17\|miR10a/miR34b | miR210/miR17\|miR210/miR10a |
| miR210/miR409\|miR10a/miR34b | miR16/miR10a\|miR155/miR339 | miR210/miR20a\|miR210/miR10a |
| miR26b/miR10a\|Let7d/miR409 | miR16/miR155\|miR10a/miR339 | miR210/miR339\|miR210/miR10a |
| miR151/miR155\|miR10a/miR34b | miR126/Let7f\|miR10a/miR34b | miR210/miR34b\|miR210/miR10a |
| miR16/miR374\|miR10a/miR34b | miR16/Let7f\|Let7f/miR409 | miR210/miR374\|miR210/miR10a |
| miR26b/Let7a\|miR10a/miR339 | miR26b/miR10a\|Let7f/miR409 | miR210/miR409\|miR210/miR10a |
| miR127/miR409\|miR10a/miR34b | miR16/miR15b\|miR10a/miR339 | miR26b/Let7a\|miR210/miR10a |
| miR16/miR126\|miR10a/miR339 | miR126/miR10a\|Let7f/miR409 | miR26b/miR10a\|miR210/miR10a |
| miR210/miR17\|Let7f/miR409 | miR20a/miR10a\|miR10a/miR34b | miR26b/miR155\|miR210/miR10a |
| miR155/Let7f\|miR10a/miR34b | miR16/Let7f\|miR15b/miR134 | miR26b/miR15b\|miR210/miR10a |
| miR155/miR17\|miR127/miR126 | miR374/miR10a\|miR210/miR339 | miR210/miR339\|miR15b/miR134 |
| miR210/miR374\|miR155/miR374 | miR16/miR20a\|miR155/miR339 | miR15b/miR10a\|miR155/miR374 |
| miR210/miR339\|miR155/miR339 | miR210/miR15b\|miR155/miR339 | miR155/miR374\|miR10a/miR34b |
| miR210/miR34b\|miR155/miR34b | miR155/miR17\|Let7d/miR409 | miR210/miR17\|miR127/miR126 |
| miR20a/miR10a\|Let7d/miR10a | miR26b/miR10a\|miR151/miR155 | miR151/miR17\|miR134/miR20a |
| miR210/miR10a\|miR134/miR17 | miR210/Let7d\|miR155/miR374 | miR16/miR374\|miR155/miR339 |
| miR210/miR34b\|miR210/miR20a | miR210/miR34b\|miR155/miR374 | miR16/miR210\|miR10a/miR34b |
| miR210/miR127\|Let7f/miR409 | miR210/miR374\|miR15b/miR155 | miR20a/miR10a\|miR15b/miR34b |
| miR210/Let7f\|miR127/miR409 | miR15b/miR134\|miR10a/miR339 | miR15b/miR10a\|miR127/miR126 |
| miR16/miR155\|miR155/miR374 | miR374/miR10a\|miR15b/miR34b | miR16/miR10a\|miR134/miR409 |
| miR16/miR374\|miR15b/miR34b | miR210/miR17\|miR155/miR34b | miR151/miR17\|Let7d/Let7f |
| miR134/miR409\|miR126/miR155 | miR374/miR10a\|miR26b/miR155 | miR210/Let7f\|miR15b/miR34b |

(suite)

| | | |
|---|---|---|
| miR20a/miR10a\|miR151/miR155 | miR210/miR374\|miR210/miR134 | miR210/miR17\|miR210/miR126 |
| miR15b/miR10a\|miR155/miR17 | miR26b/miR10a\|miR210/miR409 | miR210/miR17\|miR210/Let7d |
| miR210/Let7f\|miR17/Let7d | miR134/miR409\|miR127/miR126 | miR20a/miR10a\|miR16/miR155 |
| miR210/Let7d\|miR126/miR155 | miR16/miR10a\|miR15b/miR10a | miR210/miR20a\|miR15b/miR134 |
| miR210/miR17\|miR16/Let7a | miR16/miR155\|miR15b/miR155 | miR16/miR10a\|miR155/Let7f |
| miR210/miR374\|miR127/miR126 | miR210/miR374\|Let7d/miR409 | miR134/Let7f\|miR126/miR155 |
| miR134/miR409\|Let7f/miR10a | miR210/miR134\|miR10a/miR34b | miR26b/miR15b\|miR15b/miR134 |
| miR210/miR34b\|miR126/miR155 | miR210/Let7d\|miR127/miR126 | miR16/Let7f\|miR134/miR20a |
| miR210/Let7d\|Let7f/miR10a | miR210/miR10a\|miR134/Let7d | miR210/Let7d\|miR155/miR339 |
| miR210/Let7f\|Let7d/miR10a | miR210/miR34b\|miR127/miR126 | miR210/miR10a\|miR16/miR15b |
| miR210/miR409\|miR16/miR155 | miR20a/miR155\|miR15b/miR134 | miR134/Let7f\|Let7f/miR10a |
| miR134/Let7d\|Let7d/Let7f | miR15b/miR155\|miR10a/miR34b | miR210/miR34b\|miR155/miR339 |
| miR210/miR34b\|Let7f/miR10a | miR15b/miR10a\|miR155/miR34b | miR210/miR409\|miR20a/miR10a |
| miR26b/Let7a\|miR20a/miR10a | miR16/miR155\|Let7d/miR409 | miR16/miR10a\|miR127/miR409 |
| miR16/miR155\|miR127/miR126 | miR210/miR17\|miR126/miR155 | miR210/Let7f\|miR16/miR155 |
| miR210/Let7f\|miR151/miR155 | miR20a/miR10a\|miR16/miR210 | miR134/Let7d\|Let7d/miR409 |
| miR134/miR409\|Let7a/miR409 | miR210/miR126\|miR15b/miR134 | miR16/miR10a\|Let7d/miR10a |
| miR210/Let7d\|Let7a/miR409 | miR210/miR17\|Let7f/miR10a | miR16/Let7f\|Let7d/Let7f |
| miR210/miR34b\|Let7a/miR409 | miR134/miR17\|Let7d/Let7f | miR134/miR17\|miR127/miR126 |
| miR210/miR374\|miR210/miR126 | miR15b/miR10a\|miR126/miR155 | miR16/miR10a\|miR151/miR155 |
| miR16/miR374\|miR16/miR155 | miR374/miR10a\|miR16/miR155 | miR26b/miR10a\|miR134/miR20a |
| miR16/Let7f\|miR134/Let7d | miR15b/miR10a\|Let7f/miR10a | miR210/miR10a\|miR155/miR17 |
| miR210/miR20a\|miR155/miR339 | miR210/miR17\|Let7a/miR409 | miR151/miR17\|miR126/miR155 |
| miR26b/miR15b\|miR155/miR339 | miR210/Let7f\|miR16/miR210 | miR151/miR17\|Let7f/miR10a |
| miR210/miR374\|miR210/Let7d | miR126/miR10a\|Let7d/miR10a | miR374/miR10a\|miR210/miR127 |
| miR155/miR34b\|miR155/miR339 | miR126/Let7f\|Let7d/Let7f | miR210/miR10a\|miR16/Let7a |
| miR210/miR34b\|miR210/miR339 | miR16/Let7a\|miR155/miR339 | miR26b/miR10a\|Let7d/Let7f |
| miR10a/miR34b\|miR10a/miR339 | miR210/miR34b\|miR210/miR126 | miR26b/Let7a\|miR10a/miR34b |
| miR134/miR20a\|Let7d/miR409 | miR374/miR10a\|miR26b/Let7a | miR16/miR20a\|Let7f/miR409 |
| miR210/Let7d\|miR15b/miR34b | miR210/Let7d\|miR210/Let7d | miR151/miR17\|Let7a/miR409 |
| miR26b/miR155\|miR20a/miR10a | miR210/miR127\|miR10a/miR34b | miR210/miR15b\|Let7f/miR409 |
| miR210/miR34b\|miR15b/miR34b | miR210/miR34b\|miR210/Let7d | miR16/miR126\|miR10a/miR34b |
| miR210/miR134\|miR15b/miR134 | miR15b/miR10a\|Let7a/miR409 | miR16/Let7f\|miR155/miR34b |
| miR210/miR10a\|miR15b/miR10a | miR26b/miR10a\|miR16/miR20a | miR374/miR10a\|miR15b/miR155 |
| miR210/miR17\|miR210/miR15b | miR210/miR17\|miR15b/miR34b | miR210/Let7d\|miR20a/miR10a |
| miR210/miR17\|miR16/miR210 | miR210/miR15b\|miR20a/miR10a | miR17/Let7d\|miR10a/miR339 |
| miR151/miR17\|miR134/miR409 | miR210/miR374\|miR155/miR339 | miR210/miR34b\|miR20a/miR10a |
| miR15b/miR155\|miR15b/miR134 | miR16/miR15b\|miR15b/miR155 | miR16/Let7f\|miR126/miR155 |
| miR26b/miR10a\|miR134/Let7d | miR26b/Let7a\|Let7f/miR409 | miR374/miR10a\|miR210/miR134 |
| miR16/miR15b\|miR155/miR374 | miR16/miR210\|miR15b/miR134 | miR374/miR10a\|Let7d/miR409 |
| miR17/Let7d\|miR10a/miR34b | miR16/miR126\|Let7f/miR409 | miR16/Let7f\|Let7f/miR10a |
| miR210/miR339\|miR20a/miR10a | miR134/Let7d\|miR127/miR126 | miR26b/miR10a\|miR155/miR34b |
| miR134/Let7f\|miR127/miR409 | miR16/miR155\|miR155/miR339 | miR26b/miR155\|miR10a/miR34b |
| miR20a/miR10a\|miR16/miR20a | miR210/miR10a\|miR210/Let7f | miR15b/miR34b\|miR10a/miR34b |
| miR374/miR10a\|miR134/miR20a | miR16/miR15b\|Let7d/miR409 | miR20a/miR10a\|miR16/miR374 |
| miR134/Let7d\|miR126/miR155 | miR210/miR10a\|miR134/miR409 | miR210/miR10a\|miR16/miR210 |
| miR16/miR15b\|miR127/miR126 | miR134/miR17\|miR126/miR155 | miR126/miR10a\|Let7d/Let7f |
| miR134/Let7f\|Let7d/miR10a | miR210/miR409\|miR210/miR127 | miR26b/miR10a\|miR210/miR20a |
| miR134/Let7d\|Let7f/miR10a | miR134/miR17\|Let7f/miR10a | miR16/Let7f\|Let7a/miR409 |
| miR210/miR15b\|miR210/miR127 | miR210/miR17\|miR16/miR155 | miR16/Let7a\|Let7f/miR409 |

(suite)

| | | |
|---|---|---|
| miR210/Let7f\|miR16/miR20a | miR20a/miR10a\|miR134/miR20a | miR210/miR339\|miR10a/miR34b |
| miR210/Let7d\|miR16/miR155 | miR126/Let7f\|Let7f/miR10a | miR20a/miR10a\|miR155/miR374 |
| miR210/miR34b\|miR16/miR155 | miR26b/miR155\|Let7f/miR409 | miR26b/miR10a\|miR126/miR155 |
| miR374/miR10a\|Let7d/Let7f | miR15b/miR34b\|Let7f/miR409 | miR210/miR17\|miR210/miR127 |
| miR210/miR127\|miR15b/miR134 | miR210/miR10a\|miR155/Let7f | miR210/Let7f\|miR16/miR374 |
| miR134/Let7d\|Let7a/miR409 | miR210/Let7f\|miR134/miR20a | miR26b/miR10a\|Let7f/miR10a |
| miR151/miR17\|miR127/miR409 | miR126/Let7f\|Let7a/miR409 | miR155/miR17\|Let7f/miR409 |
| miR16/Let7f\|miR134/miR409 | miR20a/miR10a\|Let7d/Let7f | miR20a/miR10a\|miR127/miR126 |
| miR210/miR409\|miR16/miR374 | miR210/miR339\|Let7f/miR409 | miR210/Let7f\|miR155/miR374 |
| miR151/miR17\|Let7d/miR10a | miR16/Let7f\|miR155/miR17 | miR26b/miR10a\|Let7a/miR409 |
| miR210/miR20a\|miR20a/miR10a | miR26b/miR10a\|miR16/miR15b | miR16/Let7f\|miR15b/miR34b |
| miR16/miR20a\|miR15b/miR155 | miR210/miR10a\|miR127/miR409 | miR210/miR374\|Let7f/miR409 |
| miR26b/miR15b\|miR20a/miR10a | miR210/miR409\|miR15b/miR155 | miR210/Let7f\|miR127/miR126 |
| miR20a/miR155\|Let7f/miR409 | miR374/miR10a\|miR16/miR374 | miR126/miR155\|miR10a/miR34b |
| miR210/miR15b\|miR15b/miR155 | miR210/miR10a\|miR17/Let7d | miR16/miR155\|Let7f/miR409 |
| miR151/miR17\|miR151/miR155 | miR210/miR409\|miR210/miR134 | miR210/miR17\|miR15b/miR155 |
| miR210/miR409\|miR155/miR374 | miR26b/miR10a\|miR210/miR374 | miR26b/miR10a\|miR210/miR339 |
| miR210/miR15b\|miR210/miR134 | miR210/Let7f\|Let7d/Let7f | miR26b/miR210\|miR10a/miR339 |
| miR210/miR126\|Let7f/miR409 | miR210/miR409\|Let7d/miR409 | miR16/miR20a\|miR15b/miR134 |
| miR16/miR20a\|Let7d/miR409 | miR210/miR10a\|Let7d/miR10a | miR210/miR15b\|miR15b/miR134 |
| miR210/miR15b\|Let7d/miR409 | miR16/Let7f\|miR16/Let7a | miR210/miR20a\|miR10a/miR34b |
| miR26b/miR10a\|miR210/miR34b | miR374/miR10a\|miR155/miR374 | miR210/miR17\|miR210/miR134 |
| miR26b/miR210\|miR10a/miR34b | miR16/miR15b\|miR155/miR339 | miR374/miR10a\|miR155/miR339 |
| miR26b/miR10a\|miR210/Let7f | miR210/miR10a\|miR151/miR155 | miR26b/miR10a\|miR15b/miR34b |
| miR20a/miR10a\|miR16/miR15b | miR210/miR374\|miR20a/miR10a | miR26b/miR15b\|miR10a/miR34b |
| miR16/miR374\|miR16/miR374 | miR151/miR155\|Let7d/miR409 | miR210/miR17\|Let7d/miR409 |
| miR26b/miR10a\|miR134/miR409 | miR26b/miR10a\|miR155/miR17 | miR155/miR34b\|miR10a/miR34b |
| miR374/miR10a\|miR155/miR34b | miR16/miR374\|miR15b/miR155 | miR155/miR339\|miR10a/miR339 |
| miR210/miR409\|miR127/miR126 | miR374/miR10a\|miR127/miR126 | miR126/miR10a\|Let7f/miR10a |
| miR16/miR10a\|miR126/Let7f | miR126/miR155\|Let7f/miR409 | miR374/miR10a\|miR26b/miR210 |
| miR374/miR10a\|miR26b/miR15b | miR16/miR374\|Let7d/miR409 | miR15b/miR10a\|Let7d/miR409 |
| miR210/miR17\|miR134/miR20a | miR16/miR10a\|miR134/Let7d | miR210/Let7f\|miR210/Let7d |
| miR155/miR339\|miR10a/miR34b | miR26b/miR10a\|miR16/Let7a | miR126/miR10a\|Let7a/miR409 |
| miR16/Let7f\|miR155/Let7f | miR127/miR409\|Let7d/miR409 | miR210/miR10a\|miR134/miR20a |
| miR16/miR374\|miR155/miR374 | miR26b/Let7a\|miR15b/miR134 | miR16/miR10a\|miR155/miR17 |
| miR151/miR155\|miR127/miR126 | miR20a/miR155\|miR10a/miR34b | miR26b/miR10a\|miR16/miR155 |
| miR210/Let7f\|miR16/miR15b | miR20a/miR10a\|miR155/miR34b | miR210/miR10a\|Let7d/Let7f |
| miR16/miR10a\|miR134/miR17 | miR210/miR34b\|miR210/miR127 | miR26b/miR10a\|miR26b/Let7a |
| miR16/Let7f\|miR127/miR409 | miR210/miR20a\|Let7f/miR409 | miR16/miR10a\|miR16/Let7a |
| miR374/miR10a\|miR126/miR155 | miR26b/miR15b\|Let7f/miR409 | miR16/Let7a\|miR15b/miR134 |
| miR15b/miR10a\|miR134/miR20a | miR155/Let7f\|Let7d/miR409 | miR134/Let7f\|miR127/miR126 |
| miR210/miR17\|Let7d/Let7f | miR16/miR126\|miR15b/miR134 | miR16/miR15b\|Let7f/miR409 |
| miR127/miR126\|Let7f/miR409 | miR155/miR34b\|Let7f/miR409 | miR210/miR17\|miR155/miR339 |
| miR20a/miR10a\|miR155/miR17 | miR210/miR126\|miR10a/miR34b | miR134/miR20a\|miR10a/miR34b |
| miR374/miR10a\|Let7f/miR10a | miR210/Let7f\|miR155/miR34b | miR210/miR134\|miR10a/miR339 |
| miR16/miR374\|miR127/miR126 | miR374/miR10a\|miR210/miR126 | miR134/miR17\|Let7d/miR409 |
| miR210/miR374\|miR210/miR127 | miR20a/miR10a\|miR126/miR155 | miR374/miR10a\|miR20a/miR10a |
| miR16/Let7f\|Let7d/miR10a | miR20a/miR10a\|Let7f/miR10a | miR210/miR374\|miR15b/miR134 |
| miR127/miR409\|miR127/miR126 | miR374/miR10a\|miR210/Let7d | miR15b/miR10a\|miR155/miR339 |
| miR210/miR409\|miR210/miR126 | miR210/miR17\|miR16/miR374 | miR15b/miR155\|miR10a/miR339 |

(suite)

| | | |
|---|---|---|
| miR16/Let7a\|miR15b/miR155 | miR210/Let7f\|miR126/miR155 | miR151/miR17\|miR127/miR126 |
| miR26b/miR10a\|miR155/Let7f | miR210/miR409\|miR155/miR339 | miR126/Let7f\|Let7d/miR409 |
| miR15b/miR10a\|Let7d/Let7f | miR210/Let7d\|miR15b/miR155 | miR16/miR155\|miR15b/miR134 |
| miR16/Let7f\|miR151/miR155 | miR210/miR34b\|miR15b/miR155 | miR210/miR10a\|miR155/miR34b |
| miR155/miR374\|Let7f/miR409 | miR20a/miR10a\|Let7a/miR409 | miR210/miR10a\|miR126/miR155 |
| miR374/miR10a\|Let7a/miR409 | miR127/miR126\|miR10a/miR34b | miR210/miR409\|Let7f/miR409 |
| miR20a/miR10a\|miR16/Let7a | miR210/Let7f\|Let7f/miR10a | miR210/miR10a\|Let7f/miR10a |
| miR210/Let7f\|miR155/miR17 | miR26b/miR155\|miR15b/miR134 | miR210/miR127\|miR10a/miR339 |
| miR155/Let7f\|miR127/miR126 | miR210/miR17\|miR155/miR374 | miR16/Let7f\|miR155/miR374 |
| miR210/miR409\|miR210/Let7d | miR134/miR409\|Let7d/miR409 | miR151/miR155\|Let7f/miR409 |
| miR26b/miR10a\|miR127/miR409 | miR134/Let7f\|Let7d/Let7f | miR210/miR10a\|Let7a/miR409 |
| miR16/Let7a\|Let7d/miR409 | miR210/miR34b\|miR210/miR134 | miR16/miR374\|Let7f/miR409 |
| miR16/miR210\|Let7f/miR409 | miR15b/miR34b\|miR15b/miR134 | miR16/Let7f\|miR127/miR126 |
| miR26b/miR10a\|miR17/Let7d | miR210/miR10a\|miR16/miR20a | miR26b/miR10a\|miR16/miR374 |
| miR210/Let7d\|miR16/miR374 | miR210/Let7d\|Let7d/miR409 | miR127/miR409\|Let7f/miR409 |
| miR210/miR34b\|miR16/miR374 | miR210/miR34b\|Let7d/miR409 | miR210/miR17\|miR20a/miR10a |
| miR210/Let7f\|miR16/Let7a | miR26b/miR10a\|miR210/miR15b | miR26b/miR10a\|miR155/miR374 |
| miR26b/miR10a\|Let7d/miR10a | miR26b/miR10a\|miR16/miR210 | miR155/Let7f\|Let7f/miR409 |
| miR16/miR10a\|miR134/miR20aa | miR20a/miR10a\|miR15b/miR155 | miR16/miR15b\|miR15b/miR134 |
| miR134/miR409\|Let7f/miR409 | miR26b/miR10a\|miR127/miR126 | miR16/miR20a\|miR10a/miR34b |
| miR26b/miR210\|miR210/miR10a | miR20a/miR10a\|Let7d/miR409 | miR210/miR15b\|miR10a/miR34b |
| miR374/miR10a\|miR210/miR10a | miR210/Let7f\|miR15b/miR155 | miR210/miR10a\|miR15b/miR34b |
| miR374/miR10a\|miR210/miR20a | | |

## Revendications

1. Utilisation d'au moins deux miARNs pour la mise en oeuvre d'une méthode diagnostic spécifique in vitro d'une tumeur cérébrale appartenant au groupe constitué de 2 types de tumeurs : les oligodendrogliomes (ODG) et les glioblastomes (GBM), et d'identification du type de la tumeur, comprenant :

   (i) la mesure du niveau d'expression dans un échantillon biologique provenant d'un patient suspecté de présenter un gliome,

      - un premier miARN choisi parmi hsa-miR-155, hsa-miR-10a, hsa-miR-409 ou hsa-miR-134, et
      - un deuxième miARN choisi parmi hsa-miR-126, hsa-miR-15b, hsa-miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-151, hsa-miR-26b, hsa-miR-34b*, hsa-miR-155 ou hsa-miR-10a, ledit deuxième miARN choisi étant différent du susdit premier miARN choisi,

   (ii) la comparaison entre les niveaux d'expression des susdits miARNs dans le susdit échantillon et les niveaux d'expression des susdits miARNs dans un échantillon sain de référence, un échantillon de GBM de référence, et un échantillon d'ODG de référence,

   dans laquelle la déduction de la présence de GBM dans l'échantillon provenant du patient suspecté de présenter un gliome, est faite lorsque :

   (i) le niveau d'expression du premier miARN mesuré dans ledit échantillon est au moins 3 fois supérieur ou inférieur au niveau d'expression du susdit miARN mesuré dans l'échantillon sain de référence, et
   (ii) le niveau d'expression du premier miARN mesuré dans ledit échantillon est au moins 3 fois supérieur au niveau d'expression du susdit miARN mesuré dans l'échantillon d'ODG de référence, et
   (iii) le niveau d'expression du deuxième miARN mesuré dans ledit échantillon est au moins 3 fois supérieur ou inférieur au niveau d'expression du susdit miARN mesuré dans l'échantillon sain de référence, et

(iv) le niveau d'expression du deuxième miARN mesuré dans ledit échantillon est égal, dans la limite de 20% en plus ou 20% en moins, au niveau d'expression du susdit miARN dans l'échantillon de GBM de référence,

et dans laquelle la déduction de la présence d'ODG dans l'échantillon provenant du patient suspecté de présenter un gliome, est faite lorsque :

(i) le niveau d'expression du premier miARN mesuré dans ledit échantillon est au moins 3 fois supérieur ou inférieur au niveau d'expression du susdit miARN mesuré dans l'échantillon sain de référence, et
(ii) le niveau d'expression du premier miARN mesuré dans ledit échantillon est au moins 3 fois inférieur au niveau d'expression du susdit miARN mesuré dans l'échantillon de GBM de référence, et
(iii) le niveau d'expression du deuxième miARN mesuré dans ledit échantillon est au moins 3 fois supérieur ou inférieur au niveau d'expression du susdit miARN mesuré dans l'échantillon sain de référence, et
(iv) le niveau d'expression du deuxième miARN mesuré dans ledit échantillon est égal, dans la limite de 20% en plus ou 20% en moins, au niveau d'expression du susdit miARN dans l'échantillon d'ODG de référence.

## Patentansprüche

1. Verwendung von mindestens zwei miRNAs für die Durchführung eines spezifischen diagnostischen Verfahrens in vitro eines Gehirntumors, der zu der Gruppe gehört, die aus zwei Arten von Tumoren besteht:

Oligodendrogliomen (ODG) und Glioblastomen (GBM),
und der Identifizierung des Tumortyps, umfassend:

(i) das. Bestimmen in einer biologischen Probe, die von einem Patienten stammt, der im Verdacht steht, ein Gliom zu haben, des Expressionsniveaus

- einer ersten miRNA ausgewählt aus hsa-miR-155, hsa-miR-10a, hsa-miR-409 und hsa-miR-134 und
- einer zweiten miRNA ausgewählt aus hsa-miR-126, hsa-miR-15b, hsa-miR-16, hsa-miR-17, hsa-miR-20a, hsa-let7a, hsa-let7f, hsa-let7d, hsa-miR-374, hsa-miR-409, hsa-miR-134, hsa-miR-339, hsa-miR-127, hsa-miR-151, hsa-miR-26b, hsa-miR-34b*, hsa-mir-155 oder hsa-miR-10a, wobei sich die zweite ausgewählte miRNA von der ersten ausgewählten miRNA unterscheidet,

(ii) das Vergleichen der Expressionsniveaus der miRNAs in der Probe und der Expressionsniveaus der miRNAs in einer gesunden Referenzprobe, einer GBM- Referenzprobe und einer ODG-Referenzprobe,

wobei das Vorhandensein von GBM in der Probe, die von einem Patienten stammt, der unter Verdacht steht, ein Gliom zu haben, gefolgert wird, wenn:

(i) das Expressionsniveau der ersten in der Probe gemessenen miRNA mindestens 3 Mal höher oder niedriger ist als das Expressionsniveau der in der gesunden Referenzprobe gemessenen miRNA und
(ii) das Expressionsniveau des ersten in der Probe gemessenen miRNA mindestens 3 Mal höher ist als das Expressionsniveau der in der ODG-Referenzprobe gemessenen miRNA und
(iii) das Expressionsniveau der zweiten in der Probe gemessenen miRNA mindestens 3 Mal höher oder niedriger ist als das Expressionsniveau der in der gesunden Referenzprobe gemessenen miRNA und
(iv) das Expressionsniveau der zweiten in der Probe gemessenen miRNA gleich ist, innerhalb einer Spanne von 20% mehr oder von 20% weniger, wie das Expressionsniveau der miRNA in der GBM-Referenzprobe,

und wobei das Vorhandensein von ODG in der Probe, die von einem Patienten stammt, der unter Verdacht steht, ein Gliom zu haben, gefolgert wird, wenn:

(i) das Expressionsniveau der ersten in der Probe gemessenen miRNA mindestens 3 Mal höher oder niedriger ist als das Expressionsniveau der in der gesunden Referenzprobe gemessenen miRNA und
(ii) das Expressionsniveau des ersten in der Probe gemessenen miRNA mindestens 3 Mal niedriger ist als das Expressionsniveau der in der GBM-Referenzprobe gemessenen miRNA und
(iii) das Expressionsniveau der zweiten in der Probe gemessenen miRNA mindestens 3 Mal höher oder niedriger ist als das Expressionsniveau der in der gesunden Referenzprobe gemessenen miRNA und
(iv) das Expressionsniveau der zweiten in der Probe gemessenen miRNA gleich ist, innerhalb einer Spanne von 20% mehr oder von 20% weniger, wie das Expressionsniveau der miRNA in der ODG-Referenzprobe.

**Claims**

1. Use of at least two miRNAs for the implementation of a specific method for the *in vitro* diagnosis of a brain tumour belonging to the group constituted by 2 types of tumours:

   oligodendrogliomas (ODG) and glioblastomas (GBM), and identification of the type of the tumour, comprising:

   (i) measurement of the expression level of at least two miRNAs in a biological sample originating from a patient with a suspected glioma:

   - a first miRNA being chosen from hsa-miR-155, hsa-miR-10a, hsa-miR-409 or hsa-miR-134;
   - a second miRNA being chosen from hsa-miR-126, hsa-miR-15b, hsa-miR-16, hsa-miR-17, hsa-miR-20a, hsa-let7a, hsa-let 7f, hsa-let 7d, hsa-miR-374, hsa-miR-409, hsa-miR-134, hsa-miR-339, hsa-miR-127, hsa-miR-151, hsa-miR-26b, hsa-miR-34b*, hsa-miR-155 or hsa-miR-10a, said second chosen miRNA being different from the abovementioned first chosen miRNA;

   (ii) comparison between the expression levels of the abovementioned miRNAs in the abovementioned sample and the expression levels of the abovementioned miRNA in a healthy reference sample, a reference GBM sample and a reference ODG sample,

   wherein the deduction of the presence of GBM in the sample originating from the patient with a suspected glioma is made when:

   (i) the expression level of the first miRNA measured in said sample is at least 3 times greater than or less than the expression level of the abovementioned miRNA measured in the healthy reference sample, and
   (ii) the expression level of the first miRNA measured in said sample is at least 3 times greater than the expression level of the abovementioned miRNA measured in the reference ODG sample, and
   (iii) the expression level of the second miRNA measured in said sample is at least 3 times greater than or less than the expression level of the abovementioned miRNA measured in the healthy reference sample, and
   (iv) the expression level of the second miRNA measured in said sample is equal, within a limit of 20% more or 20% less, to the expression level of the abovementioned miRNA in the reference GBM sample;

   wherein the deduction of the presence of ODG in the sample originating from the patient with a suspected glioma is made when:

   (i) the expression level of the first miRNA measured in said sample is at least 3 times greater than or less than the expression level of the abovementioned miRNA measured in the healthy reference sample, and
   (ii) the expression level of the first miRNA measured in said sample is at least 3 times less than the expression level of the abovementioned miRNA measured in the reference GBM sample, and
   (iii) the expression level of the second miRNA measured in said sample is at least 3 times greater than or less than the expression level of the abovementioned miRNA measured in the healthy reference sample, and
   (iv) the expression level of the second miRNA measured in said sample is equal, within a limit of 20% more or 20% less, to the expression level of the abovementioned miRNA in the reference ODG sample.

Figure 1

**Figure 2**

## Figure 3

## Figure 4

## Figure 5

## Figure 6

**Figure 7**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **BARTEL et al.** *Cell,* 2004, vol. 116 (2), 281-97
- **BENARD, J. ; S. DOUC-RASY.** *Bull Cancer,* 2005, vol. 92 (9), 757-62
- *Hammond Curr Opin Genet Dev,* 2006, vol. 16 (1), 4-9
- **CIAFRE et al.** *Biochem Biophys Res Commun,* 09 Septembre 2005, vol. 334 (4), 1351-8
- **CHEN.** *N Engl J Med,* 2005, vol. 353 (17), 1768-71
- **SILBER et al.** *BMC Med.,* 24 Juin 2008, vol. 6, 14
- **GRIFFITHS-JONES et al.** *NAR,* 2008, vol. 36, D154-D158
- **AMBROS et al.** *RNA,* 2003, vol. 9 (3), 277-279
- **LI A ; WALLING J ; AHN S ; KOTLIAROV Y ; SU Q ; QUEZADO M ; OBERHOLTZER JC ; PARK J ; ZENKLUSEN JC ; FINE HA.** *Cancer Res.,* 01 Mars 2009, vol. 69 (5), 2091-9
- **MITCHELL PS et al.** *Proc Natl Acad Sci USA.,* 29 Juillet 2008, vol. 105 (30), 10513-8
- **LODES MJ et al.** *PLoS One,* 14 Juillet 2009, vol. 4 (7), e6229
- **SKOG et al.** *Nat Cell Biol.,* Décembre 2008, vol. 10 (12), 1470-6

SEQUENCE LISTING

<110> UNIVERSITE JOSEPH FOURIER

<120> Utilisation de miARNs comme biomarqueurs dans le diagnostic de gliomes

<130> IFB 09 BO UJF MIRN

<160> 19

<170> PatentIn version 3.5

<210> 1
<211> 22
<212> RNA
<213> Homo sapiens

<220>
<221> misc_RNA
<222> (1)..(22)

<400> 1
ucguaccgug aguaauaaug cg        22

<210> 2
<211> 22
<212> RNA

<213> Homo sapiens

<220>
<221> misc_RNA
<222> (1)..(22)

<400> 2
uagcagcacg uaaauauugg cg     22

<210> 3
<211> 23
<212> RNA
<213> Homo sapiens

<400> 3
uaaagugcuu auagugcagg uag     23

<210> 4
<211> 22
<212> RNA
<213> Homo sapiens

<400> 4
ugagguagua gguuguauag uu     22

<210> 5
<211> 22
<212> RNA
<213> Homo sapiens

<400> 5
ugagguagua gauuguauag uu     22

<210> 6
<211> 22
<212> RNA
<213> Homo sapiens

<400> 6
agagguagua gguugcauag uu     22

<210> 7
<211> 22
<212> RNA
<213> Homo sapiens

<400> 7
uuauaauaca accugauaag ug     22

<210> 8
<211> 22
<212> RNA
<213> Homo sapiens

<400> 8
uagcagcaca ucaugguuua ca     22

<210> 9

<211> 22
<212> RNA
<213> Homo sapiens

<400> 9
ucggauccgu cugagcuugg cu       22

<210> 10
<211> 23
<212> RNA
<213> Homo sapiens

<400> 10
ucccuguccu ccaggagcuc acg     23

<210> 11
<211> 22
<212> RNA
<213> Homo sapiens

<400> 11
agguuacccg agcaacuuug ca       22

<210> 12
<211> 22
<212> RNA
<213> Homo sapiens

<400> 12
ugugacuggu ugaccagagg gg       22

<210> 13
<211> 23
<212> RNA
<213> Homo sapiens

<400> 13
uuaaugcuaa ucgugauagg ggu     23

<210> 14
<211> 22
<212> RNA
<213> Homo sapiens

<400> 14
cugugcgugu gacagcggcu ga       22

<210> 15
<211> 23
<212> RNA
<213> Homo sapiens

<400> 15
uacccuguag auccgaauuu gug     23

<210> 16
<211> 21
<212> RNA

<213> Homo sapiens

<400> 16
uucaaguaau ucaggauagg u          21

<210> 17
<211> 22
<212> RNA
<213> Homo sapiens

<400> 17
caaucacuaa cuccacugcc au          22

<210> 18
<211> 23
<212> RNA
<213> Homo sapiens

<400> 18
caaagugcuu acagugcagg uag          23

<210> 19
<211> 21
<212> RNA
<213> Homo sapiens

<400> 19
cuagacugaa gcuccuugag g          21